# EUROPEAN PATENT SPECIFICATION

(11) **EP 0 434 965 B1**
(45) Date of publication and mention of the grant of the patent: **20.05.1998**
(21) Application number: 90122074.9
(22) Date of filing: 19.11.1990
(51) Int. Cl.: C07D 405/04, C07D 403/04, C07D 409/04, C07D 413/04, C07D 411/04, A01N 43/90, C07D 417/04, C07D 405/14, C07D 413/14, C07D 409/14

(54) **Alkyl esters of 5-heterocyclic-pyridine-2,3-dicarboxylic acids and 5-heterocyclic 2-(2-imidazolin-2-yl) pyridines and methods for preparation thereof**
Alkylester von 5-heterocyclische-Pyridin-2,3-Dicarbonsäuren und 5-heterocyclische-2-(2-Imidazolin-2-yl)Pyridinen und Verfahren zu ihrer Herstellung
Esters alcoyliques d'acides 5-hétérocyclique-pyridine-2,3-dicarboxyliques et 5-hétérocyclique-2-(2-imidazolin-2-yl)pyridines et leur procédé de préparation

(30) Priority: 27.12.1989 US 457607; 27.12.1989 US 457606
(43) Date of publication of application: 03.07.1991
(73) Proprietor: AMERICAN CYANAMID COMPANY, Wayne, NJ 07470-8426 (US)
(72) Inventor: Finn, John Michael, Mercerville, New Jersey 08619 (US)
(74) Representative: Wächtershäuser, Günter, Prof. Dr.

(56) References cited:
- EP-A- 0 216 360
- EP-A- 0 254 951
- EP-A- 0 296 109
- EP-A- 0 322 616

## Description

The invention relates to herbicidally active compounds having a 2-(2-imidazolin-2-yl)pyridin-3-carboxylic structure. Such compounds are known from EP-A-296 109 and EP-A-216 360.

It is an object of the present invention to provide 5-heterocyclic-pyridine-2,3-dicarboxylate esters useful as intermediates in the preparation of 5-heterocyclic-2-(2-imidazolin-2-yl)pyridine herbicidal agents and processes for the preparation thereof.

It is a further object of the present invention to provide 5-heterocyclic 2-(2-imidazolin-2-yl)pyridine compounds that are highly effective for controlling undesirable plant species.

These problems are solved according to the claims.

The present invention relates to 5-heterocyclic-pyridine-2,3-dicarboxylates having the structure 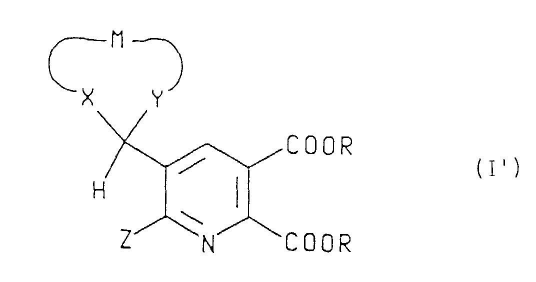 wherein
R is C₁-C₆ alkyl;
X and Y are each independently oxygen, sulfur or NR₄;
R₄ is hydrogen, C₁-C₆ alkyl optionally substituted with C₁-C₄ alkoxy or 1-3 halogens, SO₂R₅, COR₅, CO₂R₅ or CONR₅R₅;
R₅ is hydrogen, C₁-C₆ alkyl optionally substituted with 1-3 halogens, or C₂-C₆ alkenyl;
M is C₂-C₅ alkylene optionally substituted with 1 or 2 C₁-C₄ alkyl groups, C₁-C₄ alkoxy, halogen, CO₂R₆ or oxo, and optionally interrupted by one oxygen or one sulfur,
   C₂ alkenylene optionally substituted with 1 or 2 C₁-C₄ alkyl groups or CO₂R₆,
   C₃ alkenylene optionally substituted with 1 or 2 C₁-C₄ alkyl groups, CO₂R₆ or oxo,
   methyleneamino, optionally substituted with C₁-C₄ alkyl or CO₂R₆, or
   a single bond, with the proviso that both X and Y are NR₄,
   provided that the ring formed by M, X and Y and the carbon to which they are attached is no more than 8 atoms and provided that when the substituents on M are either alkoxy or halogen the substituted carbon is not bound to X or Y;
R₆ is hydrogen, methyl or ethyl;
Z is hydrogen, halogen, C₁-C₆ alkoxy, or C₁-C₆ alkyl optionally substituted with C₁-C₄ alkoxy or halogen.

The compounds of the present invention are useful as intermediates in the preparation of highly effective 5-heterocyclic-2-(2-imidazolin-2-yl)pyridine herbicidal agents.

The present invention also describes 5-heterocyclic 2-(2-imidazolin-2-yl)pyridine compounds that are highly effective herbicidal agents useful for the control of undesirable plant species and methods for their preparation.

The 5-heterocyclic 2-(2-imidazolin-2-yl)pyridine compounds of the present invention have the structural formula illustrated as formula Ia : 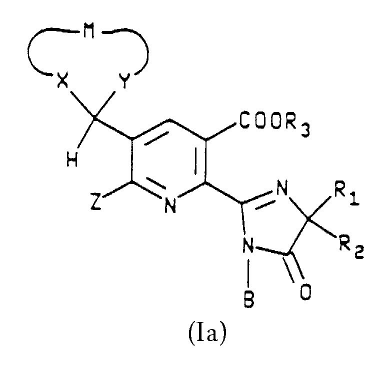 wherein
R₁ is C₁-C₄ alkyl;
R₂ is C₁-C₄ alkyl or C₃-C₆ cycloalkyl, and when R₁ and R₂ are taken together with the carbon to which they are attached they may represent C₃-C₆ cycloalkyl;
R₃ is hydrogen,
   C₁-C₆ alkyl optionally substituted with one of the following groups: C₁-C₃ alkoxy, halogen or phenyl,
   C₃-C₆ alkenyl optionally substituted with one of the following groups: C₁-C₃ alkoxy, phenyl or halogen,
   C₃-C₆ alkynyl,
   C₃-C₆ cycloalkyl optionally substituted with C₁-C₃ alkyl or
   a cation of alkali metals, ammonium or organic ammonium;
B is hydrogen or COR₇, provided that when B is COR₇, R₃ is other than hydrogen or a cation;
R₇ is C₁-C₅ alkyl or phenyl optionally substituted with halogen, nitro or methoxy;
X, Y, M and Z are as described above for formula I';
the N-oxides thereof, when R₃ is not unsaturated alkyl and when M is not alkenylene;
the optical isomers thereof, when R₁ and R₂ represent different substitutents;
the acid addition salts thereof, when R₃ is other than a cation and the tautomers thereof.

The present invention also relates to novel substituted imidazopyrrolopyridinediones illustrated by formulas II and III. Carbamoyl nicotinic acids, esters and salts depicted by formula IV and pyridinedicarboxylic acids, diesters and salts depicted by formula V are intermediates in the inventive methods for the preparation of compounds of formula Ia. 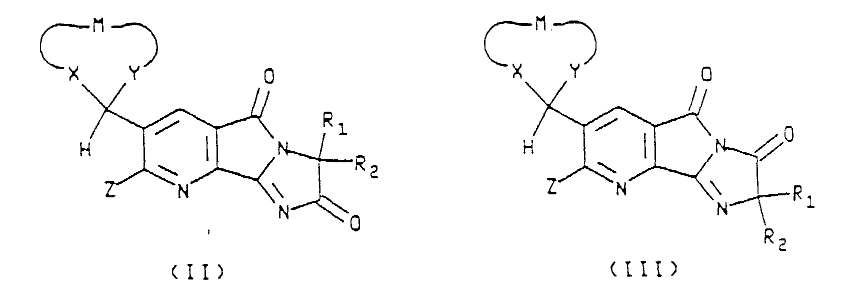 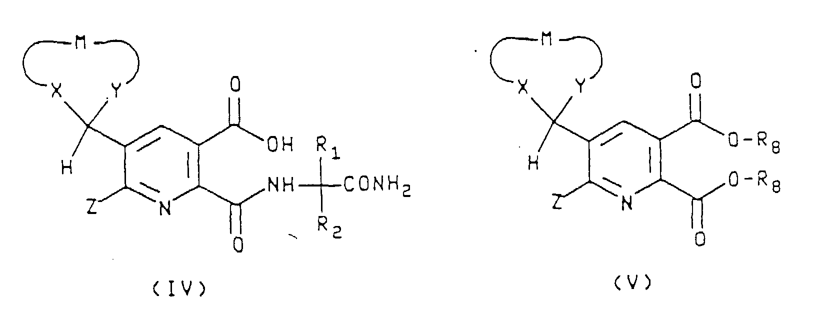 In the above structures II-V, M, X, Y, Z, R₁ and R₂ are as described above and R₈ is C₁-C₄ alkyl.

Potent herbicides, such as the imidazolinone family of herbicides, are highly desirable for use in agronomic practice because their high potency implies a low use rate combined with effective weed control. A low use rate means increased safety for the farmer and the environment. However, increased crop injury inherent in high herbicidal potency means the range of agronomic crops in which weeds can be controlled can be limited. Surprisingly, it has been found that the herbicidal imidazolinone compounds of the present invention demonstrate enhanced tolerance for sensitive crop species while retaining effective weed control.

The 5-heterocyclic-pyridinedicarboxylates of the present invention are important intermediates in the preparation of highly effective 5-heterocyclic-2-(2-imidazolin-2-yl)pyridine herbicidal agents of formula IIa. 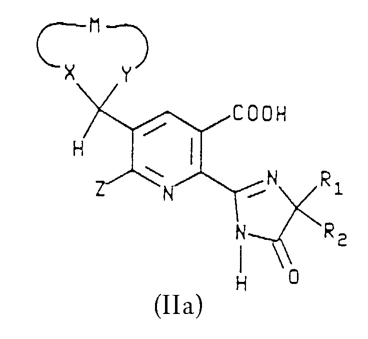 wherein
R₁ is C₁-C₄ alkyl;
R₂ is C₁-C₄ alkyl or C₃-C₆ cycloalkyl and when R₁ and R₂ are taken together with the carbon to which they are attached they may represent C₃-C₆ cycloalkyl;
Z, X, M and Y are as described above for formula I'.

Conversion of the formula I' 5-heterocyclicpyridine diesters of the present invention to the highly effective herbicidal agents of formula IIa is achieved by the reaction of the formula I' 5-heterocyclic-2,3-pyridinedicarboxylate ester with an aminocarboxamide having the structure 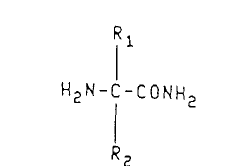 in the presence of a base, such as an alkali metal butoxide, and a solvent followed by treatment with an aqueous acid. The conversion is shown in Flow Diagram I.

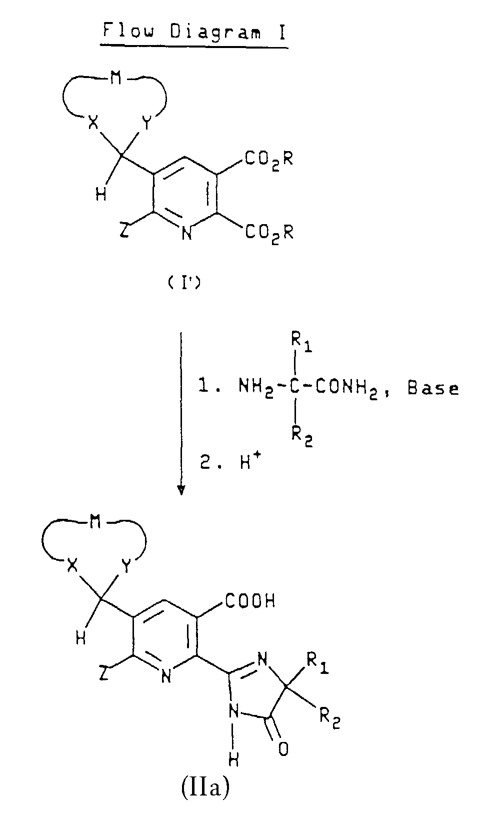

Alternatively, the compounds of the present invention may be utilised to prepare herbicidal agents of formula IIa by saponifing the formula I' pyridinedicarboxylate to obtain the corresponding dicarboxylic acid (formula IIIa), reacting said formula IIIa dicarboxylic acid with acetic anhydride to obtain the corresponding anhydride of formula IVa, reacting the formula IVa anhydride with at least one equivalent of an aminocarboxamide in the presence of a base to yield an intermediate having the structural formula Va and reacting said formula Va intermediate sequentially with a strong base and an aqueous acid to give the herbicidal formula IIa compound as illustrated below in Flow Diagram IIa.

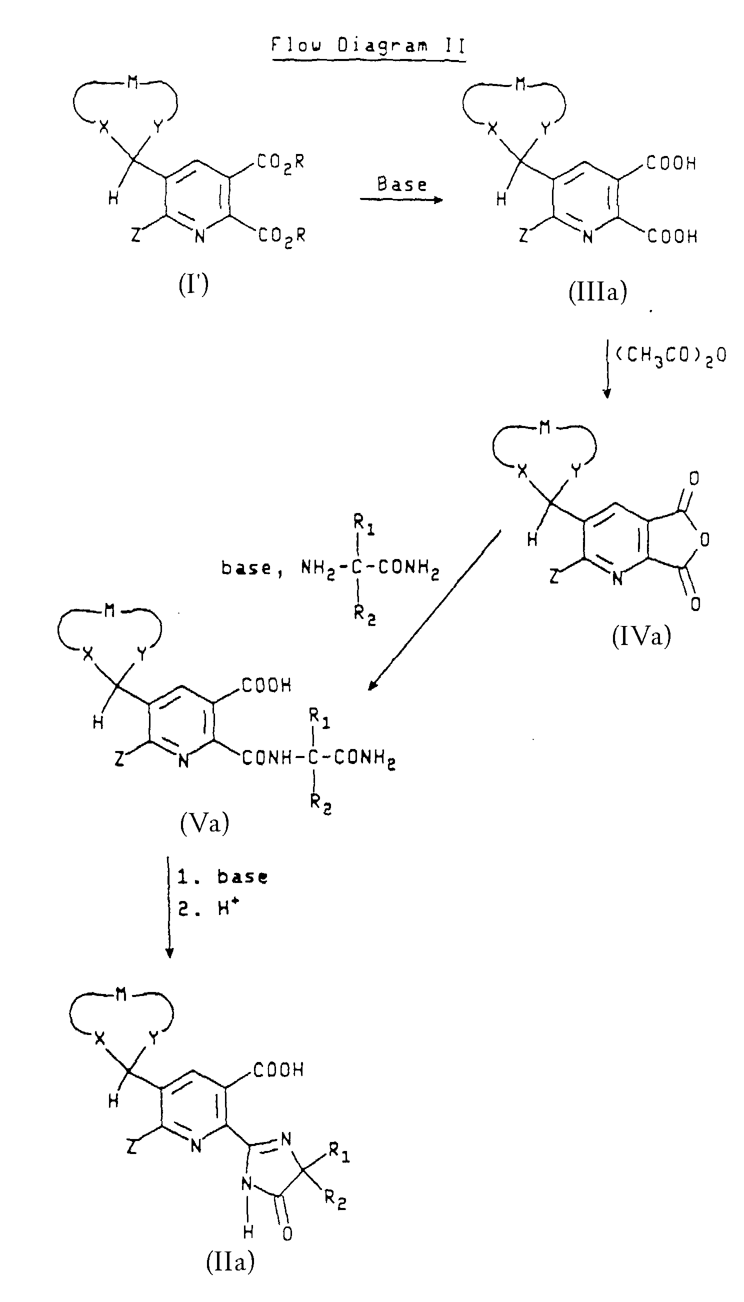

In accordance with the process of the present invention the versatile compounds of formula I' are prepared by the esterification and halogenation of 5-methyl-2,3-pyridinedicarboxylic acid to yield both a 5-(dihalomethyl)-2,3-pyridinedicarboxylate first intermediate having the structural formula VI and a 5-halomethyl)-2,3-pyridinedicarboxylate second intermediate of formula VII, reaction of the formula VI, first intermediate with a (C₁-C₄)alkyl alcohol and at least 2 equivalents of silver nitrate to give a 5-formyl-2,3-pyridinedicarboxylate, 5-di(C₁-C₄)alkyl acetal of formula VIII and reaction of said formula VIII acetal with an agent having the structural formula IX

HX-M-YH (IX)

wherein M, X and Y are as described above for formula I' in the presence of a catalytic amount of an acid and a solvent gives the formula I' 5-heterocyclic-pyridinedicarboxylate compound. Alternatively, the formula VI first intermediate is reacted with at least 2 equivalents of aqueous silver nitrate to yield a 5-formyl-2,3-pyridinedicarboxylate of formula X and the formula X compound is reacted with a formula IX agent as described above to give the desired formula I' compound. similarly, the formula VII 5-(halomethyl)-2,3-pyridinedicarboxylate is readily converted to the formula X 5-formyl-2,3-pyridinedicarboxylate by reacting said formula VII diester with silver tetrafluoroborate in the presence of dimethyl sulfoxide. Additionally, the formula VI first intermediate is directly converted to the desired 5-heterocyclic-pyridine-2,3-dicarboxylate of formula I' by reacting said formula VI compound with a formula IX agent in the presence of silver nitrate and a solvent. The reaction processes are illustrated in Flow Diagram III.

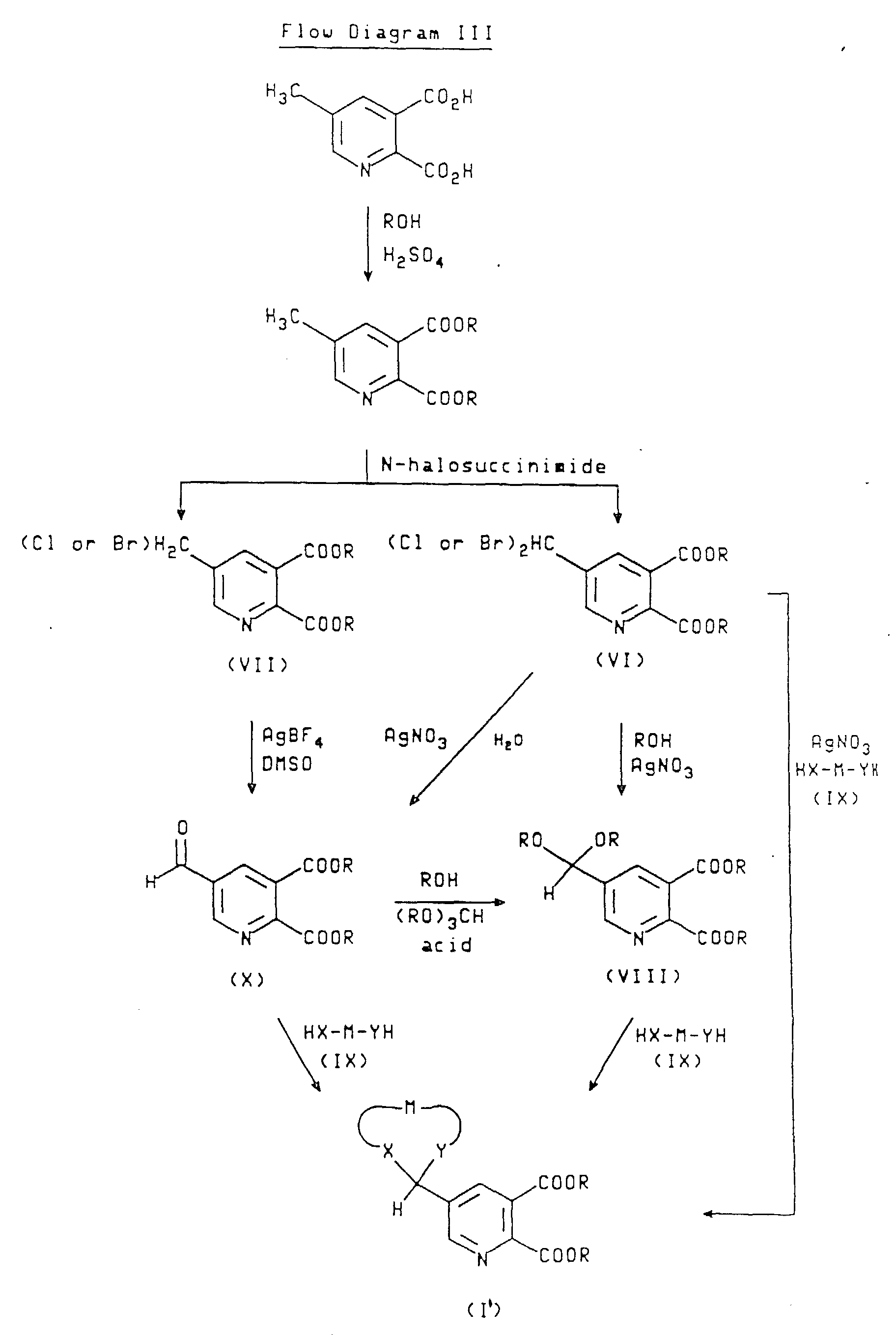

A further process of the invention is the reaction of a 2',5'-diloweralkoxyacetanilide of formula XI with phosphorous oxychloride and dimethylformamide to give a 2-chloro-5,8-diloweralkoxyquinolinecarboxaldehyde of formula XII which is advantageously converted to the formula XIII 5-acetal-6-chloropyridinedicarboxylate intermediate in a single step by the reaction of said formula XII quinoline carboxaldehyde with ozone, at least 2 equivalents of triloweralkylorthoformate and a loweralkyl alcohol in the presence of a mineral acid to yield the formula XIII pyridinedicarboxylate which is converted to the desired formula I'product, wherein Z is chlorine, by the reaction of said formula XIII compound with a formula IX agent as described hereinabove. The formula XII quinolinecarboxaldehyde is also obtained by reacting a formula IV β-anilino-α,β-unsaturated ester with phosphorous oxychloride and dimethylformamide to give the formula XV 2-chloro-3-quinolinecarboxylato and sequentially reacting said formula XV carboxylate with lithium aluminum hydride (LAH) and pyridinium chlorochromate (PCC) to give the corresponding formula XII quinolinecarboxaldehyde. Compounds of formula I'wherein Z is chlorine may be converted to compounds of formula I' wherein Z is hydrogen via catalytic hydrogenation. The above-described processes are illustrated in Flow Diagram IV.

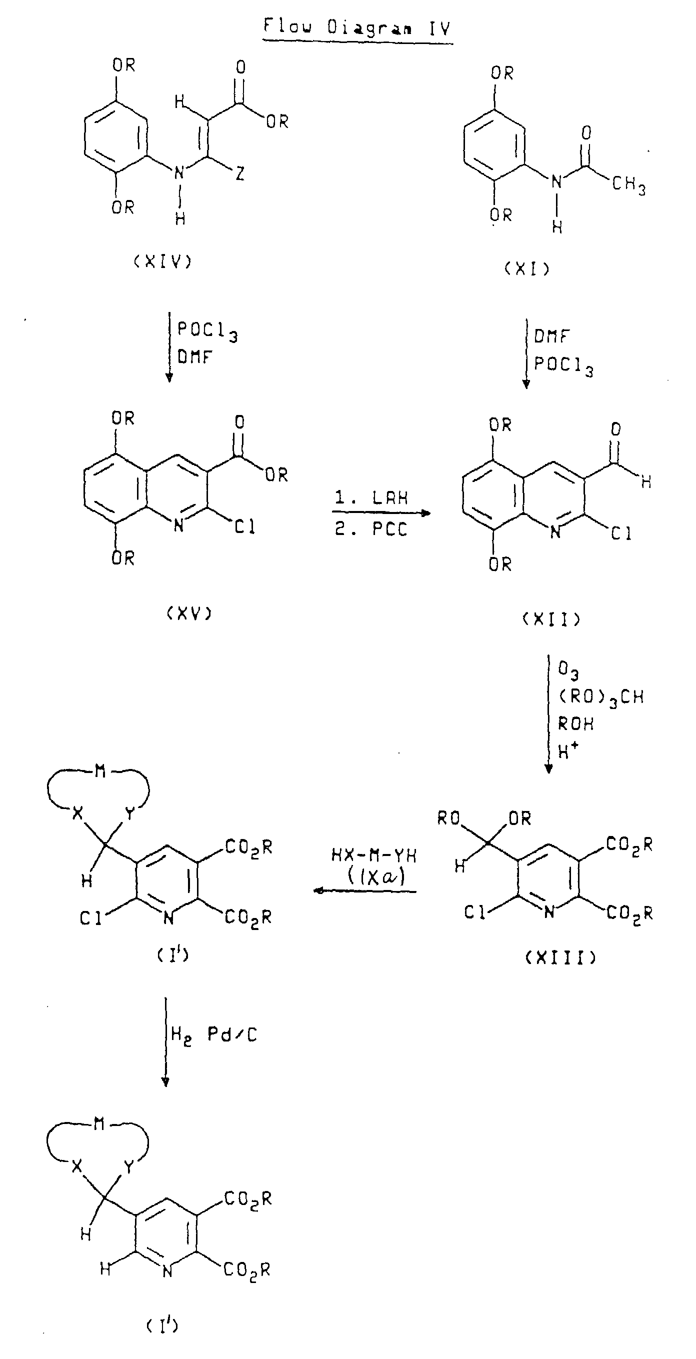

Formula I' compounds of the present invention wherein Z is C₁-C₆ alkoxy are prepared by converting the intermediate acetal of formula XIII to the corresponding 5-formylpyridinedicarboxylate of formula XVI, reacting said formula XVI compound with an alkali metal C₁-C₆ alkoxide to obtain the 6-alkoxypyridine of formula XVII and reacting said formula XVII compound with a formula IX agent as described hereinabove to obtain the desired formula I' compound wherein Z is C₁-C₆ alkoxy. This reaction sequence is illustrated in Flow Diagram V 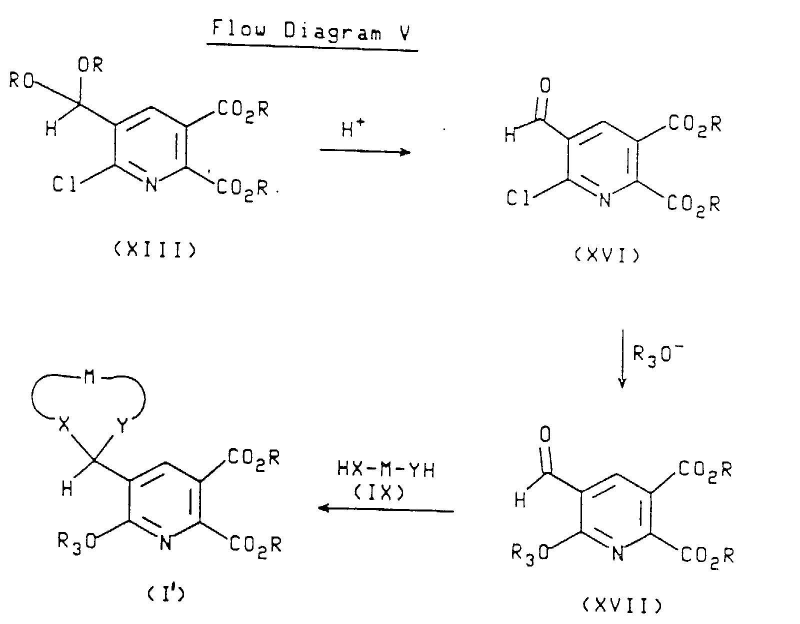 wherein R₃ is C₁-C₆ alkyl.

The 5-heterocyclic 2-(2-imidazolin-2-yl)pyridine compounds of the present invention are effective in the control of undesirable plant species.

The 5-heterocyclic 2-(2-imidazolin-2-yl)pyridine compounds of the present invention are represented by formula Ia: 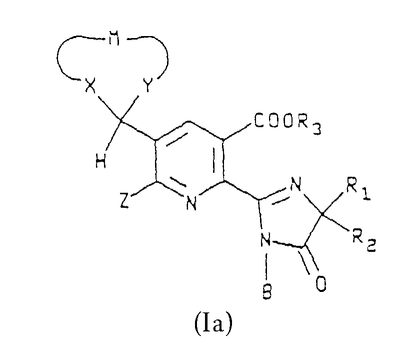 wherein
R₁ is C₁-C₄ alkyl;
R₂ is C₁-C₄ alkyl or C₃-C₆ cycloalkyl and when R₁ and R₂ are taken together with the carbon to which they are attached they may represent C₃-C₆ cycloalkyl;
R₃ is hydrogen,
   C₁-C₆ alkyl optionally substituted with one of the following groups: C₁-C₃ alkoxy, halogen or phenyl,
   C₃-C₆ alkenyl optionally substituted with one of the following groups: C₁-C₃ alkoxy, phenyl or halogen,
   C₃-C₆ alkynyl,
   C₃-C₆ cycloalkyl optionally substituted with C₁-C₃ alkyl or
   a cation of alkali metals, ammonium or organic ammonium;
B is hydrogen or COR₇, provided that when B is COR₇, R₃ is other than hydrogen or a cation;
R₇ is C₁-C₅ alkyl or phenyl optionally substituted with halogen, nitro or methoxy;
X, Y, M, and Z are as described above for formula I';
the N-oxides thereof, when R₃ is not unsaturated alkyl and when M is not alkenylene;
the optical isomers thereof, when R₁ and R₂ represent different substitutents;
the acid addition salts thereof, when R₃ is other than a cation and the tautomers thereof.

A preferred group of 5-heterocyclic 2-(2-imidazolin-2-yl)pyridine compounds that exhibit excellent herbicidal activity one having structural formula Ia as described above and
wherein
R₁ is methyl;
R₂ is isopropyl;
R₃ is hydrogen, C₁-C₆ alkyl or a cation of alkali metals, ammonium or organic ammonium;
B is hydrogen and
Z is hydrogen, C₁-C₄ alkyl, C₁-C₄ alkoxy or halogen.

Another preferred group of 5-heterocyclic 2-(2-imidazolin-2-yl)pyridine compounds of formula I is one wherein
R₁ is methyl;
R₂ is isopropyl;
R₃ is hydrogen, C₁-C₆ alkyl or a cation of alkali metals, ammonium or organic ammonium;
B is hydrogen;
Z is hydrogen;
M is unsubstituted alkylene and
X and Y are oxygen.

The seasonal rotation of crops from one field to another is a common agricultural practice. Replacement crops often vary in their tolerance to the herbicides used to control weeds in the previous crops planted in any given field. This variance in tolerance limits the crops to be rotated and/or the types of herbicides used. However the 5-heterocyclic 2-(2-imidazolin-2-yl)pyridine compounds of the present invention when used in rotational agronomic conditions, demonstrate an improved tolerance for sensitive replacement crop species while retaining highly effective weed control in the presence of the imminent crop species.

Certain 5-heterocyclic pyridine compounds of formula Vb can be prepared by esterfication and halogenation of 5-methyl-2,3-pyridinedicarboxylic acid to yield both a 5-(dihalomethyl)-2,3-pyridinedicarboxylate first intermediate, having the structural formula VIIIa, and a 5-(halomethyl)-2,3-pyridinedicarboxylate second intermediate of formula IXa Reacting the formula VIII first intermediate with a loweralkyl alcohol and silver nitrate yields a 5-formyl-2,3-pyridinedicarboxylate, 5-diloweralkyl acetal having the structural formula X. Alternatively, reacting the formula VIIIa first intermediate with water and silver nitrate yields a 5-formyl-2,3-pyridinedicarboxylate of formula XIa. Reacting a formula IXa 5-halomethyl-2,3-pyridinedicarboxylate with dimethyl sulfoxide and silver tetrafluoroborate also yields the desired formula XIa compound. Said formula XIa compound can be converted to the formula Xa diloweralkyl acetal by reaction with a loweralkyl alcohol, loweralkylorthoformate and an acid. Both the formula XIa compounds and the formula Xa diloweralkyl acetals are converted to 5-heterocyclic pyridine compounds of formula Vb by reaction with the appropriate reagent having the structural formula IXa.

HX-M-YH (IXa)

wherein M, X and Y are as described for formula I'. Reacting a 5-(dihalomethyl)-2,3-pyridinedicarboxylate with silver nitrate and the appropriate formula VII reagent yields a 5-heterocyclic-2,3-pyridinedicarboxylate of formula Vb. The above-described reaction schemes are illustrated in Flow Diagram Ia. 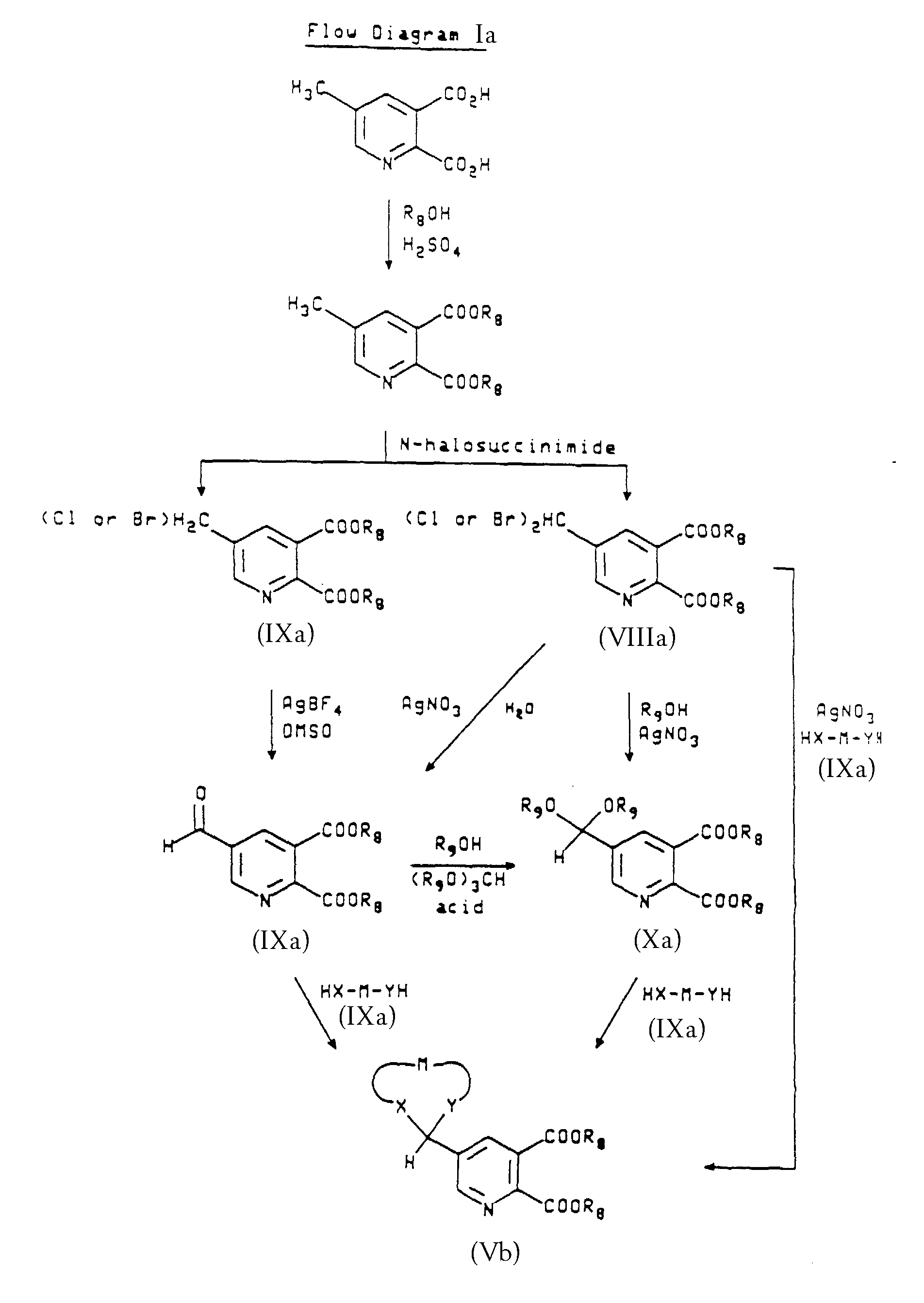 In flow diagram Ia, X, Y, and M are as described for formula I' and R₈ and R₉ are C₁-C₄ alkyl.

Formula Ia compounds of the present invention may be prepared by the reaction of a formula Vb 5-heterocyclic-2,3-pyridinedicarboxylate with an aminocarboxamide and a strong base, such as an alkali metal butoxide followed by acidification to pH 2.0-4.0, to give the desired formula Ia compound as illustrated below in Flow Diagram IIa.

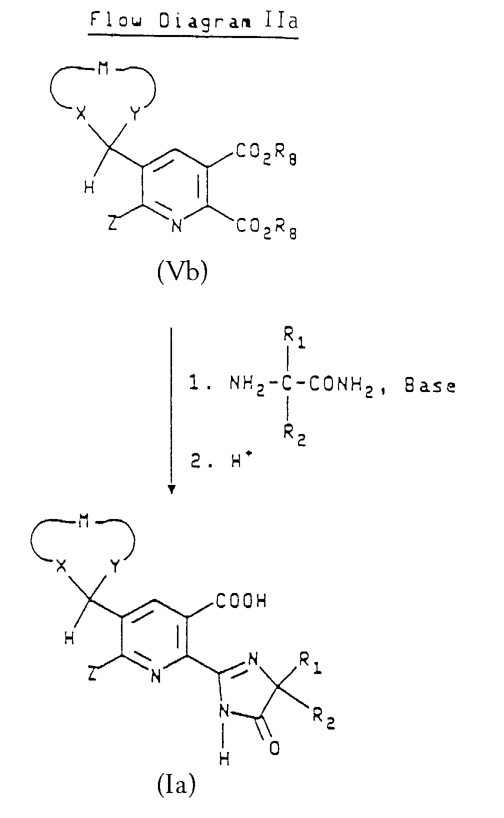

Alternatively, formula Ia 5-heterocyclic 2-(2-imidazolin-2-yl)pyridine compounds may be prepared by saponification of a formula Va pyridinedicarboxylate to give the corresponding dicarboxylic acid (formula XVII), reaction of said formula XVII dicarboxylic acid with acetic anhydride to give the corresponding anhydride of formula XVIII, reaction of the formula XVIII anhydride with at least an one equivalent of an aminocarboxamide to yield a compound having the structural formula XIX and reaction of the formula XIX compound with a base followed by acidification to pH 2.0-4.0 to yield the desired formula Ia compound as illustrated below in Flow Diagram IIIa.

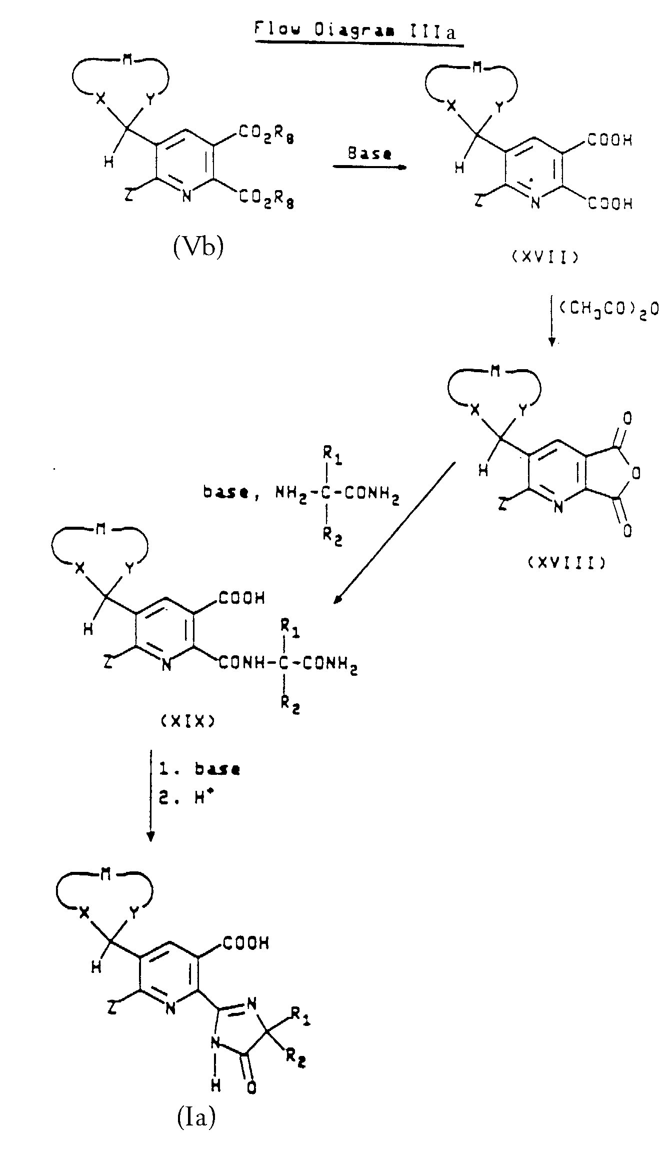

Certain 5-heterocyclic 2-(2-imidazolin-2-yl)-pyridine compounds of formula Ia can be prepared by reacting a formula X 5-formyl-2,3-pyridinedicarboxylate, 5-diloweralkylacetal with an aminocarboxamide having the structure 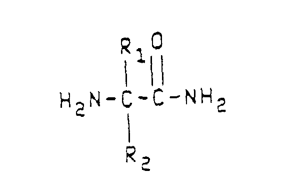 in an inert solvent and in the presence of a strong base, such as an alkali metal butoxide, followed by acidification to pH 2.0-4.0 to yield a compound having the structural formula XX. Reacting the thus-formed formula XX compound with the appropriate formula VII reagent yields the desired formula Ia compound as illustrated in Flow Diagram IVa.

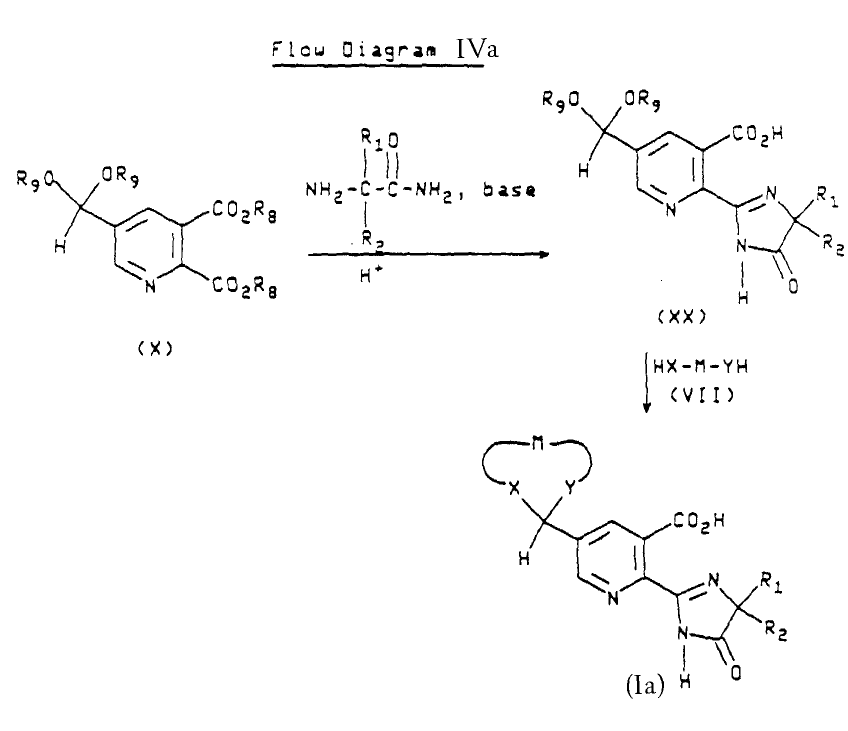

Alternatively, the formula XX intermediate can be reacted with a mineral acid in the presence of an inert organic solvent to give the corresponding 5-formyl-2-(imidazolin-2-yl)nicotinic acid having the structural formula XXI. Reacting the formula XXI compound with the appropriate formula VII reagent yields the desired formula Ia compound as illustrated below in Flow Diagram Va.

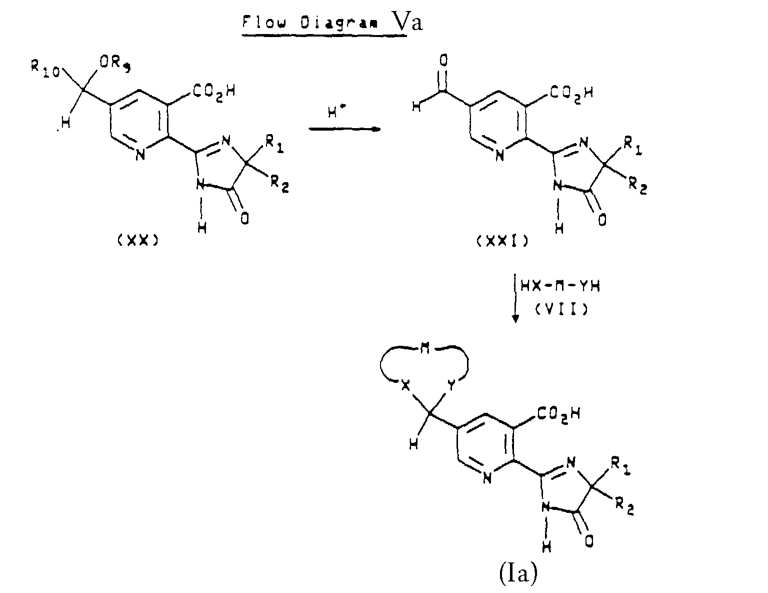

Formula II 5H-imidazo[1',2':1,2]pyrrolo-[3,4-b]pyridine-2(3H), 5-dione compounds of the present invention may be prepared by the reaction of a formula Ia 5-heterocyclic 2-(2-imidazolin-2-yl))pyridine compound, wherein R₃ is hydrogen, with an acid anhydride as shown below:

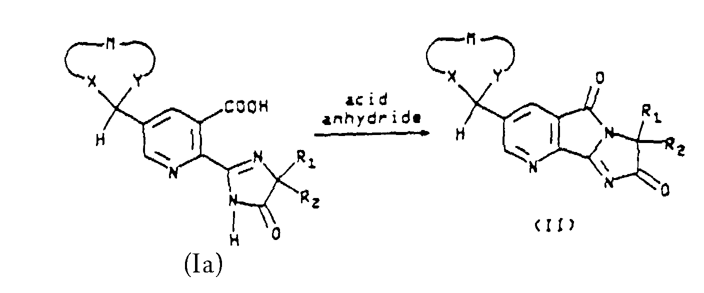

Formula III 5H-imidazo[1',2':1,2]pyrrolo-[3,4-b]pyridine-3(2H), 5-dione compounds of the present invention can be prepared by the reaction of formula Ia 5-heterocyclic 2-(2-imidazolin-2-yl)pyridine compounds, wherein R₃ is hydrogen, with dicyclohexylcarbodiimide in the presence of a solvent as shown below.

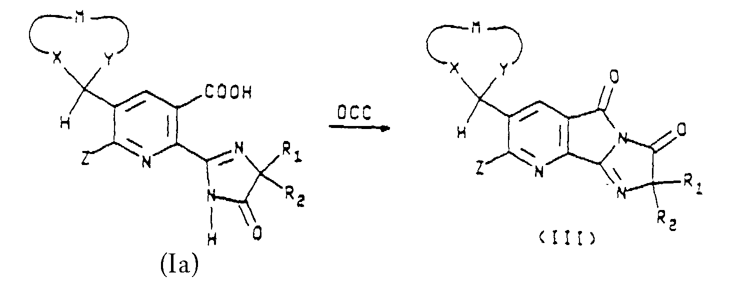

Certain formula Ia 5-heterocyclic 2-(2-imidazolin-2-yl)pyridine compounds wherein R₃ is not hydrogen can be prepared by reacting the formula II or formula III dione with the appropriate alkoxide as shown below.

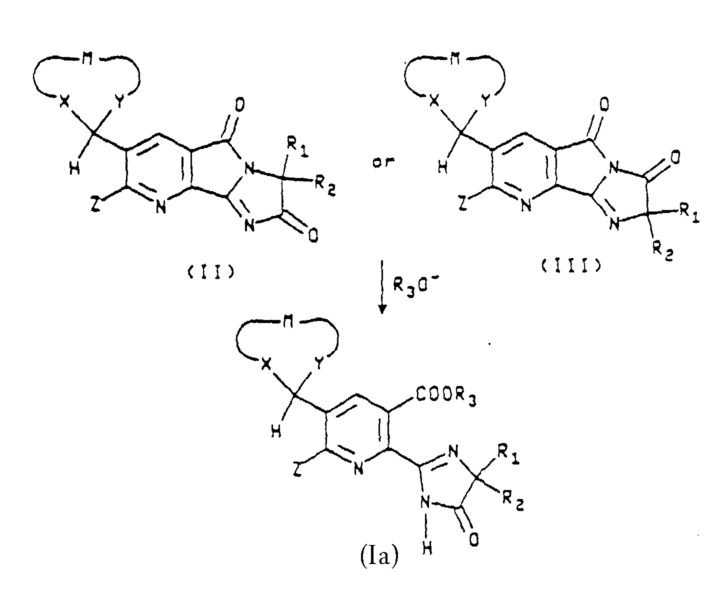

The formula Ia 5-heterocyclic 2-(2-imidazolin-2-yl)pyridine compounds of the present invention are effective herbicidal agents useful for the control of a wide variety of monocotyledonous and dicotyledonous plants. These compounds are effective for controlling weeds native to both dry land and wet land areas. The compounds are effective in controlling the above-said plants when applied to the foliage thereof or to soil or water containing seeds or other propagating organs of said plants such as stolons, tubers or rhizomes, at rates of from about 0.016 to 2.0 kg/ha.

Since the formula Ia 5-heterocyclic 2-(2-imidazolin-2-yl)pyridine compounds, wherein R₃ is a salt-forming cation, are water soluble, these compounds can simply be dispersed in water and applied as a dilute aqueous spray to the foliage of plants or to soil containing propagating organs thereof. These salts also lend themselves to formulation as flowable concentrates.

The formula Ia 5-heterocyclic 2-(2-imidazolin-2-yl)pyridines can also be formulated as emulsifiable concentrates, wettable powders, granular formulations, flow concentrates and the like.

A typical emulsifiable concentrate can be prepared by dissolving about 5% to 25% by weight of the active ingredient in about 65% to 90% by weight of N-methylpyrrolidone, isophorono, butyl cellosolve, methylacetate or the like and dispersing therein about 5% to 10% by weight of a nonionic surfactant such as an alkylphenoxy polyethoxy alcohol. This concentrate is dispersed in water for application as a liquid spray.

Wettable powders can be prepared by grinding together about 20% to 45% by weight of a finely divided carrier such as kaolin, bentonite, diatomaceous earth, attapulgite, or the like, 45% to 80% by weight of the active compound, 2% to 5% by weight of a dispersing agent such as sodium lignosulfonate and 2% to 5% by weight of a nonionic surfactant, such as octylphenoxy polyethoxy ethanol, nonylphenoxy polyethoxy ethanol or the like.

Where soil treatments are involved, the compounds may be prepared and applied as granular products. Preparation of the granular product can be achieved by dissolving the active compound in a solvent such as methylene chloride, N-methylpyrrolidone or the like and spraying the thus prepared solution on a granular carrier such as corncob grits, sand, attapulgite, kaolin or the like.

The granular product thus prepared generally comprises about 3% to 20% by weight of the active ingredient and about 97% to 80% by weight of the granular carrier.

A typical flowable liquid can be prepared by admixing about 40% by weight of the active ingredient with about 2% by weight of a gelling agent such as bentonite, 3% by weight of a dispersing agent such as sodium lignosulfonate, 1% by weight of polyethylene glycol and 54% by weight of water.

In order to facilitate a further understanding of the invention, the following examples are presented primarily for the purpose of illustrating more specific details thereof. The invention is not to be deemed limited thereby except as defined in the claims. IR and NMR designate infrared and proton nuclear magnetic resonance, respectively.

### EXAMPLE 1

### Preparation of Dimethyl 5-(dibromomethyl)-2,3-pyridinedicarboxylate And Dimethyl 5-(bromomethyl)-2,3-pyridinedicarboxylate

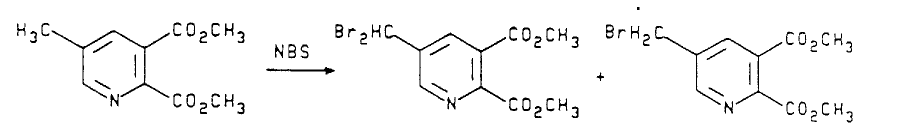

N-bromosuccinimide (83.06 g, 0.46 mol) and benzoyl peroxide (11.28 g, 0.046 mol) are added, in 5 portions over a 7 hour period, to a solution of 5-methyl-2,3-pyridinedicarboxylic acid, dimethyl ester (42.5 g, 0.20 mol) in carbon tetrachloride heated at reflux temperature. The reaction mixture is heated at reflux temperature for 2 hours, cooled to room temperature, filtered and the solids are washed with methylene chloride. The filtrate is washed sequentially with 5% sodium metabisulfite solution and 5% sodium bicarbonate solution, dried over anhydrous magnesium sulfate and concentrated in vacuo to give an oil. The oil is chromatographed on silica gel using 16% ethyl acetate in hexanes and 33% ethyl acetate in hexanes as eluant to give dimethyl 5-(dibromomethyl)-2-3-pyridinedicarboxylate as a white solid, (47.2 g, 64%), mp 61-65°C, identified by IR and NMR spectral analyses and dimethyl 5-(bromomethyl)2,3-pyridinedicarboxylate (11.4, 20%) as a clear oil, identified by IR and NMR spectral analyses.

### EXAMPLE 2

### Preparation of Ethyl 3-(2,5-dimethylanilino)crotonate

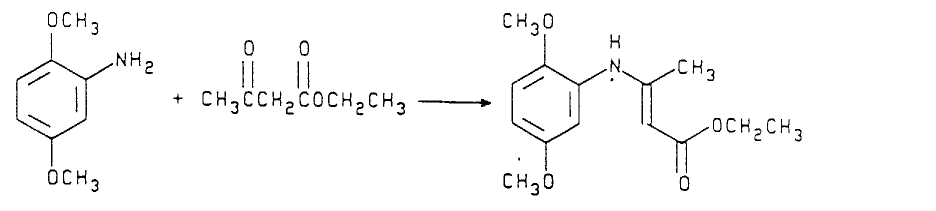

A solution of 2,5-dimethoxyaniline (80 g, 0.52 mol) and ethyl acetoacetate (66 mL, 0.52 mol) in toluene (200 mL) and acetic acid (2 mL) is stirred at reflux temperature for 6 hours. The reaction mixture is concentrated in vacuo and the crude product is purified by chromatography using silica gel and ethyl acetate in hexanes as eluant to yield the title compound (7.32 g, 12%) as a white solid, mp 67°C, identified by IR and NMR spectral analyses.

### EXAMPLE 3

### Preparation of Ethyl 5,8-Dimethoxy-2-methylquinoline-3-carboxylate

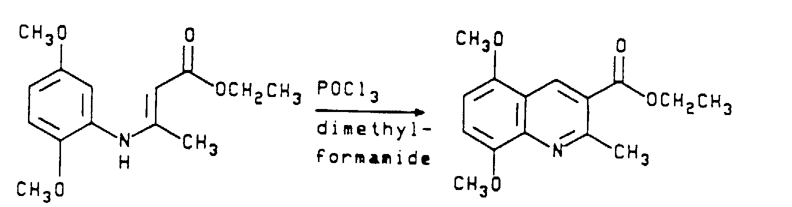

To a 0°C solution of dimethylformamide (7.5 mL, 0.097 mol) in dichloroethane (20 mL) is added dropwise phosphorous oxychloride (9.0 mL, 0.097 mol). After stirring for 3.5 hours at room temperature, a solution of ethyl 3-(2,5-dimethoxyanilino)crotonate (25.8 g, 0.097 mol) in dichloroethane (130 mL) is added dropwise at 25°C. The resulting solution is heated at reflux temperature for 3 hours. After cooling to room temperature, the reaction is quenched by the addition of an aqueous ammonium chloride solution. Methylene chloride (200 mL) is added and the layers are separated. The organic layer is dried over anhydrous magnesium sulfate and concentrated in vacuo to give a residue. The residue is purified by chromatography using silica gel and ethyl acetate in hexanes as eluant to yield the title compound (34 g, 100%) as a yellow solid identified by IR and NMR spectral analyses.

### EXAMPLE 4

### Preparation of 5,8-Dimethoxy-3-formyl-2-methylquinoline

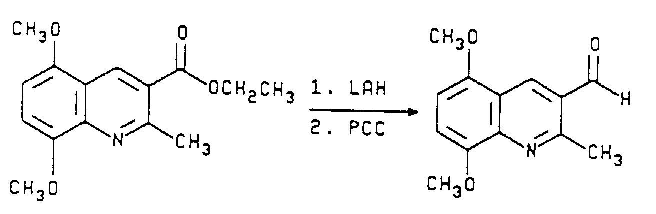

A solution of ethyl 5,8-dimethoxy-2-methylquinoline-3-carboxylate (4.0 g, 0.0145 mol) in tetrahydrofuran is added to a slurry of lithium aluminum hydride (0.72 g, 0.019 mol) in ether. The reaction is stirred for 16 hours at 50°C. After cooling, the reaction is quenched by the sequential addition of water and 15% aqueous sodium hydroxide. Additional ether and anhydrous magnesium sulfate are added and the reaction mixture is filtered. The product is purified by chromatography using silica gel and ethyl acetate in hexanes as eluant. The resulting alcohol (1.50 g) is dissolved in methylene chloride and added to a slurry of pyridinium chlorochromate in methylene chloride. The resulting solution is stirred for 3 hours at room temperature. Diatomaceous earth is added, the reaction mixture is diluted with ether and filtered. The filtrate is concentrated in vacuo and the product purified by chromatography using silica gel and ethyl acetate in hexanes as eluant to yield the title compound (0.10 g, 5%) as a yellow solid, identified by IR and NMR spectral analysis.

### EXAMPLE 5

### Preparation of 2-Chloro-5,8-dimethoxy-3-quinolinecarboxaldehyde

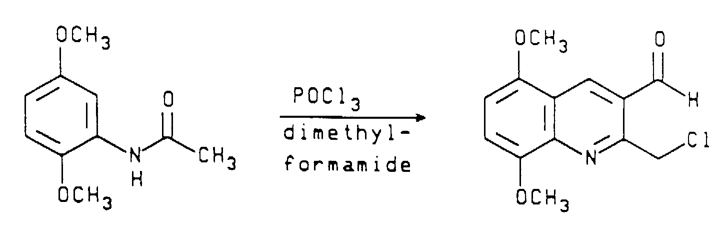

To a 0°C solution of dimethylformamide (80 mL, 1.04 mol) is added phosphorus oxychloride (250 mL, 2.7 mol). The reaction mixture is heated at 50°C for 45 minutes, then solid 2',5'-dimethoxyacetanilide (93.62 g, 0.48 mol) is added. The reaction mixture is heated for 4.5 hours at 75°C, cooled to room temperature, quenched with water and ice, stirred for 3 hours and 30 minutes and filtered to give a solid. The solid is recrystallized from ethyl acetate to yield the title compound as a yellow powder (13.64 g, 11%), mp 177-178°C, identified by IR and NMR spectral analyses.

### EXAMPLE 6

### Preparation of Dimethyl 6-chloro-5-formyl-2,3-pyridinedicarboxylate, 5-(dimethyl acetal)

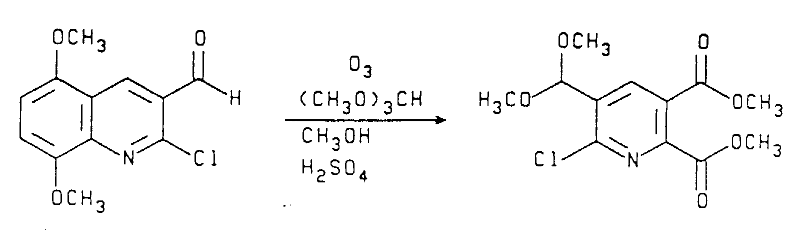

To a solution of 2-chloro-5,8-dimethoxy-3-quinolinecarboxaldehyde (27.32 g, 0.11 mol) and methanol is added trimethylorthoformate (86 mL, 0.785 mol) and concentrated sulfuric acid (2 mL). Ozone is then bubbled through the reaction mixture for a total of 11 hours. Concentration in vacuo yields an oil which is dissolved into ether, washed sequentially with saturated sodium bicarbonate solution, saturated sodium metabisulfite solution and brine. The solution is dried over anhydrous magnesium sulfate and concentrated in vacuo to yield the title compound as an orange oil (20.13 g, 61%), identified by IR and NMR spectral analyses.

Following the above procedure and employing the appropriately substituted quinoline carboxaldehyde the following compounds are obtained.

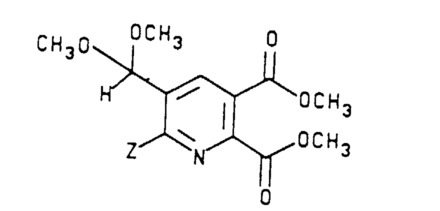

| Z | mp |
|---|---|
| CH₃ | oil |
| Cl | oil |
| CH₂CH₃ | |
| OCH₃ | |
| CH₂CH₃ | |
| Br | |

### EXAMPLE 7

### Preparation of Dimethyl 5-formylpyridine-2,3-dicarboxylate

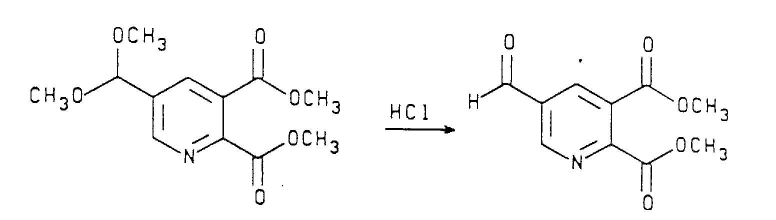

A solution of dimethyl 5-dimethoxymethylpyridine-2,3-dicarboxylate (6.57 g, 0.024 mol) in 2N HCl (15 mL) and tetrahydrofuran (25 mL) is stirred at reflux temperature for 2 hours. The tetrahydrofuran is removed in vacuo, the aqueous solution is diluted with water (to 30 mL) and extracted with methylene chloride. The combined methylene chloride extracts are dried over anhydrous magnesium sulfate and concentrated in vacuo to yield the title compound (4.51 g, 82%) as a yellow solid, mp 75°-77°C, identified by IR and NMR spectral analyses.

Using the above procedure and employing dimethyl 5-dimethoxymethyl-6-chloropyridine-2 , 3-dicarboxylate as starting material affords dimethyl 5-formyl-6-chloropyridine-2,3-dicarboxylate as an oil.

### EXAMPLE 8

### Preparation of Dimethyl 5-formyl-6-methoxypyridine-2,3-dicarboxylate

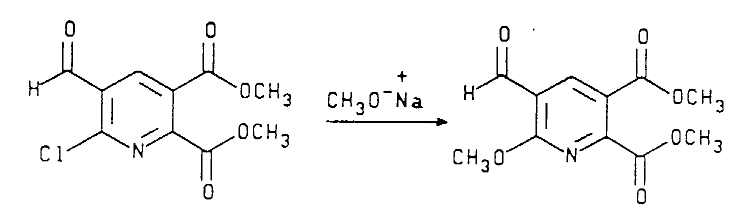

A solution of dimethyl 6-chloro-5-formylpyridine-2,3-dicarboxylate (1.0 g, 0.0039 mol) in methanol (10 mL) is added to sodium methoxide (0.42 g, 0.0078 mol) in methanol (2 mL). The resulting mixture is stirred for 15 hours at 90°C. After cooling to room temperature, the reaction mixture is acidified to pH 3.0 with acetic acid. The reaction is poured into methylene chloride, washed with water, dried over anhydrous magnesium sulfate and concentrated in vacuo to yield the title compound (0.62 g, 63%) as an orange oil, identified by IR and NMR spectral analyses.

### EXAMPLE 9

### Preparation of Dimethyl 5-formyl-2,3-pyridinedicarboxylate, 5-dimethyl acetal

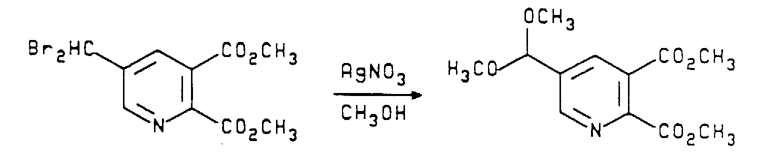

Silver nitrate (134.49 g, 0.816 mol) is added to a solution of 5-(dibromomethyl)-2,3-pyridinedicarboxylic acid, dimethyl ester (146.01 g, 0.398 mol) and methanol. The reaction mixture is stirred for 30 minutes at 25°C then for 1 hour and 30 minutes at reflux temperature. The reaction mixture is cooled to room temperature, filtered through a pad of diatomaceous earth and the diatomaceous earth is rinsed with methanol. The filtrate is concentrated in vacuo to give an oil. The oil is dissolved in methylene chloride, washed with 5% sodium bicarbonate solution, dried over anhydrous magnesium sulfate and concentrated in vacuo to give an oil. The residue oil is chromatographed using silica gel, 16% ethyl acetate in hexanes and 50% ethyl acetate in hexanes as eluant to yield the title compound as a clear oil, (98.39 g, 92%), identified by IR and NMR spectral analyses.

### EXAMPLE 10

### Preparation of Dimethyl 5-dimethoxymethylpyridine-2,3-dicarboxylate

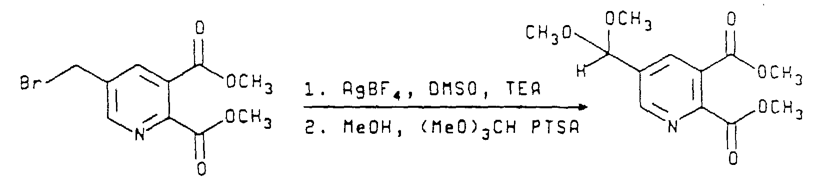

To a solution of dimethyl 5-bromomethylpyridine-2,3-dicarboxylate (10.55 g, 0.0366 mol) in dimethylsulfoxide (50 mL) is added solid silver tetrafluoroborate (8.91 g, 0.0458 mol). The resulting mixture is stirred for 1.5 hours at 80°C-90°C, then triethylamine (6.75 mL 0.458 mol) is added and the reaction mixture is stirred for an additional 1 hour at 80°C. The reaction is filtered through a pad of diatomaceous earth and the diatomaceous earth is rinsed with methanol. The filtrate is concentrated in vacuo and the crude product is dissolved in methanol (100 mL) and trimethylorthoformate (15 mL). A catalytic amount of para-toluenesulfonic acid is added and the reaction mixture is stirred at reflux temperature for 4.5 days. The reaction mixture is concentrated in vacuo and diluted with H₂O and passed through a pad of C-18 silica gel. Elution with methanol in water affords a crude product, which is purified by chromatography on silica gel using ethyl acetate in hexanes as eluant to yield the title compound (1.96 g, 20%) as a clear oil, identified by IR and NMR spectral analyses.

### EXAMPLE 11

### Preparation of Dimethyl 5-formylpyridine-2,3-dicarboxylate

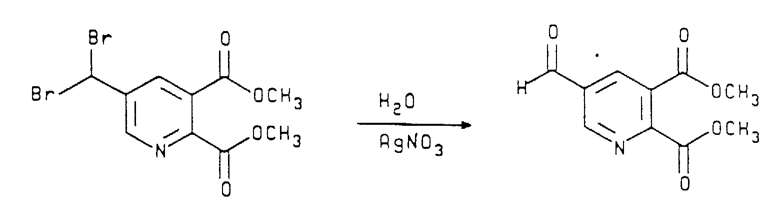

To a solution of dimethyl 5-dibromomethylpyridine-2,3-dicarboxylate (8.32 g, 0.0227 mol) in dioxane (60 mL) and water (20 mL) is added silver nitrate (7.85 g, 0.0476 mol). The resulting slurry is heated at reflux for 3 hours. The reaction mixture is cooled to room temperature, filtered through a pad of diatomaceous earth and the diatomaceous earth is rinsed with tetrahydrofuran. The filtrate is concentrated in vacuo to remove the dioxane and tetrahydrofuran. The resultant aqueous solution is made basic by the addition to saturated aqueous sodium bicarbonate (100 mL) and extracted with methylene dichloride. The combined extracts are dried over anhydrous magnesium sulfate and concentrated in vacuo to give a residue. Chromatography using silica gel and 33% ethyl acetate in hexanes as eluant yields the title compound as a white solid, (4.31 g, 85%) mp 75°C-77°C, identified by IR and NMR spectral analyses.

### EXAMPLE 12

### Preparation of 5-Formyl -2-(4-isopropyl-4-methyl-5-oxo-2-imidazolin-2-yl)nicotinic acid, 5-(dimethyl acetal)

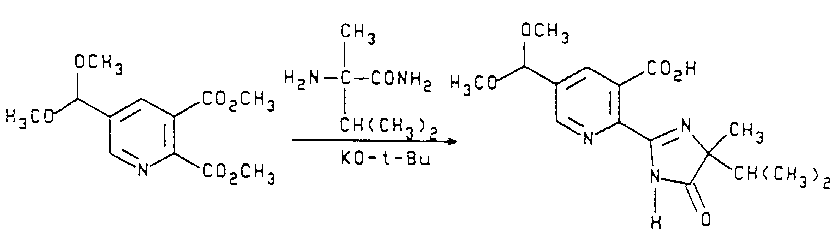

Potassium tert-butoxide (26.55 g, 0.236 mol) is added portionwise to a stirred solution of 5-formyl-2,3-pyridinedicarboxylic acid, dimethyl ester, 5-dimethyl acetal (30.31 g, 0.112 mol) and 2-amino-2,3-dimethylbutyramide (14.67 g, 0.113 mol) in toluene, the reaction mixture exotherms to about 40°C. The reaction mixture is heated at 80°C for 1 hour, cooled to room temperature and diluted with water. The layers are separated and the aqueous solution is acidified to pH 3.0 with concentrated hydrochloric acid. The aqueous solution is then extracted with methylene chloride and the combined methylene chloride extracts are dried over anhydrous magnesium sulfate and concentrated in vacuo to give the title compound as a tan solid, (32.12 g, 85%), mp 135-139°C, identified by IR and NMR spectral analyses.

### EXAMPLE 13

### Preparation of Dimethyl 6-chloro-5-(1,3-dioxolan-2-yl)-2,3-pyridinedicarboxylate

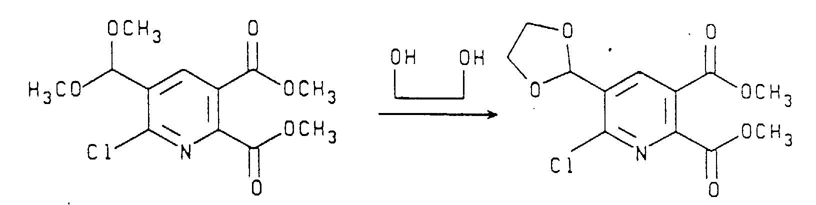

Ethylene glycol (14.3 mL, 0.255 mol), and a catalytic amount of p-toluenesulfonic acid monohydrate is added to a solution of 6-chloro-5-formyl-2,3-pyridinedicarboxylic acid, dimethyl ester, 5-(dimethyl acetal) (15.46 g, 0.051 mol) and toluene. The reaction mixture is heated at reflux temperature for 3 hours then cooled to room temperature. Ether is added and the mixture is washed with saturated sodium bicarbonate solution, dried over anhydrous magnesium sulfate and concentrated in vacuo to give the title compound as an orange oil (14.27 g, 93%), identified by IR and NMR spectral analyses.

### EXAMPLE 14

### Preparation of Dimethyl 5-(1,3-dioxolan-2-yl)-2,3-pyridinedicarboxylate

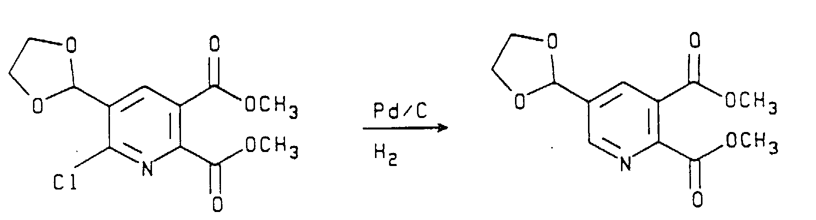

A solution of 6-chloro-5-(1,3-dioxolan-2-yl)-2,3-pyridinecarboxylic acid, dimethyl ester (9.16 g, 0.03 mol) in deoxygenated methanol is added to a Parr flask containing wet palladium on activated carbon and sodium acetate. The flask is placed in a hydrogenator where the reaction mixture absorbs hydrogen. The reaction mixture is then filtered through diatomaceous earth and the flask and diatomaceous earth are rinsed with methanol. The filtrate is concentrated in vacuo and the residual oil is partitioned between methylene chloride and water. The layers are separated and the organic layer is washed with saturated sodium bicarbonate solution, dried over anhydrous magnesium sulfate and concentrated in vacuo to give the title compound as an orange oil (3.53 g, 44%), identified by IR and NMR spectral analyses.

### EXAMPLE 15

### Preparation of Dimethyl 5-(1,3-Dioxepan-2-yl)-2,3-pyridinedicarboxylate

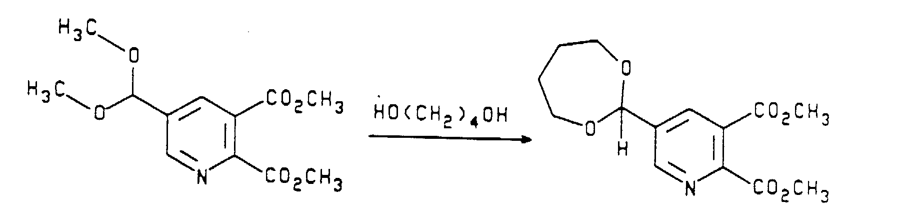

A solution of 5-formyl-2,3-pyridinedicarboxylic acid, dimethyl ester, 5-dimethyl acetal (1.3 g, 0.0048 mol), a catalytic amount of p-toluenesulfonic acid, 1,4-butanediol and toluene is heated at reflux temperature for 2 hours and 30 minutes. The reaction mixture is cooled to room temperature, made basic with sodium bicarbonate and concentrated in vacuo to give a liquid. The liquid is partitioned between methylene chloride and 5% sodium bicarbonate solution. The layers are separated and the aqueous layer is extracted with methylene chloride, the combined methylene chloride extracts are dried over anhydrous magnesium sulfate and concentrated in vacuo to give an oil. The oil is chromatographed using silica gel and hexanes/ethyl acetate (2:1 to 1:1) as eluant to give the title compound as a clear oil (0.88 g, 62%), identified by IR and NMR spectral analyses.

Following the above procedure and employing the appropriately substituted 2,3-pyridinecarboxylic acid, dimethyl ester and the appropriate diol or 2-mercaptoethanol, the compounds shown below are obtained.

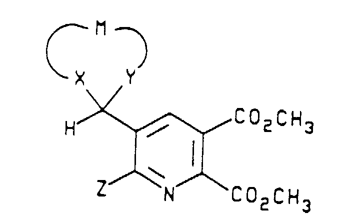

| X | Y | M | Z |
|---|---|---|---|
| O | O | C₄H₈ | H |
| S | O | C₂H₄ | H |
| O | O | C₂H₄ | OCH₃ |
| O | O | C₂H₄ | CH₃ |

### EXAMPLE 16

### Preparation of Dimethyl 5-(1,2-dimethyl-3-diaziridinyl-2,3-pyridinedicarboxylate

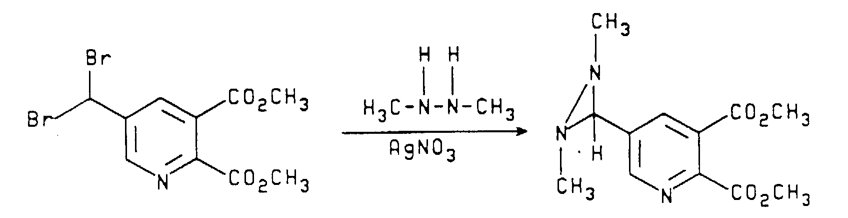

5-(Dibromomethyl)-2,3-pyridinedicarboxylic acid, dimethyl ester (2.0 g, 0.00545 mol), silver nitrate (1.85 g, 0.0109 mol) and 1,2-dimethylhydrazine dihydrochloride salt in pyridine is heated from 25°C to 100°C over 50 minutes, then at 100°C for 1 hour and 45 minutes. Concentration in vacuo gives a liquid that is triturated with water and methylene chloride and filtered. The layers are separated and the aqueous layer is extracted with methylene chloride. The combined methylene chloride extracts are dried over anhydrous magnesium sulfate and concentrated in vacuo to give an oil. The oil is chromatographed using silica gel and hexanes/ethyl acetate (2:1 to 4:3 to 1:1) as eluant to give the title compound as a yellow oil (0.48 g, 33%), identified by IR and NMR spectral analyses.

### EXAMPLE 17

### Preparation of Dimethyl 5-(1,3-dithiolan-2-yl)-2,3-pyridinedicarboxylate

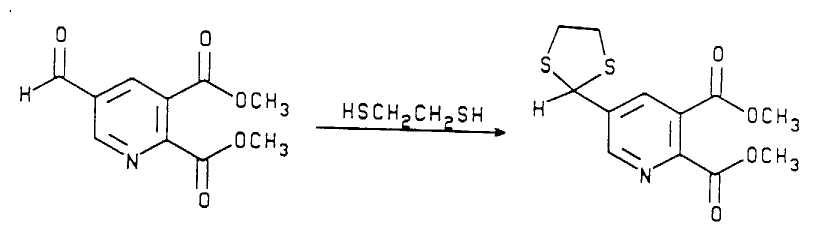

A solution of dimethyl 5-formylpyridine-2,3-dicarboxylate (0.73 g, 0.00327 mol), ethanedithiol (0.357 mL, 0.00425 mol) and a catalytic amount of para-toluenesulfonic acid in chloroform (20 mL) is heated at reflux for 5.5 hours. During this time, water is removed by placement of an addition funnel containing 3 angstrom molecular sieves in between the reaction flask and the reflux condensor. After cooling, the reaction is diluted with methylene chloride (20 mL) and is washed with 10% aqueous sodium carbonate and saturated sodium chloride solutions. The organic solution is dried over anhydrous magnesium sulfate and concentrated in vacuo to give a residue. The residue is purified by chromatography using silica gel and 33% ethyl acetate in hexanes as eluant to yield the title compound as a clear oil (0.66 g, 67%), identified by IR and NMR spectral analyses.

### EXAMPLE 18

### Preparation of Dimethyl 5-(1-acetyl-3-methyl-2-imidazolidinyl)pyridine-2,3-dicarboxylate

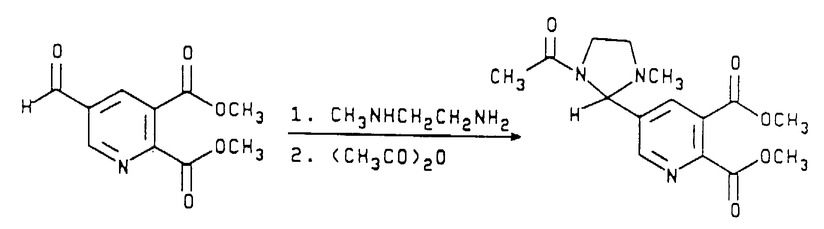

A solution of dimethyl 5-formylpyridine-2,3-dicarboxylate (0.75 g, 0.0036 mol), N-methylethylene diamine (0.38 mL, 0.0044 mol) and a catalytic amount of para-toluenesulfonic acid in toluene (10 mL) is heated at reflux temperature for 3 hours. During this process, water is removed by placement of an addition funnel containing 3 angstrom molecular sieves between the reaction flask and the reflux condensor. The reaction mixture is concentrated in vacuo, dissolved in methylene chloride (30 mL) and is washed with saturated aqueous sodium bicarbonate solution. The organic solution is dried over anhydrous magnesium sulfate and concentrated in vacuo to give a residue. The residue is dissolved in pyridine (10 mL) at 0°C and treated with acetic anhydride (0.34 mL, 0.0036 mol). The reaction mixture is stirred for 1 hour at 0°C and for 66 hours at room temperature. The pyridine is removed in vacuo to yield an oil. The oil is dissolved in methylene chloride and washed with saturated aqueous sodium bicarbonate. The organic solution is dried over anhydrous magnesium sulfate, concentrated in vacuo and purified by chromatography using silica gel and 10% triethylamine in ethyl acetate as eluant to yield the title compound (0.66 g, 56%) as a yellow oil, identified by IR and NMR spectral analyses.

using the above procedure and substituting the appropriate diamine or amino alcohol and the appropriate acylating reagent, the following compounds are obtained.

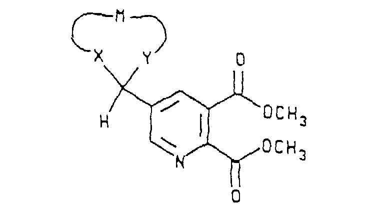

| X | Y | M |
|---|---|---|
| NCOCH₃ | NCH₃ | (CH₂)₂ |
| NCOCH₃ | O | (CH₂)₂ |
| NCO₂CH₃ | O | (CH₂)₂ |
| NCO₂CH₂CH₃ | O | (CH₂)₂ |
| NCON(CH₃)₂ | O | (CH₂)₂ |
| NCONHCH(CH₃)₂ | O | (CH₂)₂ |
| NCOCH(CH₃)₂ | O | (CH₂)₂ |
| NCO₂CH₂CH₃ | O | (CH₂)₃ |
| NSO₂CH₃ | O | (CH₂)₂ |

Using the methods described above, the following formula I' compounds may be prepared.

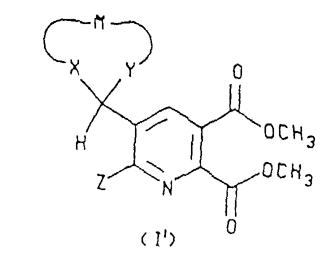

| X | Y | M | Z | mp°C |
|---|---|---|---|---|
| O | O | (CH₂)₂ | H | oil |
| O | O | CH(CH₃)CH₂ | H | oil |
| O | O | C(CH₃)₂CH₂ | H | |
| O | O | CH(CH₃)CH(CH₃) | H | |
| O | O | (CH₂)₃ | H | oil |
| O | O | CH(CH₃)CH₂CH₂ | H | |
| O | O | CH₂CH(CH₃)CH₂ | H | |
| O | O | C(CH₃)₂CH₂CH₂ | H | |
| O | O | CH(CH₃)CH(CH₃)CH₂ | H | |
| O | O | CH₂C(CH₃)₂CH₂ | H | oil |
| O | O | (CH₂)₄ | H | oil |
| O | O | CH(CH₃)CH₂CH₂CH₂ | H | |
| O | O | CH₂CH(CH₃)CH₂CH₂ | H | |
| O | O | C(CH₃)₂CH₂CH₂CH₂ | H | |
| O | O | CH(CH₃)CH(CH₃)CH₂CH₂ | H | |
| O | O | CH(CH₃)CH₂CH₂CH(CH₃) | H | |
| O | O | CH₂C(CH₃)CH₂CH₂ | H | |
| O | O | CH₂CH(CH₃)CH(CH₃)CH₂ | H | |
| O | O | (CH₂)₅ | H | |
| O | O | CH₂CH₂OCH₂CH₂ | H | |
| O | O | CH₂CH₂OCH₂CH₂ | H | |
| O | O | CH₂CHClCH₂ | H | |
| O | O | CH₂CH(OCH₃)CH₂ | H | |
| O | S | (CH₂)₂ | H | oil |
| O | S | CH(CH₃)CH₂ | H | |
| O | S | C(CH₃)₂CH₂ | H | |
| O | S | CH(CH₃)CH(CH₃) | H | |
| O | S | (CH₂)₃ | H | |
| O | S | CH(CH₃)CH₂CH₂ | H | |
| O | S | CH₂CH(CH₃)CH₂ | H | |
| O | S | C(CH₃)₂CH₂CH₂ | H | |
| O | S | CH(CH₃)CH(CH₃)CH₂ | H | |
| O | S | CH(CH₃)CH₂CH(CH₃) | H | |
| O | S | CH₂C(CH₃)₂CH₂(CH₃) | H | |
| O | S | (CH₂)₄ | H | |
| O | S | CH(CH₃)CH₂CH₂CH₂ | H | |
| O | S | CH₂CH(CH₃)CH₂CH₂ | H | |
| O | S | C(CH₃)₂CH₂CH₂CH₂ | H | |
| O | S | CH(CH₃)CH(CH₃)CH₂CH₂ | H | |
| O | S | CH(CH₃)CH₂CH(CH₃)CH₂ | H | |
| O | S | CH(CH₃)CH₂CH₂CH(CH₃) | H | |
| O | S | CH₂C(CH₃)₂CH₂CH₂ | H | |
| O | S | CH₂CH(CH₃)CH(CH₃)CH₂ | H | |
| O | S | (CH₂)₅ | H | |
| O | S | CH₂CH₂OCH₂CH₂ | H | |
| O | S | CH₂CH₂OCH₂CH₂ | H | |
| S | S | (CH₂)₂ | H | |
| S | S | CH(CH₃)CH₂ | H | |
| S | S | C(CH₃)₂CH₂ | H | |
| S | S | CH(CH₃)CH(CH₃) | H | |
| S | S | (CH₂)₃ | H | |
| S | S | CH(CH₃)CH₂CH₂ | H | |
| S | S | CH₂CH(CH₃)CH₂ | H | |
| S | S | C(CH₃)₂CH₂CH₂ | H | |
| S | S | CH(CH₃)CH(CH₃)CH₂ | H | |
| S | S | CH(CH₃)CH₂CH(CH₃) | H | |
| S | S | CH₂C(CH₃)₂CH₂ | H | |
| S | S | (CH₂)₄ | H | |
| S | S | CH(CH₃)CH₂CH₂CH₂ | H | |
| S | S | CH₂CH(CH₃)CH₂CH₂ | H | |
| S | S | C(CH₃)₂CH₂CH₂CH₂ | H | |
| S | S | CH(CH₃)CH(CH₃)CH₂CH₂ | H | |
| S | S | CH(CH₃)CH₂CH(CH₃)CH₂ | H | |
| S | S | CH(CH₃)CH₂CH₂CH(CH₃) | H | |
| S | S | CH₂C(CH₃)₂CH₂CH₂ | H | |
| S | S | CH₂CH(CH₃)CH(CH₃)CH₂ | H | |
| S | S | (CH₂)₅ | H | |
| S | S | CH₂CH₂OCH₂CH₂ | H | |
| S | S | CH₂CH₂OCH₂CH₂ | H | |
| O | O | (CH₂)₂ | CH₃ | oil |
| O | O | (CH₂)₂ | CH₂CH₃ | |
| O | O | (CH₂)₂ | CH(CH₃)₂ | |
| O | O | (CH₂)₂ | CH₂CH₂CH₃ | |
| O | O | CH(CH₃)CH₂ | CH₃ | |
| O | O | C(CH₃)₂CH₂ | CH₃ | |
| O | O | (CH₂)₃ | CH₃ | |
| O | O | (CH₂)₄ | CH₃ | |
| O | S | (CH₂)₂ | CH₃ | |
| O | S | (CH₂)₂ | CH₂CH₃ | |
| O | S | (CH₂)₂ | CH(CH₃)₂ | |
| O | S | (CH₂)₂ | CH₂CH₂CH₃ | |
| O | S | CH(CH₃)CH₂ | CH₃ | |
| O | S | C(CH₃)₂CH₂ | CH₃ | |
| O | S | (CH₂)₃ | CH₃ | |
| O | S | (CH₂)₄ | CH₃ | |
| S | S | (CH₂)₂ | CH₃ | |
| S | S | (CH₂)₂ | CH₂CH₃ | |
| S | S | (CH₂)₂ | CH(CH₃)₂ | |
| S | S | (CH₂)₂ | CH₂CH₂CH₃ | |
| S | S | CH(CH₃)CH₂ | CH₃ | |
| S | S | C(CH₃)₂CH₂ | CH₃ | |
| S | S | (CH₂)₃ | CH₃ | |
| S | S | (CH₂)₄ | CH₃ | |
| O | O | (CH₂)₂ | OCH₃ | oil |
| O | O | (CH₂)₂ | OCH₂CH₃ | |
| O | O | (CH₂)₂ | OCH(CH₃)₂ | |
| O | O | CH(CH₃)CH₂ | OCH₃ | |
| O | O | (CH₂)₃ | OCH₃ | |
| O | O | (CH₂)₄ | OCH₃ | |
| O | O | (CH₂)₂ | Cl | oil |
| O | O | (CH₂)₂ | Br | |
| O | O | (CH₂)₂ | F | |
| O | O | CH(CH₃)CH₂ | Cl | |
| O | O | CH(CH₃)CH₂ | Br | |
| O | O | CH(CH₃)CH₂ | F | |
| O | O | (CH₂)₃ | Cl | |
| O | O | (CH₂)₃ | Br | |
| O | O | (CH₂)₃ | F | |
| O | O | (CH₂)₄ | Cl | |
| O | O | (CH₂)₄ | Br | |
| O | O | (CH₂)₄ | F | |
| S | S | (CH₂)₂ | OCH₃ | |
| S | S | (CH₂)₂ | OCH₂CH₃ | |
| S | S | (CH₂)₂ | OCH(CH₃)₂ | |
| S | S | CH(CH₃)CH₂ | OCH₃ | |
| S | S | (CH₂)₃ | OCH₃ | |
| S | S | (CH₂)₄ | OCH₃ | |
| S | S | (CH₂)₂ | Cl | |
| S | S | (CH₂)₂ | Br | |
| S | S | (CH₂)₂ | F | |
| S | S | CH(CH₃)CH₂ | Cl | |
| S | S | CH(CH₃)CH₂ | Br | |
| S | S | CH(CH₃)CH₂ | F | |
| S | S | (CH₂)₃ | Cl | |
| S | S | (CH₂)₃ | Br | |
| S | S | (CH₂)₃ | F | |
| S | S | (CH₂)₄ | Cl | |
| S | S | (CH₂)₄ | Br | |
| S | S | (CH₂)₄ | F | |
| O | S | (CH₂)₂ | OCH₃ | |
| O | S | (CH₂)₂ | OCH₂CH₃ | |
| O | S | (CH₂)₂ | OCH(CH₃)₂ | |
| O | S | CH(CH₃)CH₂ | OCH₃ | |
| O | S | (CH₂)₃ | OCH₃ | |
| O | S | (CH₂)₄ | OCH₃ | |
| O | S | (CH₂)₂ | Cl | |
| O | S | (CH₂)₂ | Br | |
| O | S | (CH₂)₂ | F | |
| O | S | CH(CH₃)CH₂ | Cl | |
| O | S | CH(CH₃)CH₂ | Br | |
| O | S | CH(CH₃)CH₂ | F | |
| O | S | (CH₂)₃ | Cl | |
| O | S | (CH₂)₃ | Br | |
| O | S | (CH₂)₃ | F | |
| O | S | (CH₂)₄ | Cl | |
| O | S | (CH₂)₄ | Br | |
| O | S | (CH₂)₄ | F | |
| NCH₃ | O | (CH₂)₂ | H | oil |
| NCH₂CH₃ | O | (CH₂)₂ | H | |
| NCH₃ | O | (CH₂)₃ | H | |
| NCH₃ | O | (CH₂)₄ | H | |
| NCOCH₃ | O | (CH₂)₂ | H | oil |
| NCOCH₃ | O | (CH₂)₃ | H | |
| NCOCH₃ | O | (CH₂)₄ | H | |
| NCOCH₂CH₃ | O | (CH₂)₂ | H | |
| NCOCH(CH₃)₂ | O | (CH₂)₂ | H | oil |
| NCO₂CH₃ | O | (CH₂)₂ | H | oil |
| NCO₂CH₃ | O | CH(CH₃)CH₂ | H | |
| NCO₂CH₃ | O | (CH₂)₃ | H | |
| NCO₂CH₃ | O | (CH₂)₄ | H | |
| NCO₂CH₂CH₃ | O | (CH₂)₂ | H | oil |
| NCO₂CH(CH₃)₂ | O | (CH₂)₂ | H | |
| NCON(CH₃)₂ | O | (CH₂)₂ | H | oil |
| NCON(CH₃)₂ | O | (CH₂)₃ | H | |
| NCON(CH₃)₂ | O | (CH₂)₄ | H | |
| NCON(CH₂CH₃)₂ | O | (CH₂)₂ | H | |
| NCONHCH₃ | O | (CH₂)₂ | H | |
| NCONHCH₂CH₃ | O | (CH₂)₂ | H | |
| NCONHCH₂CH₂CH₃ | O | (CH₂)₂ | H | |
| NCONCH(CH₃)₂ | O | (CH₂)₂ | H | oil |
| NCO₂CH₂CH₃ | O | (CH₂)₃ | H | oil |
| NCO₂CH₂CH₃ | O | (CH₂)₄ | H | |
| NCH₃ | NCOCH₃ | COCH₂ | H | |
| NCH₃ | NCO₂CH₃ | COCH₂ | H | |
| NCH₃ | NCON(CH₃)₂ | COCH₂ | H | |
| NCH₂CH₃ | NCOCH₃ | COCH₂ | H | |
| NCH₂CH₃ | NCO₂CH₃ | COCH₂ | H | |
| NCH₂CH₃ | NCO₂CH₃ | COCH₂ | H | |
| O | NCOCH₃ | COCH₂ | H | |
| O | NCOCH₂CH₃ | COCH₂ | H | |
| O | NCO₂CH₃ | COCH₂ | H | |
| O | NCO₂CH₂CH₃ | COCH₂ | H | |
| O | NCON(CH₃)₂ | COCH₂ | H | |
| O | NCON(CH₂CH₃)₂ | COCH₂ | H | |
| NCH₃ | NCOCH₃ | COCH(CH₃) | H | |
| NCH₃ | NCO₂CH₃ | COCH(CH₃) | H | |
| NCH₃ | NCON(CH₃)₂ | COCH(CH₃) | H | |
| O | NCOCH₃ | COCH(CH₃) | H | |
| O | NCO₂CH₃ | COCH(CH₃) | H | |
| O | NCON(CH₃)₂ | COCH(CH₃) | H | |
| NCH₃ | O | COCH₂ | H | |
| NCH₃ | O | COCH₂CH₂ | H | |
| NCH₃ | O | COCH(CH₃) | H | |
| NCH₃ | NCH₃ | COCO | H | |
| NCH₃ | NCH₃ | COCH₂CO | H | |
| NCH₃ | O | COCO | H | |
| NCH₃ | O | COCH₂CO | H | |
| NCH₃ | NCH₃ | - | CH₃ | |
| NCH₂CH₃ | NCH₃ | - | CH₃ | |
| NCH₂CH₃ | NCH₂CH₃ | - | CH₃ | |
| NCH₃ | NH | (CH₂)₂ | CH₃ | |
| NCH₃ | NCOCH₃ | (CH₂)₂ | CH₃ | |
| NCH₃ | NCOCH₂CH₃ | (CH₂)₂ | CH₃ | |
| NCH₃ | NCOCH₃ | (CH₂)₃ | CH₃ | |
| NCH₃ | NCO₂CH₃ | (CH₂)₂ | CH₃ | |
| NCH₃ | NCO₂CH₃ | (CH₂)₃ | CH₃ | |
| NCH₃ | NCOCH₃ | (CH₂)₃ | CH₃ | |
| NCH₃ | NCOCH₃ | (CH₂)₄ | CH₃ | |
| NCH₃ | S | (CH₂)₂ | CH₃ | |
| NCH₃ | S | (CH₂)₃ | CH₃ | |
| NCH₃ | S | (CH₂)₄ | CH₃ | |
| NCOCH₃ | S | (CH₂)₂ | CH₃ | |
| NCO₂CH₃ | S | (CH₂)₃ | CH₃ | |
| NCH₃ | NCH₃ | - | H | |
| NCH₂CH₃ | NCH₃ | - | H | |
| NCH₂CH₃ | NCH₂CH₃ | - | H | |
| NCH₃ | NH | (CH₂)₂ | H | |
| NCH₃ | NCOCH₃ | (CH₂)₂ | H | |
| NCH₃ | NCOCH₂CH₃ | (CH₂)₂ | H | |
| NCH₃ | NCOCH₃ | (CH₂)₃ | H | |
| NCH₃ | NCO₂CH₃ | (CH₂)₂ | H | |
| NCH₃ | NCO₂CH₃ | (CH₂)₃ | H | |
| NCH₃ | NCOCH₃ | (CH₂)₃ | H | |
| NCH₃ | NCO₂CH₃ | (CH₂)₄ | H | |
| NCH₃ | NCOCH₃ | (CH₂)₄ | H | |
| NCH₃ | S | (CH₂)₂ | H | |
| NCH₃ | S | (CH₂)₃ | H | |
| NCH₃ | S | (CH₂)₄ | H | |
| NCOCH₃ | S | (CH₂)₂ | H | |
| NCO₂CH₃ | S | (CH₂)₃ | H | |
| O | 0 | COCH₂ | H | |
| O | 0 | COCH₂CH₂ | H | |
| O | 0 | COCH₂CH(CH₃) | H | |
| O | 0 | COCO | H | |
| O | 0 | COCH₂CO | H | |
| O | 0 | COCH=CH | H | |
| O | 0 | COCH=C(CH₃) | H | |
| O | 0 | CH=CH | H | |
| O | 0 | CH=CHCH₂ | H | |
| O | 0 | (CH₃)C=CHCH₂ | H | |
| O | 0 | CH=CHCH(CH₃) | H | |
| O | S | COCH₂ | H | |
| O | O | CH₂CH(CO₂CH₃) | H | |

### EXAMPLE 19

### Preparation of 5-(1,3-Dioxolan-2-yl)-2,3-pyridinedicarboxylic acid

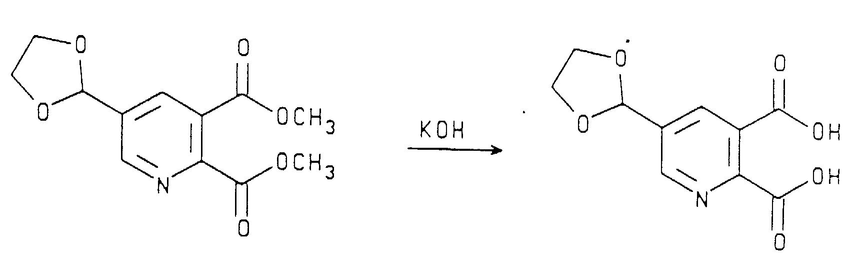

5-(1,3-Dioxolan-2-yl)-2,3-pyridinedicarboxylic acid, dimethyl ester (2.86 g, 0.011 mol) in methanol is added dropwise to a mixture of potassium hydroxide (1.26 g, 0.022 mol) and methanol. The reaction mixture is heated for 3 hours and 30 minutes at about 62°C, cooled to room temperature, acidified to pH 1 with concentrated hydrochloric acid, filtered through diatomaceous earth and the filtrate is concentrated in vacuo to yield the title compound as a pale yellow powder (2.86 g, 100%), identified by IR and NMR spectral analyses.

### EXAMPLE 20

### Preparation of 5-(1,3-Dioxolan-2-yl)-2,3-pyridinedicarboxylic acid

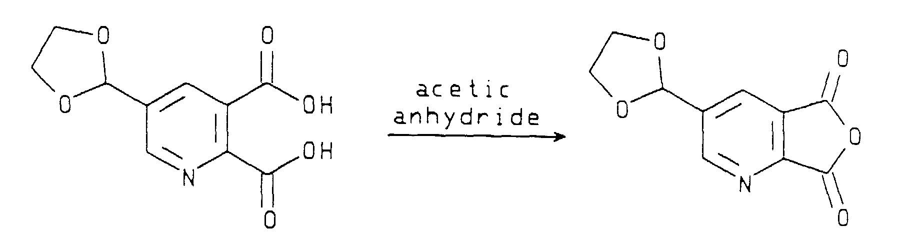

A solution of 5-(1,3-dioxolan-2-yl)-2,3-pyridinedicarboxylic acid (2.86 g, 0.012 mol), acetic anhydride (11.3 mL, 0.12 mol) and pyridine is stirred at room temperature for 1 hour and 45 minutes, then at reflux temperature for 4 hours. The reaction mixture is concentrated in vacuo to give the title compound as an oil (2.64 g, 100%), identified by IR spectra analysis.

### EXAMPLE 21

### Preparation of 2-[(1-Carbamoyl-1,2-dimethylpropyl)carbamoyl]-5-(1,3-dioxolan-2-yl)nicotinic acid

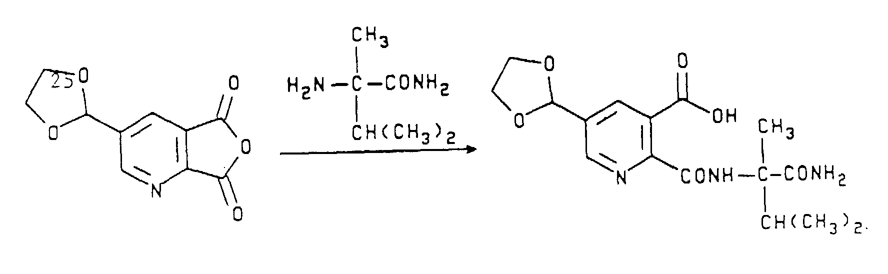

A solution of 5-(1,3-dioxolan-2-yl)-2,3-pyridinedicarboxylic anhydride (2.64 g, 0.012 mol) and 2-amino-2,3-dimethylbutyramide (1.55 g, 0.012 mol) in tetrahydrofuran is stirred for 2 days at room temperature. The reaction mixture is concentrated in vacuo to give the title compound as an orange oil (5.4 g, 100%), identified by NMR spectra analysis.

### EXAMPLE 22

### Preparation of 5-(1,3-Dioxolan-2-yl)-2-(4-isopropyl-4-methyl-5-oxo-2-imidazolin-2-yl)nicotinic acid

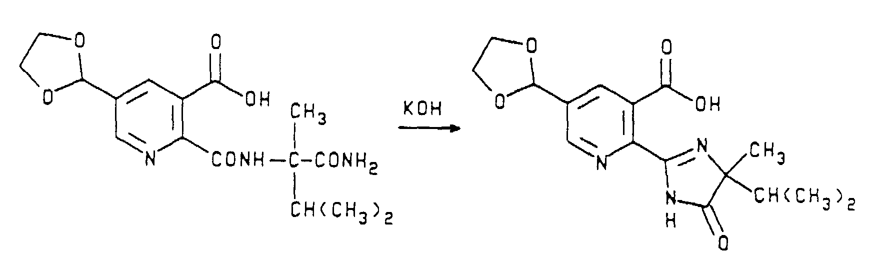

2-[(1-Carbamoyl-1,2-dimethylpropyl)carbamoyl]-5-(1,3-dioxolan-2-yl)nicotinic acid (4.19 g, 0.019 mol) and 15% potassium hydroxide is heated at 80°C for 1 hour. The reaction mixture is acidified to pH 3 with concentrated hydrochloric acid and methylene chloride is added. The methylene chloride layer is separated, dried over anhydrous magnesium sulfate and concentrated in vacuo to give an oil. The oil is chromatographed using silica gel and methylene chloride with increasing percentage of ether as eluent to give the title compound as a pale yellow solid (0.19 g, 5%), mp 156°C, identified by IR and NMR spectral analyses.

### EXAMPLE 23

### Preparation of 5-(1,3-Dioxepan-2-yl)-2-(4-isopropyl-4-methyl-5-oxo-2-imidazolin-2-yl)nicotinic acid

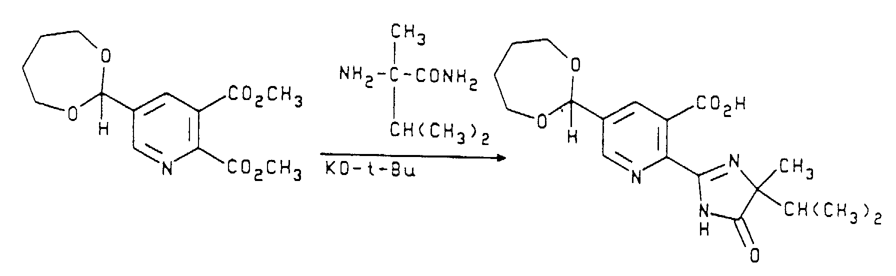

5-(1,3-Dioxepan-2-yl)-2,3-pyridinedicarboxylic acid, dimethyl ester (0.9 g, 0.00305 mol) in toluene is added to 2-amino-2,3-dimethylbutyramide (0.4 g, 0.00305 mol) and potassium tert-butoxide (0.69 g, 0.0061 mol) in toluene. The mixture is heated for 3 hours from 60° to 70°C. The reaction mixture is cooled to room temperature, water is added and the mixture is concentrated in vacuo to give an oil. The oil is diluted with water and washed with ether. The aqueous solution is acidified to pH 3.1 with 2 normal hydrochloric acid solution and extracted with methylene chloride. The combined methylene chloride extracts are dried over anhydrous magnesium sulfate and concentrated in vacuo to give the title compound as a white solid (0.63 g, 57%) mp 60-69°C, identified by IR and NMR spectral analyses.

Following the above procedure and substituting the appropriate formula I 2,3-pyridinedicarboxylate, the following formula IIa compounds are obtained.

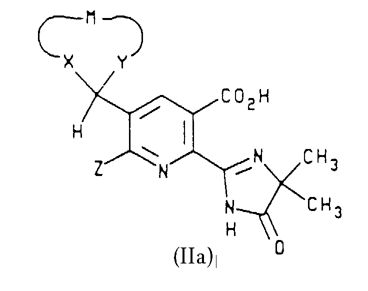

| X | Y | M | Z | mp°C |
|---|---|---|---|---|
| O | O | (CH₂)₄ | H | 60-69 |
| S | O | (CH₂)₂ | H | 72-76 |
| NCH₃ | NCH₃ | - | H | 174-176 |
| O | O | (CH₂)₂ | CH₃ | 75 |
| O | O | (CH₂)₂ | OCH₃ | 222-223 |
| O | O | (CH₂)₂ | Cl | 79-80 |
| S | S | (CH₂)₂ | H | 125-133 |
| O | NCO₂CH₃ | (CH₂)₂ | H | 178-180 |
| O | NCO₂CH₂CH₃ | (CH₂)₂ | H | 75-77 |
| O | NCOCH₃ | (CH₂)₂ | H | 167-168 |
| O | NCON(CH₃)₂ | (CH₂)₂ | H | 198-199 |
| O | NCOCH(CH₃)₂ | (CH₂)₂ | H | 91-93 |
| O | NCONHCH(CH₃)₂ | (CH₂)₂ | H | 181-183 |
| O | NCO₂CH₂CH₃ | (CH₂)₃ | H | 77-79 |
| O | NSO₂CH₃ | (CH₂)₃ | H | 188-192 |

### EXAMPLE 24

### Preparation of 5-(1-Acetyl-3-methyl-2-imidazolidinyl)-2-(4-isopropyl-4-methyl-5-oxo-2-imidazolin-2-yl)nicoti nic acid ammonium salt

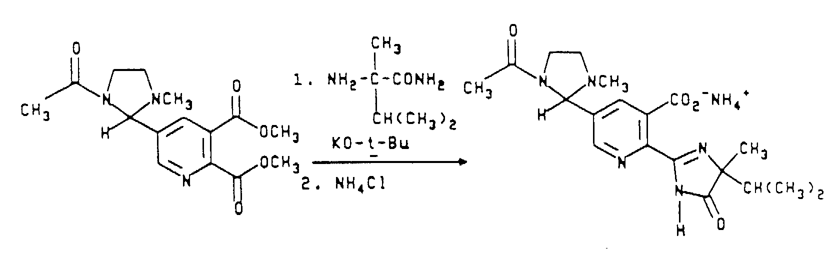

To a stirred solution of dimethyl 5-(1-acetyl-3-methyl-2-imidazolidinyl)pyridine-2,3-di-carboxylate (0.70 g, 0.0022 mol), and 2-amino-2,3-dimethylbutyramide (0.28 g, 0.0022 mol) in toluene (10 mL) is added potassium tert-butoxide (0.49 g, 0.0044 mol). The resulting mixture is stirred for 2 hours at 80°C to 90°C. After cooling to room temperature, the reaction is quenched by the addition of water (15 mL) and ammonium chloride (0.25 g). The layers are separated, the aqueous solution concentrated under high vacumn, the product triturated with 33% ethanol in chloroform and filtered. The filtrate is concentrated in vacuo to afford the title compound (0.95 g, 100%) as a gold solid, mp 93-98°C, identified by IR and NMR spectral analyses.

### EXAMPLE 25

### Preparation of Methyl 2-(1-benzoyl-4-isopropyl-4-methyl-5-oxo-2-imidazolin-2-yl)-5-methylnicotinate

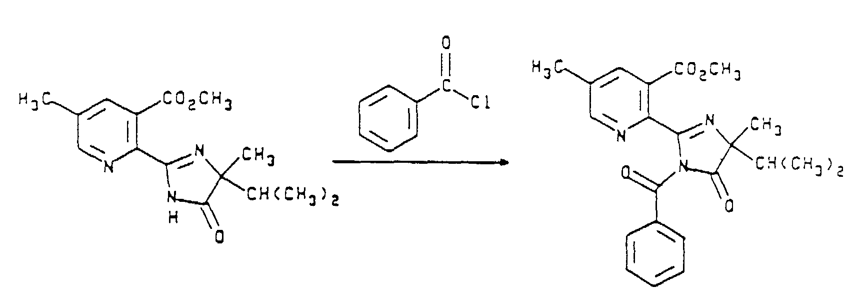

Benzoyl chloride (42.1 mL, 0.363 mol) is added dropwise to a 0°C solution of 2-(4-isopropyl-4-methyl-5-oxo-2-imidazolin-2-yl)-5-methylnicotinic acid, methyl ester (50 g, 0.173 mol) and 4-dimethylaminopyridine (4.2 g, 0.0344 mol) in pyridine. The reaction mixture is stirred for 1 hour at 0°C and 4 hours at room temperature. Concentration in vacuo gives a liquid that is dissolved into methylene chloride and washed sequentially with 2 normal hydrochloric acid and saturated sodium bicarbonate solution, dried over anhydrous magnesium sulfate and concentrated in vacuo to yield a solid that upon trituration with ether gives the title compound as a white solid (46.2 g, 68%), mp 104-106°C, identified by IR and NMR spectral analyses.

### EXAMPLE 26

### Preparation of Methyl 2-(1-benzoyl-4-isopropyl-4-methyl-5-oxo-2-imidazolin-2-yl)-5-(bromomethyl) -nicotinate

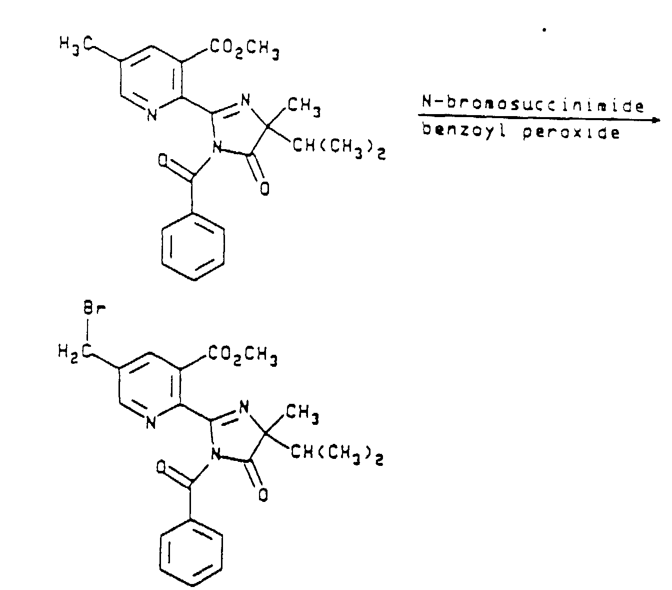

Solid N-bromosuccinimide (22.98 g, 0.129 mol) and benzoyl peroxide (3.12 g, 0.0129 mol) are added to a 25°C solution of 2-(1-benzoyl-4-isopropyl-4-methyl-5-oxo-2-imidazolin-2-yl)-5-methylnicotinic acid, methyl ester (46.2 g, 0.117 mol) in carbon tetrachloride. The reaction mixture is heated at 70°C for 16 hours, cooled to room temperature, diluted with methylene chloride, washed sequentially with 5% sodium metabisulfite solution and water, dried over anhydrous magnesium sulfate and concentrated in vacuo to give an oil. The oil is chromatographed using silica gel and hexanes/ethyl acetate (3:1) to (1:1) as eluant to give the title compound as a yellow solid (19.64 g, 35%), mp 88-119°C, identified by IR and NMR spectral analyses.

### EXAMPLE 27

### Preparation of Methyl 2-(4-isopropyl-4-methyl-5-oxo-2-imidazolin-2-yl)-5-[(phenylthio)methyl]nicotinate

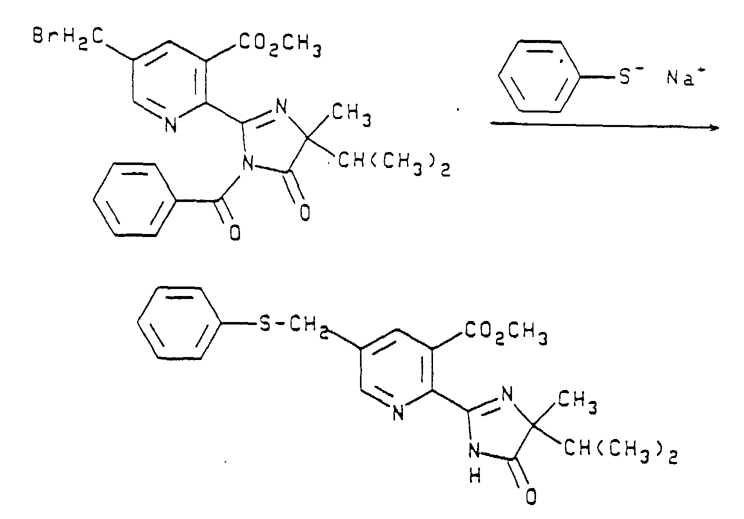

A solution of thiophenol, sodium salt and methanol is added to a solution of 2-(1-benzoyl-4-isopropyl-4-methyl-5-oxo-2-imidazolin-2-yl)-5-(bromo-methyl)nicotinic acid, methyl ester (14.08 g, 0.0298-mol) and methanol. The reaction mixture is stirred for 15 hours at room temperature, acidified with ammonium chloride (5 g, 0.0869 mol) and concentrated in vacuo to yield an oil. The oil is chromatographed using silica gel and hexanes/ethyl acetate (3:1) to (1:1) as eluant to give the title compound as a yellow solid (8.32 g, 55%), mp 135-138°C, identified by IR and NMR spectral analyses.

### EXAMPLE 28

### Preparation of Methyl 2-(4-isopropyl-4-methyl-5-oxo-2-imidazolin-2-yl)-5-[(phenylsulfinyl)methyl]nicotinate

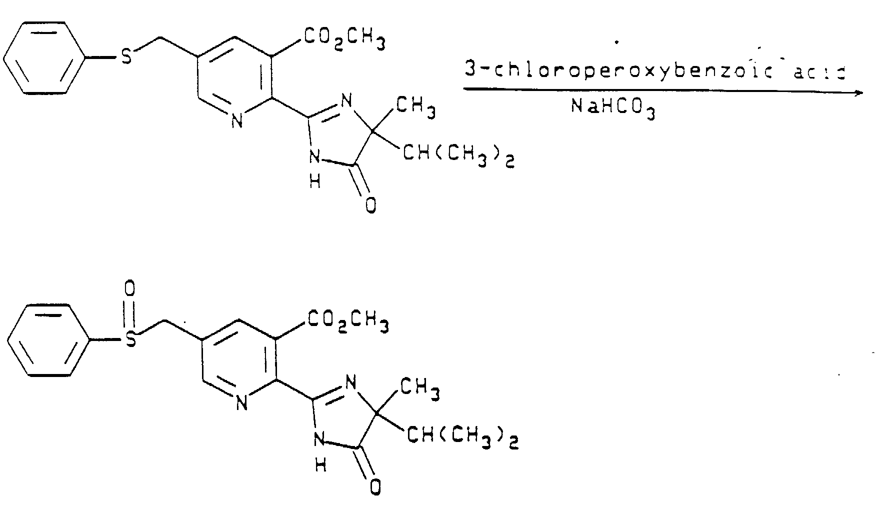

3-Chloroperoxybenzoic acid (20 mL, 0.00252-mol) is added dropwise to a -78°C solution of 2-(4-isopropyl-4-methyl-5-oxo-2-imidazolin-2-yl)-5-[(phenylthio)methyl)nicotinic acid, methyl ester (1.0 g, 0.00252 mol) and sodium bicarbonate (0.2 g, 0.00238 mol) in methylene chloride. The reaction mixture is stirred at -78°C for 30 minutes and then at room temperature for 24 hours. The reaction mixture is diluted with 5% sodium metabisulfite solution. The methylene chloride layer is separated, washed with saturated sodium bicarbonate solution, dried over anhydrous magnesium sulfate and concentrated in vacuo to yield a yellow oil. The oil is chromatographed using silica gel and hexanes/ethyl acetate (1:1) to (1:2) to (1:9) and finally 100% ethyl acetate as eluant to give the title compound as a yellow solid (0.97 g, 90%), mp 132-136°C, identified by IR and NMR spectral analyses.

### EXAMPLE 29

### Preparation of Methyl 2-(1-acetyl-4-isopropyl-4-methyl-5-oxo-2-imidazolin-2-yl)-5-[hydroxy(phenylthio)methyl] -nicotinate, 5-acetate

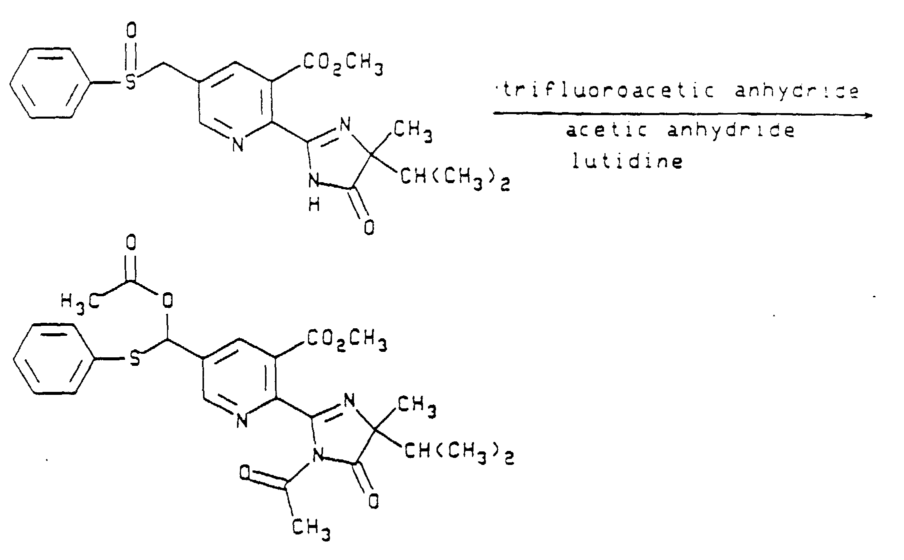

A solution of trifluoroacetic anhydride (0.85 mL, 0.006 mol) in acetic anhydride is stirred at room temperature for 3 hours. 2-(4-Isopropyl-4-methyl-5-oxo-2-imidazolin-2-yl)-5-[(phenylsulfinyl)methyl]-nicotinic acid, methyl ester (1.02 g, 0.0024 mol) is dissolved into the solution and stirred at room temperature for 15 minutes. Lutidine (840 µL, 0.072 mol) is added and the reaction mixture is stirred for 2 hours at room temperature Concentration in vacuo yields an oil which is dissolved into methylene chloride and washed sequentially with 2 normal hydrochloric acid and saturated sodium bicarbonate solution, dried over anhydrous magnesium sulfate and concentrated in vacuo to give the title compound as an orange oil (1.49 g, 100%), identified by IR and NMR spectral analyses.

### EXAMPLE 30

### Preparation of 5-Formyl-2-(4-isopropyl-4-methyl-5-oxo-2-imidazolin-2-yl)nicotinic acid

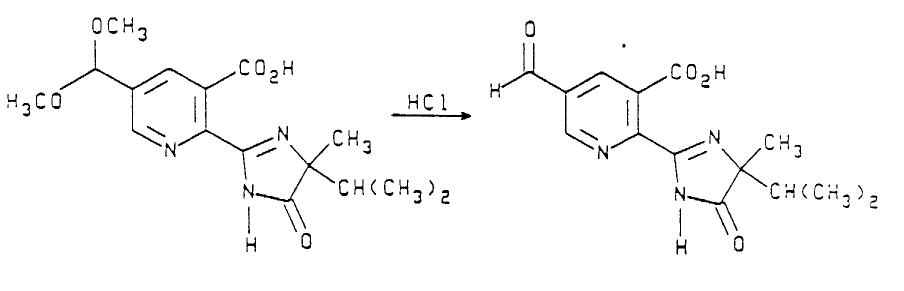

A solution of 5-formyl-2-(4-isopropyl-4-methyl-5-oxo-2-imidazolin-2-yl)nicotinic acid, 5-(dimethyl acetal) (3.65 g, 0.0109 mol) and 2N hydrochloric acid is stirred for 2 hours at room temperature. The pE of the reaction mixture is adjusted to 3.0 with sodium bicarbonate and the water is evaporated in vacuo to give a solid. The solid is triturated with acetone/ethanol (2:1) and removed by filtration. The filtrate is concentrated in vacuo to yield the title compound as a tan solid (3.5 g, 100%), mp 187-198°C, identified by IR and NMR spectral analyses.

### EXAMPLE 31

### Preparation of 5-(4-Methyl-1,3-dioxolan-2-yl)-2-(4-isopropyl-4-methyl-5-oxo-2-imidazolin-2-yl)nicotinic acid

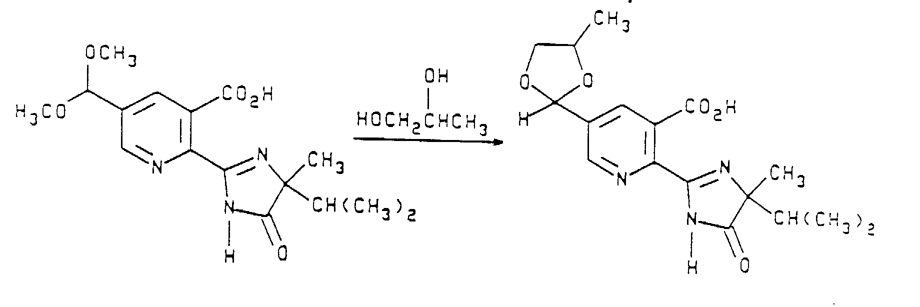

A solution of 5-formyl-2-(4-isopropyl-4-methyl-5-oxo-2-imidazolin-2-yl)nicotinic acid, 5-(dimethyl acetal) (0.65 g, 0.00194 mol), 1,2-propanediol (0.65 g, 0.0085 mol) and a catalytic amount of p-toluenesulfonic acid in toluene is heated at reflux temperature for 2 hours. The reaction mixture is cooled to room temperature, concentrated in vacuo and the residue is dissolved in methylene chloride. The methylene chloride solution is washed sequentially with water and brine, dried over anhydrous magnesium sulfate and concentrated in vacuo to give a solid. The solid is chromatographed using silica gel and a gradient elution of 100% methylene chloride to 5% methanol in methylene chloride to yield the title compound as a yellow solid (0.5 g, 74%), mp 50-62°C, identified by IR and NMR spectral analyses.

### EXAMPLE 32

### Preparation of 2-(4-Isopropyl-4-methyl-5-oxo-2-imidazolin-2-yl)-5-(3-methyl-2-oxazolidinyl)nicotinic acid, compound with 2-(methylamino)ethanol)

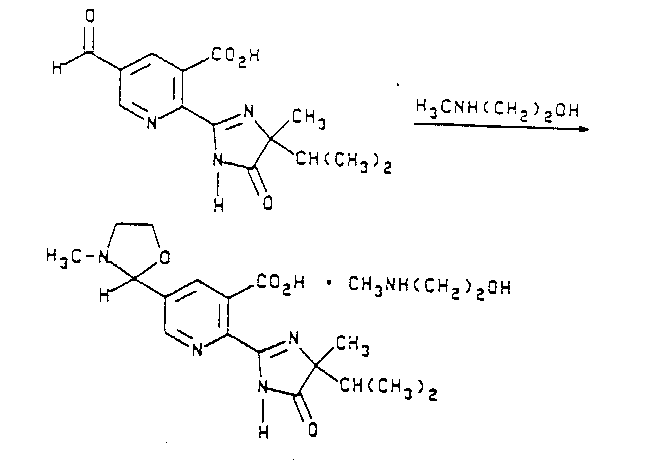

A solution of 5-formyl-2-(4-isopropyl-4-methyl-5-oxo-2-imidazolin-2-yl)nicotinic acid (0.36 g, 0.00125 mol) and 2-(methylamino)ethanol (0.19 g, 0.0025 mol) in chloroform is heated for 2 hours and 30 minutes at reflux temperature. The reaction mixture is then concentrated in vacuo at aspirator pressure to give the title compound as a yellow gum (0.40 g, 76%), identified by IR and NMR spectral analyses.

### EXAMPLE 33

### Preparation of 5-m-Dioxan-2-yl)-2-(4-isopropyl-4-methyl-5-oxo-2-imidazolin-2-yl)nicotinic acid

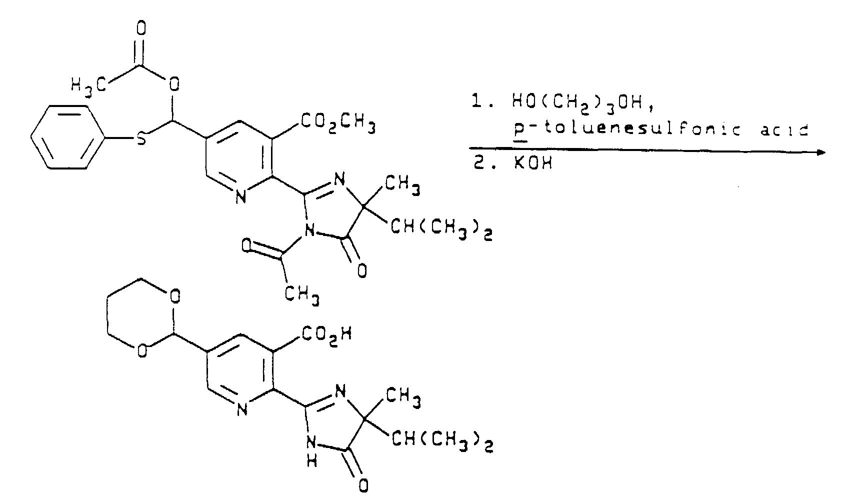

A solution of 2-(1-acetyl-4-isopropyl-4-methyl-5-oxo-2-imidazolin-2-yl)-5-[hydroxy(phenylthio)-methyl]nicotinic acid, methyl ester, 5-acetate (0.48 g, 0.00096 mol), a catalytic amount of p-toluenesulfonic acid, 1,3-propanediol and toluene is heated at reflux temperature for 3 hours. Concentration in vacuo yields an oil which is dissolved into methylene chloride, washed with 5% sodium bicarbonate solution, dried over anhydrous magnesium sulfate and concentrated in vacuo to yield an oil. The oil is dissolved into tetrahydrofuran and 1 molar potassium hydroxide is added and the reaction mixture is stirred at 0°C for 30 minutes, then at room temperature for 2 hours. Concentration in vacuo gives an oil to which water is added and the pH is adjusted to 3.0 with concentrated hydrochloric acid. The aqueous solution is saturated with sodium chloride and extracted with methylene chloride. The combined methylene chloride extracts are dried over anhydrous magnesium sulfate and concentrated in vacuo to give an oil. The oil is chromatographed using silica gel and 100% methylene chloride to 10% methanol in methylene chloride as eluant to give the title compound as a yellow solid (0.2 g, 60%), mp 188-196°C, identified by IR and NMR spectral analyses.

Using the above procedure and substituting 2,2-dimethyl-1,3-propanediol gives 5-(5,5-dimethyl-m-dioxan-2-yl)-2-(4-isopropyl-4-methyl-5-oxo-2-imidazolin-2-yl)nicotinic acid, mp 163-170°C, identified by IR and NMR spectral analyses.

### EXAMPLE 34

### Preparation of Methyl 2-(4-Isopropyl-4-methyl-5-oxo-2-imidazolin-2-yl)-5-(4-methyl-6-oxo-1,3-dioxan-2-yl) -nicotinate

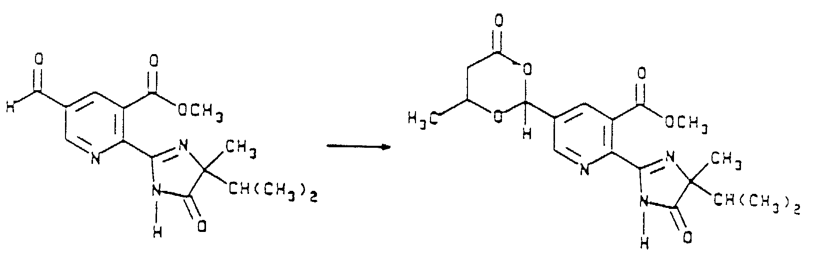

A solution of methyl 5-formyl-2-(4-isopropyl-4-methyl-5-oxo-2-imidazolin-2-yl)nicotinate (0.55 g, 0.0018 mol), 3-hydroxybutyric acid (0.28 g, 0.0027 mol) and a catalytic amount of para-toluenesulfonic acid in chloroform (25 mL) is heated at reflux temperature for 113 hours. During this time, water is removed by placement of an addition funnel containing 3 angstrom molecular sieves between the reaction flask and the reflux condensor. The reaction mixture is poured into aqueous saturated sodium bicarbonate and the layers are separated. The aqueous portion is extracted with additional methylene chloride and the combined organic extracts are dried over anhydrous magnesium sulfate and concentrated in vacuo to give a residue. The residue is purified by chromatography using silica gel and ethyl acetate in hexanes as eluant to yield the title compound (0.14 g, 20%) as a white solid, mp 65-75°C, identified by IR and NMR spectral analyses.

Formula Ia compounds that may be prepared using the above-described procedure are listed below.

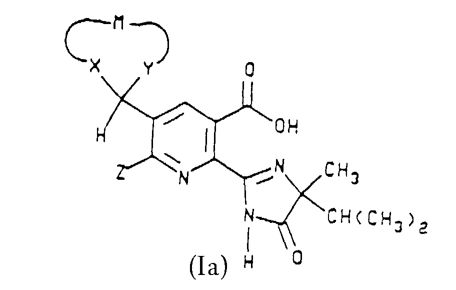

| X | Y | M | Z | mp°C |
|---|---|---|---|---|
| O | O | (CH₂)₂ | H | 156 |
| O | O | CH(CH₃)CH₂ | H | 50-62 |
| O | O | C(CH₃)₂CH₂ | H | |
| O | O | CH(CH₃)CH(CH₃) | H | |
| O | O | (CH₂)₃ | H | 188-196 |
| O | O | CH(CH₃)CH₂CH₂ | H | |
| O | O | CH₂CH(CH₃)CH₂ | H | |
| O | O | C(CH₃)₂CH₂CH₂ | H | |
| O | O | CH(CH₃)CH(CH₃)CH₂ | H | |
| O | O | CH(CH₃)CH₂CH(CH₃) | H | |
| O | O | CH₂C(CH₃)₂CH₂ | H | 163-170 |
| O | O | (CH₂)₄ | H | 60-69 |
| O | O | CH(CH₃)CH₂CH₂CH₂ | H | |
| O | O | CH₂CH(CH₃)CH₂CH₂ | H | |
| O | O | C(CH₃)₂CH₂CH₂CH₂ | H | |
| O | O | CH(CH₃)CH(CH₃)CH₂CH₂ | H | |
| O | O | CH(CH₃)CH₂CH(CH₃)CH₂ | H | |
| O | O | CH(CH₃)CH₂CH₂CH(CH₃) | H | |
| O | O | CH₂C(CH₃)₂CH₂CH₂ | H | |
| O | O | CH₂CH(CH₃)CH(CH₃)CH₂ | H | |
| O | O | (CH₂)₅ | H | |
| O | O | CH₂CH₂OCH₂CH₂ | H | |
| O | O | CH₂CH₂SCH₂CH₂ | H | |
| O | S | (CH₂)₂ | H | 72-76 |
| O | S | CH(CH₃)CH₂ | H | |
| O | S | C(CH₃)₂CH₂ | H | |
| O | S | CH(CH₃)CH(CH₃) | H | |
| O | S | (CH₂)₃ | H | |
| O | S | CH(CH₃)CH₂CH₂ | H | |
| O | S | CH₂CH(CH₃)CH₂ | H | |
| O | S | C(CH₃)₂CH₂CH₂ | H | |
| O | S | CH(CH₃)CH(CH₃)CH₂ | H | |
| O | S | CH(CH₃)CH₂CH(CH₃) | H | |
| O | S | CH₂C(CH₃)₂CH₂ | H | |
| O | S | (CH₂)₄ | H | |
| O | S | CH(CH₃)CH₂CH₂CH₂ | H | |
| O | S | CH₂CH(CH₃)CH₂CH₂ | H | |
| O | S | C(CH₃)₂CH₂CH₂CH₂ | H | |
| O | S | CH(CH₃)CH(CH₃)CH₂CH₂ | H | |
| O | S | CH(CH₃)CH₂CH(CH₃)CH₂ | H | |
| O | S | CH(CH₃)CH₂CH₂CH(CH₃) | H | |
| O | S | CH₂C(CH₃)₂CH₂CH₂ | H | |
| O | S | CH₂CH(CH₃)CH(CH₃)CH₂ | H | |
| O | S | (CH₂)₅ | H | |
| O | S | CH₂CH₂OCH₂CH₂ | H | |
| O | S | CH₂CH₂SCH₂CH₂ | H | |
| S | S | (CH₂)₂ | H | 125-133 |
| S | S | CH(CH₃)CH₂ | H | |
| S | S | C(CH₃)₂CH₂ | H | |
| S | S | CH(CH₃)CH(CH₃) | H | |
| S | S | (CH₂)₃ | H | |
| S | S | CH(CH₃)CH₂CH₂ | H | |
| S | S | CH₂CH(CH₃)CH₂ | H | |
| S | S | C(CH₃)₂CH₂CH₂ | H | |
| S | S | CH(CH₃)CH(CH₃)CH₂ | H | |
| S | S | CH(CH₃)CH₂CH(CH₃) | H | |
| S | S | CH₂C(CH₃)₂CH₂ | H | |
| S | S | (CH₂)₄ | H | |
| S | S | CH(CH₃)CH₂CH₂CH₂ | H | |
| S | S | CH₂CH(CH₃)CH₂CH₂ | H | |
| S | S | C(CH₃)₂CH₂CH₂CH₂ | H | |
| S | S | CH(CH₃)CH(CH₃)CH₂CH₂ | H | |
| S | S | CH(CH₃)CH₂CH(CH₃)CH₂ | H | |
| S | S | CH(CH₃)CH₂CH(CH₃)CH₂ | H | |
| S | S | CH(CH₃)CH₂CH₂CH(CH₃) | H | |
| S | S | CH₂C(CH₃)₂CH₂CH₂ | H | |
| S | S | CH₂CH(CH₃)CH(CH₃)CH₂ | H | |
| S | S | (CH₂)₅ | H | |
| S | S | CH₂CH₂OCH₂CH₂ | H | |
| S | S | CH₂CH₂SCH₂CH₂ | H | |
| O | O | (CH₂)₂ | CH₃ | 75 |
| O | O | (CH₂)₂ | CH₂CH₃ | |
| O | O | (CH₂)₂ | CH(CH₃)₂ | |
| O | O | (CH₂)₂ | CH₂CH₂CH₃ | |
| O | O | CH(CH₃)CH₂ | CH₃ | |
| O | O | C(CH₃)₂CH₂ | CH₃ | |
| O | O | (CH₂)₃ | CH₃ | |
| O | O | (CH₂)₄ | CH₃ | |
| O | S | (CH₂)₂ | CH₃ | |
| O | S | (CH₂)₂ | CH₂CH₃ | |
| O | S | (CH₂)₂ | CH(CH₃)₂ | |
| O | S | (CH₂)₂ | CH₂CH₂CH₃ | |
| O | S | CH(CH₃)CH₂ | CH₃ | |
| O | S | C(CH₃)₂CH₂ | CH₃ | |
| O | S | (CH₂)₃ | CH₃ | |
| O | S | (CH₂)₄ | CH₃ | |
| S | S | (CH₂)₂ | CH₃ | |
| S | S | (CH₂)₂ | CH₂CH₃ | |
| S | S | (CH₂)₂ | CH(CH₃)₂ | |
| S | S | (CH₂)₂ | CH₂CH₂CH₃ | |
| S | S | CH(CH₃)CH₂ | CH₃ | |
| S | S | C(CH₃)₂CH₂ | CH₃ | |
| S | S | (CH₂)₃ | CH₃ | |
| S | S | (CH₂)₄ | CH₃ | |
| O | O | (CH₂)₂ | OCH₃ | 222-223 |
| O | O | (CH₂)₂ | OCH₂CH₃ | |
| O | O | (CH₂)₂ | OCH(CH₃)₂ | |
| O | O | CH(CH₃)CH₂ | OCH₃ | |
| O | O | (CH₂)₃ | OCH₃ | |
| O | O | (CH₂)₄ | OCH₃ | |
| O | O | (CH₂)₂ | Cl | 79-80 |
| O | O | (CH₂)₂ | Br | |
| O | O | (CH₂)₂ | F | |
| O | O | CH(CH₃)CH₂ | Cl | |
| O | O | CH(CH₃)CH₂ | Br | |
| O | O | CH(CH₃)CH₂ | F | |
| O | O | (CH₂)₃ | Cl | |
| O | O | (CH₂)₃ | Br | |
| O | O | (CH₂)₃ | F | |
| O | O | (CH₂)₄ | Cl | |
| O | O | (CH₂)₄ | Br | |
| O | O | (CH₂)₄ | F | |
| S | S | (CH₂)₂ | OCH₃ | |
| S | S | (CH₂)₂ | OCH₂CH₃ | |
| S | S | (CH₂)₂ | OCH(CH₃)₂ | |
| S | S | CH(CH₃)CH₂ | OCH₃ | |
| S | S | (CH₂)₃ | OCH₃ | |
| S | S | (CH₂)₄ | OCH₃ | |
| S | S | (CH₂)₂ | Cl | |
| S | S | (CH₂)₂ | Br | |
| S | S | (CH₂)₂ | F | |
| S | S | CH(CH₃)CH₂ | Cl | |
| S | S | CH(CH₃)CH₂ | Br | |
| S | S | CH(CH₃)CH₂ | F | |
| S | S | (CH₂)₃ | Cl | |
| S | S | (CH₂)₃ | Br | |
| S | S | (CH₂)₃ | F | |
| S | S | (CH₂)₄ | Cl | |
| S | S | (CH₂)₄ | Br | |
| S | S | (CH₂)₄ | F | |
| O | S | (CH₂)₂ | OCH₃ | |
| O | S | (CH₂)₂ | OCH₂CH₃ | |
| O | S | (CH₂)₂ | OCH(CH₃)₂ | |
| O | S | CH(CH₃)CH₂ | OCH₃ | |
| O | S | (CH₂)₃ | OCH₃ | |
| O | S | (CH₂)₄ | OCH₃ | |
| O | S | (CH₂)₂ | Cl | |
| O | S | (CH₂)₂ | Br | |
| O | S | (CH₂)₂ | F | |
| O | S | CH(CH₃)CH₂ | Cl | |
| O | S | CH(CH₃)CH₂ | Br | |
| O | S | CH(CH₃)CH₂ | F | |
| O | S | (CH₂)₃ | Cl | |
| O | S | (CH₂)₃ | Br | |
| O | S | (CH₂)₃ | F | |
| O | S | (CH₂)₄ | Cl | |
| O | S | (CH₂)₄ | Br | |
| O | S | (CH₂)₄ | F | |
| NCH₃ | O | (CH₂)₂ | H | oil |
| NCH₂CH₃ | O | (CH₂)₂ | H | |
| NCH₃ | O | (CH₂)₃ | H | |
| NCH₃ | O | (CH₂)₄ | H | |
| NCOCH₃ | O | (CH₂)₂ | H | 167-168 |
| NCOCH₃ | O | (CH₂)₃ | H | |
| NCOCH₃ | O | (CH₂)₄ | H | |
| NCOCH₂CH₃ | O | (CH₂)₂ | H | |
| NCOCH(CH₃)₂ | O | (CH₂)₂ | H | 91-93 |
| NCO₂CH₃ | O | (CH₂)₂ | H | 178-180 |
| NCO₂CH₃ | O | CH(CH₃)CH₂ | H | |
| NCO₂CH₃ | O | (CH₂)₃ | H | |
| NCO₂CH₃ | O | (CH₂)₄ | H | |
| NCO₂CH₂CH₃ | O | (CH₂)₂ | H | 75-77 |
| NCO₂CH(CH₃)₂ | O | (CH₂)₂ | H | |
| NCON(CH₃)₂ | O | (CH₂)₂ | H | 198-199 |
| NCON(CH₃)₂ | O | (CH₂)₃ | H | |
| NCON(CH₃)₂ | O | (CH₂)₄ | H | |
| NCON(CH₂CH₃)₂ | O | (CH₂)₂ | H | |
| NCONHCH₃ | O | (CH₂)₂ | H | |
| NCONHCH₂CH₃ | O | (CH₂)₂ | H | |
| NCONHCH₂CH₂CH₃ | O | (CH₂)₂ | H | |
| NCONHCH(CH₃)₂ | O | (CH₂)₂ | H | 181-183 |
| NCO₂CH₂CH₃ | O | (CH₂)₃ | H | 77-79 |
| NCO₂CH₂CH₃ | O | (CH₂)₄ | H | |
| NCH₃ | NCOCH₃ | COCH₂ | H | |
| NCH₃ | NCO₂CH₃ | COCH₂ | H | |
| NCH₃ | NCON(CH₃)₂ | COCH₂ | H | |
| NCH₂CH₃ | NCOCH₃ | COCH₂ | H | |
| NCH₂CH₃ | NCO₂CH₃ | COCH₂ | H | |
| NCH₂CH₃ | NCO₂CH₃ | COCH₂ | H | |
| O | NCOCH₃ | COCH₂ | H | |
| O | NCOCH₂CH₃ | COCH₂ | H | |
| O | NCO₂CH₃ | COCH₂ | H | |
| O | NCO₂CH₂CH₃ | COCH₂ | H | |
| O | NCON(CH₃)₂ | COCH₂ | H | |
| O | NCON(CH₂CH₃)₂ | COCH₂ | H | |
| NCH₃ | NCOCH₃ | COCH(CH₃) | H | |
| NCH₃ | NCO₂CH₃ | COCH(CH₃) | H | |
| NCH₃ | NCON(CH₃)₂ | COCH(CH₃) | H | |
| O | NCOCH₃ | COCH(CH₃) | H | |
| O | NCO₂CH₃ | COCH(CH₃) | H | |
| O | NCON(CH₃)₂ | COCH(CH₃) | H | |
| NCH₃ | O | COCH₂ | H | |
| NCH₃ | O | COCH₂CH₂ | H | |
| NCH₃ | O | COCH(CH₃) | H | |
| NCH₃ | NCH₃ | COCO | H | |
| NCH₃ | NCH₃ | COCH₂CO | H | |
| NCH₃ | O | COCO | H | |
| NCH₃ | O | COCH₂CO | H | |
| NCH₃ | NCH₃ | - | CH₃ | |
| NCH₂CH₃ | NCH₃ | - | CH₃ | |
| NCH₂CH₃ | NCH₂CH₃ | - | CH₃ | |
| NCH₃ | NH | (CH₂)₂ | CH₃ | |
| NCH₃ | NCOCH₃ | (CH₂)₂ | CH₃ | |
| NCH₃ | NCOCH₂CH₃ | (CH₂)₂ | CH₃ | |
| NCH₃ | NCOCH₃ | (CH₂)₃ | CH₃ | |
| NCH₃ | NCO₂CH₃ | (CH₂)₂ | CH₃ | |
| NCH₃ | NCO₂CH₃ | (CH₂)₃ | CH₃ | |
| NCH₃ | NCO₂CH₃ | (CH₂)₄ | CH₃ | |
| NCH₃ | NCOCH₃ | (CH₂)₄ | CH₃ | |
| NCH₃ | S | (CH₂)₂ | CH₃ | |
| NCH₃ | S | (CH₂)₃ | CH₃ | |
| NCH₃ | S | (CH₂)₄ | CH₃ | |
| NCOCH₃ | S | (CH₂)₂ | CH₃ | |
| NCO₂CH₃ | S | (CH₂)₃ | CH₃ | |
| NCH₃ | NCH₃ | - | H | 174-176 |
| NCH₂CH₃ | NCH₃ | - | H | |
| NCH₂CH₃ | NCH₂CH₃ | - | H | |
| NCH₃ | NH | (CH₂)₂ | H | |
| NCH₃ | NCOCH₃ | (CH₂)₂ | H | |
| NCH₃ | NCOCH₂CH₃ | (CH₂)₂ | H | |
| NCH₃ | NCOCH₃ | (CH₂)₃ | H | |
| NCH₃ | NCO₂CH₃ | (CH₂)₂ | H | |
| NCH₃ | NCO₂CH₃ | (CH₂)₃ | H | |
| NCH₃ | NCOCH₃ | (CH₂)₃ | H | |
| NCH₃ | NCO₂CH₃ | (CH₂)₄ | H | |
| NCH₃ | NCOCH₃ | (CH₂)₄ | H | |
| NCH₃ | S | (CH₂)₂ | H | |
| NCH₃ | S | (CH₂)₃ | H | |
| NCH₃ | S | (CH₂)₄ | H | |
| NCOCH₃ | S | (CH₂)₂ | H | |
| NCO₂CH₃ | S | (CH₂)₃ | H | |
| O | 0 | COCH₂ | H | |
| O | 0 | COCH₂CH₂ | H | |
| O | 0 | COCH₂CH(CH₃) | H | |
| O | 0 | COCO | H | |
| O | 0 | COCH₂CO | H | |
| O | 0 | COCH=CH | H | |
| O | 0 | COCH=C(CH₃) | H | |
| O | 0 | CH=CH | H | |
| O | 0 | CH=CHCH₂ | H | |
| O | 0 | (CH₃)=CHCH₂ | H | |
| O | 0 | CH=CHCH(CH₃) | H | |
| O | S | COCH₂ | H | |
| O | O | CH₂CH(CO₂CH₃) | H | |

### EXAMPLE 35

### Preparation of Sodium 5-(1,3-dioxolan-2-yl)-2-(4-isopropyl-4-methyl-5-oxo-2-imidazolin-2-yl)nicotinate

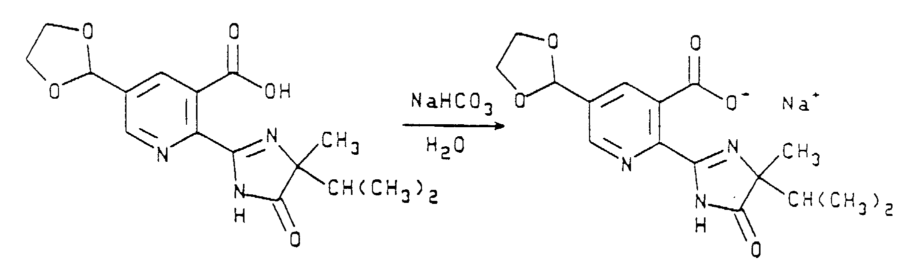

5-(1,3-Dioxolan-2-yl)-2-(4-isopropyl-4-methyl-5-oxo-2-imidazolin-2-yl)nicotinic acid (0.507 g, 0.00152 mol), sodium bicarbonate (0.12 g, 0.00143 mol), water and tetrahydrofuran is heated at reflux temperature for 1 hour. Concentration in vacuo yields a solid which is triturated with ether to give the title compound as an orange solid (0.39 g, 72%), mp 209-210°C, identified by IR and NMR spectral analyses.

### EXAMPLE 36

### Preparation of 5-(1,3-Dioxolan-2-yl)-2-(4-isopropyl-4-methyl-5-oxo-2-imidazolin-2-yl)nicotinic acid, compound with diisopropylamine

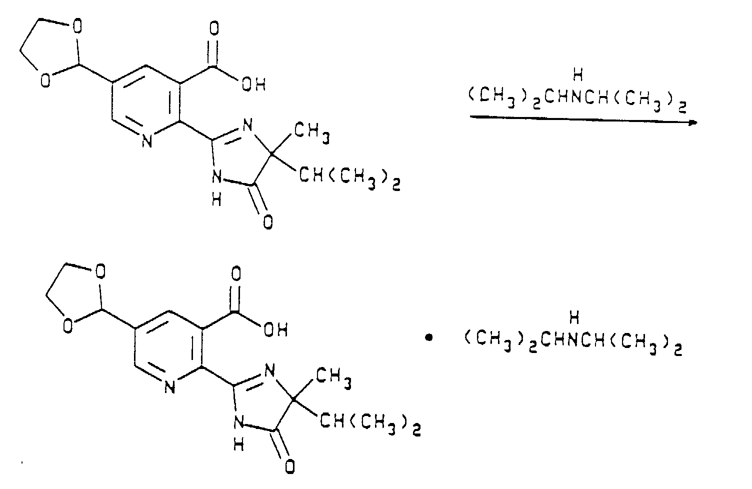

A solution of 5-(1,3-dioxolan-2-yl)-2-(4-isopropyl-4-methyl-5-oxo-2-imidazolin-2-yl)nicotinic acid (0.505 g, 0.00152 mol), diisopropylamine (0.23 g, 0.00163 mol) and tetrahydrofuran is heated for one hour and 30 minutes. Concentration in vacuo gives the title compound as a pale yellow solid (0.54 g, 82%), mp 102-104°C, identified by IR and NMR spectral analyses.

### EXAMPLE 37

### Preparation of 7-(1,3-Dioxolan-2-yl)-3-isopropyl-3-methyl-5H-imidazo[1',2':1,2]pyrrolo[3,4-b]pyridine -2(3H), 5-dione

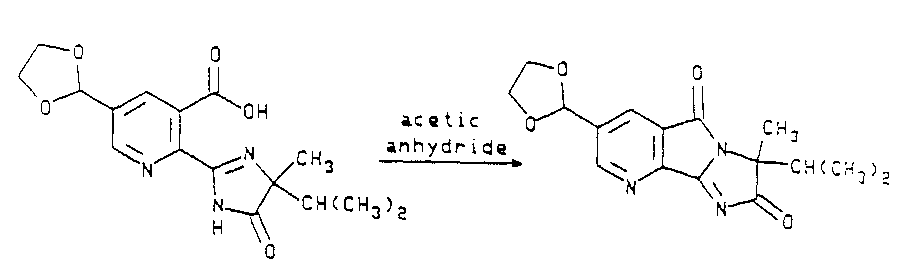

5-(1,3-Dioxolan-2-yl)-2-(4-isopropyl-4-methyl-5-oxo-2-imidazolin-2-yl)nicotinic acid (10.02 g, 0.03 mol) and acetic anhydride in toluene is heated at reflux temperature for 5 hours. Concentration in vacuo gives the title compound as an orange solid (11.10 g, 100%), mp 146-148°C, identified by IR and NMR spectral analyses.

### EXAMPLE 38

### Preparation of 7-(1,3-Dioxolan-2-yl)-2-isopropyl-2-methyl-5H-imidazo[1',2':1,2]pyrrolo[3,4-b]pyridine -3(2H), 5-dione

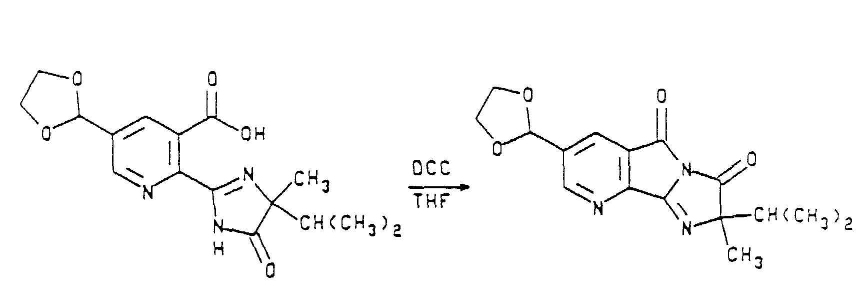

A solution of 5-(1,3-dioxolan-2-yl)-2-(4-isopropyl-4-methyl-5-oxo-2-imidazolin-2-yl)nicotinic acid (1.0 g, 0.003 mol) and dicyclohexylcarbodiimide (0.65 g, 0.003 mol) in tetrahydrofuran (5 mL) is stirred at reflux temperature for 1.5 hours. After cooling, the reaction mixture is filtered. The filtrate is concentrated in vacuo to afford the title compound (0.68 g, 72%) as a pale yellow solid, mp 102-104°C, identified by IR and NMR spectral analyses.

### EXAMPLE 39

### Preparation of Methyl 2-(4-isopropyl-4-methyl-5-oxo-2-imidazolin-2-yl)-5-(1,3-dioxolan-2-yl)nicotinate

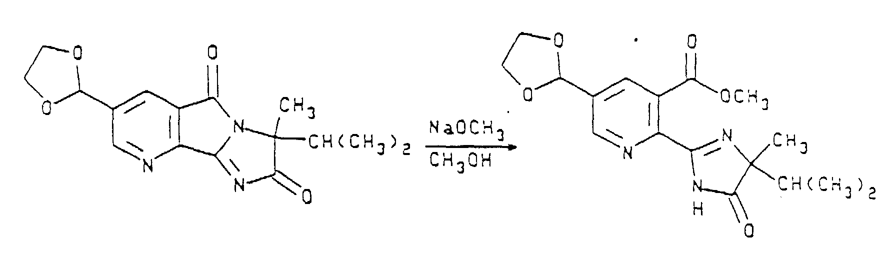

7-(1,3-Dioxolan-2-yl)-3-isopropyl-3-methyl-5H-imidazo[1',2':1,2]pyrrolo[3,4-b]pyridine-2(3H), 5-dione (4.05 g, 0.0128 mol) and sodium methoxide (0.73 g, 0.0135 mol) in methanol is heated at reflux temperature for 40 minutes then cooled to room temperature. The reaction mixture is quenched with 2 molar hydrochloric acid, poured into water and extracted with methylene chloride. The combined methylene chloride extracts are dried over anhydrous magnesium sulfate and concentrated in vacuo to give the title compound as an orange oil (3.88 g, 87%), identified by IR and NMR spectral analyses.

### EXAMPLE 40

### Preemergence herbicidal evaluation of test compounds

The preemergence herbicidal activity of the compounds of the present invention is exemplified by the following tests in which the seeds of a variety of monocotyledonous and dicotyledonous plants are separately mixed with potting soil and planted on top of approximately one inch of soil in separate pint cups. After planting, the cups are sprayed with the selected aqueous acetone solution containing test compound in sufficient quantity to provide the equivalent of about 0.016 to 8.0 kg per hectare of test compound per cup. The treated cups are then placed on greenhouse benches, watered and cared for in accordance with conventional greenhouse procedures. From four to five weeks after treatment, the tests are terminated and each cup is examined.

| **PLANT SPECIES EMPLOYED IN HERBICIDAL EVALUATIONS** | | |
|---|---|---|
| **HEADER ABB** | **COMMON NAME** | **SCIENTIFIC NAME** |
| BARNYARDGR | BARNYARDGRASS | ECHINOCHLOA CRUS-GALLI, (L)BEAU |
| | | |
| FOXTAIL SP P NUTSEDGE WILD OATS | FOXTAIL SPP. NUTSEDGE,PURPLE OAT, WILD | SETARIA SPP. CYPERUS RONTUNDUS, L. AVENA FATUA, L. |
| | | |
| QUACKGRASS | QUACKGRASS | AGROPYRON REPENS, (L)BEAUV |
| | | |
| FLD BINDWD | BINDWEED, FIELD (RHIZOME) | CONVOLVULUS ARVENSIS, L. |
| | | |
| MRNGLRY SP | MORNINGGLORY SPP. | IPOMOEA SPP. |
| | | |
| WILD MUSTD | MUSTARD, WILD | BRASSICA KABER, (DC)L.C.WHEELR |
| | | |
| RAGWEED | RAGWEED, COMMON | AMBROSIA ARTEMISIIFOLIA, L. |
| | | |
| VELVETLEAF | VELVETLEAF | ABUTILON THEOPHRASTI, MEDIC. |
| | | |
| SUGARBEETS CORN FIELD, COTTON SOYBEAN | BUGARBEETS CORN, FIELD COTTON SOYBEAN | BETA VULGARIS, L. ZEA MAYS, L. GOSSYPIUM HIRSUTUM, L. GLYCINE MAX |

| **COMPOUNDS EVALUATED AS HERBICIDAL AGENTS** | |
|---|---|
| **Compound No.** | |
| 1 | 5-(1,3-dioxolan-2-yl)-2-(4-isopropyl-4-methyl-5-oxo-2-imidazolin-2-yl)nicotinic acid |
| | |
| 2 | 5-(5,5-dimethyl-1,3-dioxan-2-yl)-2-(4-isopropyl-4-methyl-5-oxo-2-imidazolin-2-yl)nicotinic acid |
| | |
| 3 | 5-(1,3-dioxan-2-yl)-2-(4-isopropyl-4-methyl-5-oxo-2-imidazolin-2-yl)nicotinic acid |
| | |
| 4 | 6-chloro-5-(1,3-dioxolan-2-yl)-2-(4-isopropyl-4-methyl-5-oxo-2-imidazolin-2-yl)nicotinic acid |
| | |
| 5 | 5-(1,3-dioxolan-2-yl)-2-(4-isopropyl-4-methyl-5-oxo-2-imidazolin-2-yl)-6-methoxynicotinic acid |
| | |
| 6 | 5-(1,2-dimethyl-3-diaziridinyl)-2-(4-isopropyl-4-methyl-5-oxo-2-imidazolin-2-yl)nicotinic acid |
| | |
| 7 | 5-(1,3-dioxolan-2-yl)-2-(4-isopropyl-4-methyl-5-oxo-2-imidazolin-2-yl)-6-methylnicotinic acid |
| | |
| 8 | 2-(4-isopropyl-4-methyl-5-oxo-2-imidazo-lin-2-yl)-5-(1,3-oxathiolan-2-yl)-nicotinic acid |
| | |
| 9 | 5-(1,3-dioxepan-2-yl)-2-(4-isopropyl-4-methyl-5-oxo-2-imidazolin-2-yl)nicotinic acid |
| | |
| 10 | 5-(1,3-dioxolan-2-yl)-2-(4-isopropyl-4-methyl-5-oxo-2-imidazolin-2-yl)nicotinic acid, compound with diisopropylamine |
| | |
| 11 | 5-(1,3-dioxolan-2-yl)-2-(4-isopropyl-4-methyl-5-oxo-2-imidazolin-2-yl)nicotinic acid, sodium salt |
| | |
| 12 | methyl 2-(4-isopropyl-4-methyl-5-oxo-2-imidazolin-2-yl)-5-(1,3-dioxolan-2-yl)-nicotinate |
| | |
| 13 | 2-(4-isopropyl-4-methyl-5-oxo-2-imidazolin-2-yl)-5-(3-methyl-2-oxazolidinyl)nicotinic acid, compound with 2-(methylamino)ethanol |
| | |
| 14 | 2-(4-isopropyl-4-methyl-5-oxo-2-imidazolin-2-yl)-5-(4-methyl-1,3-dioxolan-2-yl)nicotinic acid |
| | |
| 15 | ammonium 5-(1-acetyl-3-methyl-2-imidasolidinyl)-2-(4-isopropyl-4-methyl-5-oxo-2-imidazolin-2-yl)nicotinate |
| | |
| 16 | 5-(3-acetyl-2-oxazolidinyl)-2-(4-isopropyl-4-methyl-5-oxo-2-imidazolin-2-yl)nicotinic acid |
| | |
| 17 | 5-(3-carboxy-2-oxazolidinyl)-2-(4-isopropyl-4-methyl-5-oxo-2-imidazolin-2-yl)nicotinic acid, 5-ethyl ester |
| | |
| 18 | 5-[3-(dimethylcarbamoyl)-2-oxazolidinyl]-2-(4-isopropyl-4-methyl-5-oxo-2-imidazolin-2-yl)nicotinic acid, 5-methyl ester |
| | |
| 19 | 5-(3-carboxy-2-oxazolidinyl)-2-(4-isopropyl-4-methyl-5-oxo-2-imidazolin-2-yl)nicotinic acid, 5-methyl ester |
| | |
| 20 | methyl 2-(4-isopropyl-4-methyl-5-oxo-2-imidazolin-2-yl)-5-(4-methyl-6-oxo-1,3-dioxan-2-yl)nicotinate |
| | |
| 21 | 5-(1,3-dithiolan-2-yl)-2-(4-isopropyl-4-methyl-5-oxo-2-imidazolin-2-yl)-nicotinic acid |
| | |
| 22 | 7-(1,3-dioxolan-2-yl)-3-isopropyl-3-methyl-5H-imidazo[1',2':1,2]pyrrolo-[3,4-b]pyridine-2(3H), 5-dione |
| | |
| 23 | 7-(1,3-dioxolan-2-yl)-2-isopropyl-2-methyl-5H-imidazo[1',2':1,2]pyrrolo-[3,4-b]pyridine-3(2H), 5-dione |
| | |
| 24 | 5-(3-isobutyryl-2-oxazolidinyl)-2-(4-isopropyl-4-methyl-5-oxo-2-imidazolin-2-yl)nicotinic acid |
| | |
| 25 | ethyl 2-[5-carboxy-6-(4-isopropyl-4-methyl-5-oxo-2-imidazolin-2-yl)-3-pyridyl]tetrahydro-2H-1,3-oxazine-3-carboxylate |

### EXAMPLE 41

### Postemergence herbicidal evaluation of test compounds

The postemergence herbicidal activity of the compounds of the present invention is determined by the following tests, wherein a variety of monocotyledonous and dicotyledonous plants are treated with test compounds dispersed in aqueous acetone mixtures. In the tests, seedling plants are grown in jiffy flats for about two weeks. The test compounds are dispersed in 50/50 acetone/water mixtures containing 0.5% TWEEN® 20, a polyoxyethylene sorbitan monolaurate surfactant of Atlas Chemical Industries, in sufficient quantities to provide the equivalent of about 0.16 kg to 8.0 kg per hectare of active compound when applied to the plants through a spray nozzle operating at 40 psig for a predetermined time. After spraying, the plants are placed on greenhouse benches and are cared for in the usual manner, commensurate with conventional greenhouse practices. From four to five weeks after treatment, the seedling plants are examined and rates according to the rating system provided in Example 59above. The data obtained are recorded in Table II below. The compounds evaluated are reported by compound number given in Example 40.

## Claims (Claims for the following Contracting State(s): AT, BE, CH, DE, DK, FR, GB, IT, LI, LU, NL, SE)

1. A compound characterized by the structure 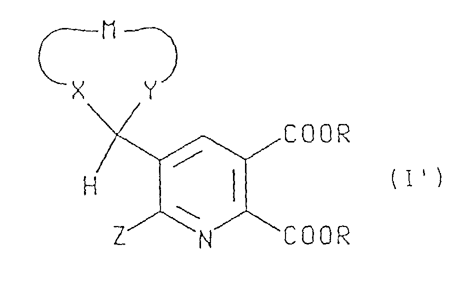 wherein
R is C₁-C₆ alkyl;
X and Y are each independently oxygen, sulfur or NR₄;
R₄ is hydrogen, C₁-C₆ alkyl optionally substituted with C₁-C₄ alkoxy or 1-3 halogens, SO₂R₅, COR₅, CO₂R₅ or CONR₅R₅;
R₅ is hydrogen, C₁-C₆ alkyl optionally substituted with 1-3 halogens, or C₂-C₆ alkenyl;
M is C₂-C₅ alkylene optionally substituted with 1 or 2 C₁-C₄ alkyl groups, C₁-C₄ alkoxy, halogen, CO₂R₆ or oxo, and optionally interrupted by one oxygen or one sulfur,
C₂ alkenylene optionally substituted with 1 or 2 C₁-C₄ alkyl groups or CO₂R₆,
C₃ alkenylene optionally substituted with 1 or 2 C₁-C₄ alkyl groups, CO₂R₆ or oxo,
methyleneamino, optionally substituted with C₁-C₄ alkyl or CO₂R_{6'} or
a single bond, with the proviso that both X and Y are NR₄,
provided that the ring formed by M, X and Y and the carbon to which they are attached is no more than 8 atoms and provided that when the substituents on M are either alkoxy or halogen the substituted carbon is not bound to X or Y;
R₆ is hydrogen, methyl or ethyl;
Z is hydrogen, halogen, C₁-C₆ alkoxy, or C₁-C₆ alkyl optionally substituted with C₁-C₄ alkoxy or halogen.

2. The compound according to claim 1 wherein Z is hydrogen, halogen or methyl; X and Y are oxygen and M is unsubstituted alkylene.

3. The compound according to claim 2 dimethyl 5-(1,3-dioxolan-2-yl)pyridine-2,3-dicarboxylate.

4. The compound according to claim 2, dimethyl 5-(1,3-dioxan-2-yl)pyridine-2,3-dicarboxylate.

5. The compound according to claim 2, dimethyl 5-(1,3-dioxolan-2-yl)-6-methylpyridine-2,3-dicarboxylate.

6. The compound according to claim 2, dimethyl 5-(1,3-dioxepan-2-yl)pyridine-2,3-dicarboxylate.

7. A method for the preparation of a compound characterized by the structure 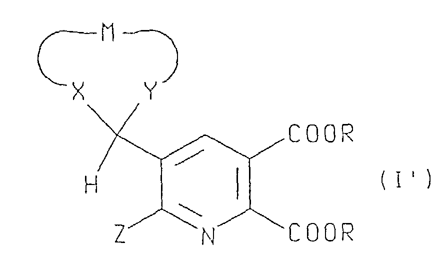 wherein R, Z, X, M and Y are as described in claim 1 which comprises reacting a compound having the structure 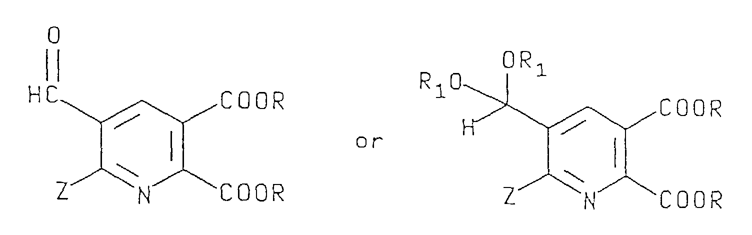 wherein R₁ is C₁-C₄ alkyl and R and Z are as described above with at least one equivalent of an agent having the structure
HX-M-YH
wherein X, M and Y are as described above in the presence of an acid and a solvent.

8. A method for the preparation of a compound characterized by the formula I' structure 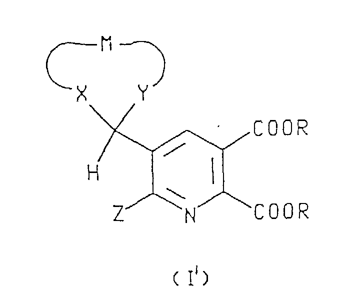 wherein R, Z, X, M and Y are as described in claim 1 which comprises reacting a compound having the structure 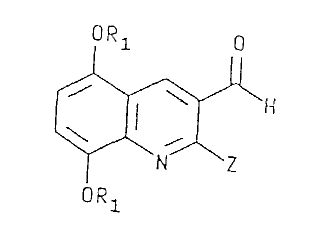 wherein R₁ is C₁-C₄ alkyl, with at least 2 molar equivalents of a tri(C₁-C₄)alkylorthoformate in the presence of a C₁-C₄ alkyl alcohol, ozone and a catalytic amount of an acid to yield a di(C₁-C₄)alkyl acetal pyridinedicarboxylate having the structure 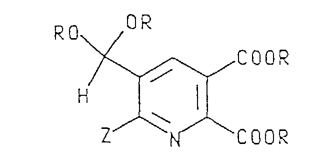 wherein R is C₁-C₄ alkyl and reacting said di(C₁-C₄)-alkyl acetal with at least one molar equivalent of an agent having the structure
HX-M-YH
wherein X, M and Y are as described above in the presence of an acid and a solvent to give the formula I 5-heterocyclic-pyridine-2,3-dicarboxylate compound.

9. A method for the preparation of a compound characterized by the formula I' structure 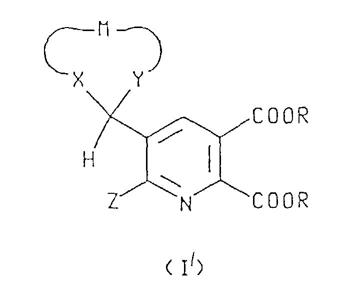 wherein R, Z, X, M and Y are as described in claim 1, which comprises reacting a compound having the structure 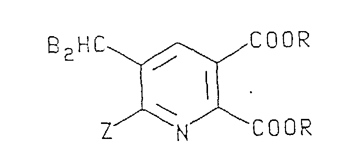 wherein B is halogen, with at least 2 molar equivalents of AgNO₃ in the presence of a C₁-C₄ alkyl alcohol at a temperature greater than about 25°C to yield an intermediate compound having the structure 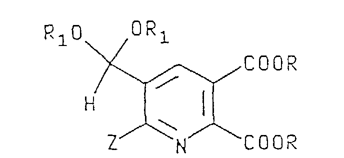 wherein R₁ is C₁-C₄ alkyl and reacting said intermediate compound with an agent having the structure
HX-M-YH
wherein X, M and Y are as described above in the presence of an acid and a solvent to give the formula I 5-heterocyclic pyridine-2,3-dicarboxylate compound.

10. A compound characterized by the structure: 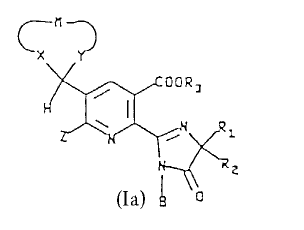 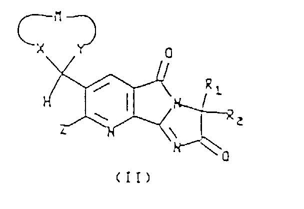 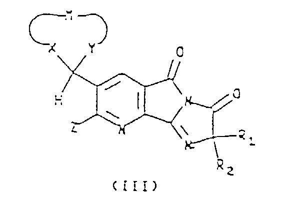 wherein
R₁ is C₁-C₄ alkyl;
R₂ is C₁-C₄ alkyl or C₃-C₆ cycloalkyl and when R₁ and R₂ are taken together with the carbon to which they are attached they may represent C₃-C₆ cycloalkyl;
R₃ is hydrogen,
C₁-C₆ alkyl optionally substituted with one of the following groups: C₁-C₃ alkoxy, halogen or phenyl,
C₃-C₆ alkenyl optionally substituted with one of the following groups: C₁-C₃ alkoxy, phenyl or halogen,
C₃-C₆ alkynyl,
C₃-C₆ cycloalkyl optionally substituted with C₁-C₃ alkyl, or
a cation of alkali metals, ammonium or organic ammonium;
and wherein X, Y, Z, M are as defined in claim 1,
and wherein B is hydrogen or COR₇, provided that when B is COR₇, R₃ is other than hydrogen or a cation;
R₇ is C₁-C₅ alkyl or phenyl optionally substituted with halogen, nitro or methoxy;
the N-oxides thereof, when R₃ is not unsaturated alkyl and when M is not alkenylene;
the optical isomers thereof, when R₁ and R₂ represent different substitutents;
the acid addition salts thereof, when R₃ is other than a cation and the tautomers thereof.

11. The compound according to claim 10 characterized by the structure 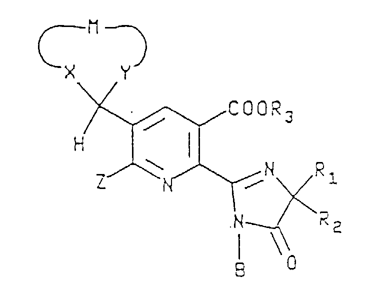 wherein R₁, R₂, R₃, B, X, Y, M and Z are as described in claim 10.

12. The compound according to claim 11 wherein M, X, Y, R₅, R₆ and R₇ are as described therein, Z is hydrogen, C₁-C₄ alkyl, C₁-C₄ alkoxy or halogen, B is hydrogen, R₁ is methyl, R₂ is isopropyl and R₃ is hydrogen, C₁-C₆ alkyl, or a cation of alkali metals, ammonium or organic ammonium.

13. The compound according to claim 12, 5-(1,3-dioxolan-2-yl)-2-(4-isopropyl-4-methyl-5-oxo-2-imidazolin-2-yl)nicotinic acid.

14. The compound according to claim 12, 5-(1,3-dioxan-2-yl)-2-(4-isopropyl-4-methyl-5-oxo-2-imidazolin-2-yl)nicotinic acid.

15. The compound according to claim 12, 5-(1,3-dioxolan-2-yl)-2-(4-isopropyl-4-methyl-5-oxo-2-imidazolin-2-yl)-6-methylnicotinic acid.

16. The compound according to claim 12, 5-(1,3-dioxepan-2-yl)-2-(4-isopropyl-4-methyl-5-oxo-2-imidazolin-2-yl)nicotinic acid.

17. A method for controlling undesirable plant species characterized by applying to the foliage of said plants or to the soil or water containing seeds or other propagating organs thereof, a herbicidally effective amount of a compound according to claim 10.

18. The method according to claim 17 wherein the compound has the structure 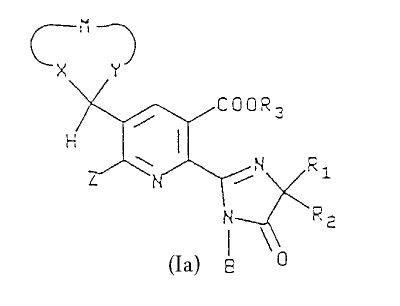 and R₁, R₂, R₃, B, X, Y, M and Z are as described in claim 10.

19. The method according to claim 18, wherein the compound is selected from the group consisting of 5-(1,3-dioxolan-2-yl)-2-(4-isopropyl-4-methyl-5-oxo-2-imidazolin-2-yl)nicotinic acid; 5-(1,3-dioxan-2-yl)-2-(4-isopropyl-4-methyl-5-oxo-2-imidazolin-2-yl)nicotinic acid; 5-(1,3-dioxolan-2-yl)-2-(4-isopropyl-4-methyl-5-oxo-2-imidazolin-2-yl)-6-methylnicotinic acid; and 5-(1,3-dioxepan-2-yl)-2-(4-isopropyl-4-methyl-5-oxo-2-imidazolin-2-yl)nicotinic acid.

20. A composition for controlling undesirable plant species characterized by a 5-heterocyclic 2-(2-imidazolin-2-yl)pyridine compound as described in claim 10 and an agronomically acceptable carrier.

21. A method for the preparation of a 5-heterocyclic 2-(2-imidazolin-2-yl)pyridine compound characterized by the structure of formula I 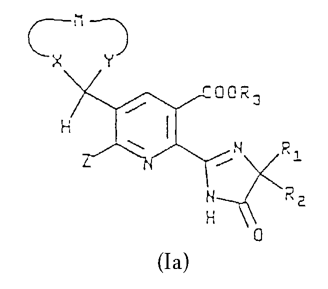 wherein R₃ is hydrogen and R₁, R₂, Z, X, M and Y are as described in claim 1 which comprises reacting a 5-heterocyclic pyridinedicarboxylate compound having the structure 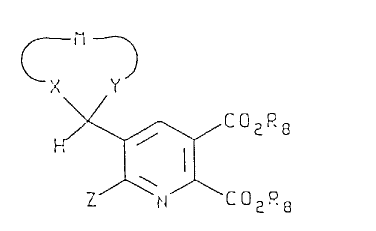 wherein R₈ is C₁-C₄ alkyl with a base to obtain the corresponding 5-heterocyclic 2,3-pyridinedicarboxylic acid; reacting said pyridinedicarboxylic acid with at least one equivalent of an acid anhydride to obtain a 5-heterocyclic-2,3-pyridinedicarboxylic acid anhydride, reacting said pyridinedicarboxylic acid anhydride with at least one equivalent of an aminocarboxamide having the structure 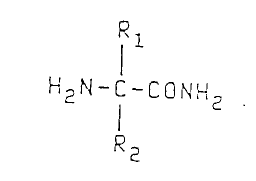 wherein R₁ and R₂ are as described above for formula I in the presence of an inert organic solvent to obtain a nicotinic acid having the structural formula 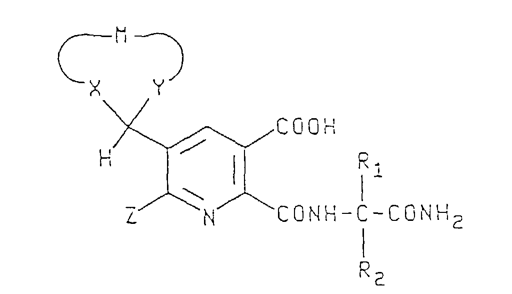 reacting said nicotinic acid with a base, in the presence of a polar solvent to obtain a 5-heterocyclic-2-(2-imidazolin-2-yl)nicotinate and treating said nicotinate with dilute aqueous acid to form a 5-heterocyclic 2-(2-imidazolin-2-yl)pyridine compound of formula I wherein R₃ is hydrogen.

22. A method for the preparation of a 5-heterocyclic 2-(2-imidazolin-2-yl)pyridine compound characterized by the formula I structure 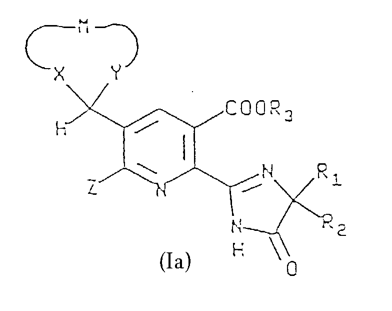 wherein R₃ is hydrogen and R₁, R₂, X, M, Y and Z are as described in claim 10 which comprises reacting a compound having the structure 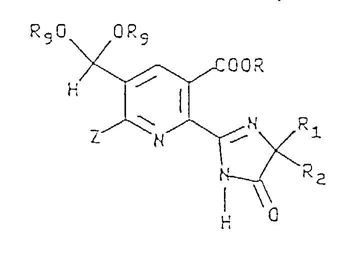 or 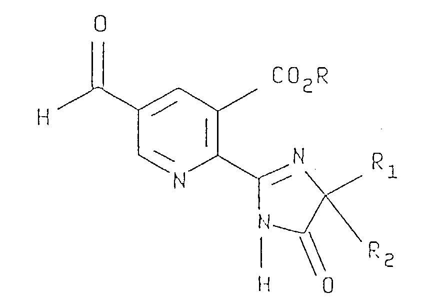 wherein R₉ is C₁-C₄ alkyl with at least two equivalents of hydrochloric acid in water to give the corresponding 5-formyl-2-(5-oxo-2-imidazolin-2-yl)nicotinic acid intermediate, reacting said intermediate with a compound having the structural formula
HX-M-YH
wherein M, X and Y are as described above in the presence of an inert organic solvent at an elevated temperature to form a 5-heterocyclic 2-(2-imidazolin-2-yl)-pyridine compound of formula I wherein R₃ is hydrogen.

## Claims (Claims for the following Contracting State(s): GR)

1. A method for the preparation of a compound characterized by the structure 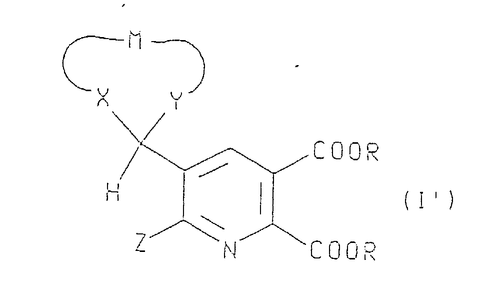 wherein
R is C₁-C₆ alkyl;
X and Y are each independently oxygen, sulfur or NR₄; R₄ is hydrogen, C₁-C₆ alkyl optionally substituted with C₁-C₄ alkoxy or 1-3 halogens, SO₂R₅, COR₅, CO₂R₅ or CONR₅R₅;
R₅ is hydrogen, C₁-C₆ alkyl optionally substituted with 1-3 halogens, or C₂-C₆ alkenyl;
M is C₂-C₅ alkylene optionally substituted with 1 or 2 C₁-C₄ alkyl groups, C₁-C₄ alkoxy, halogen, CO₂R₆ or oxo, and optionally interrupted by one oxygen or one sulfur,
C₂ alkenylene optionally substituted with 1 or 2 C₁-C₄ alkyl groups or CO₂R₆,
C₃ alkenylene optionally substituted with 1 or 2 C₁-C₄ alkyl groups, CO₂R₆ or oxo,
methyleneamino, optionally substituted with C₁-C₄ alkyl or CO₂R₆, or
a single bond, with the proviso that both X and Y are NR₄,
provided that the ring formed by M, X and Y and the carbon to which they are attached is no more than 8 atoms and provided that when the substituents on M are either alkoxy or halogen the substituted carbon is not bound to X or Y;
R₆ is hydrogen, methyl or ethyl;
Z is hydrogen, halogen C₁-C₆ alkoxy, or C₁-C₆ alkyl optionally substituted with C₁-C₄ alkoxy or halogen
which comprises reacting a compound having the structure 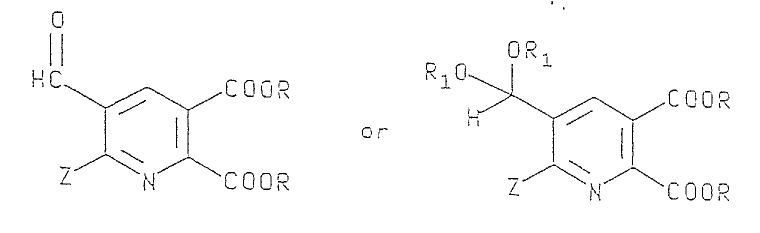 wherein R₁ is C₁-C₄ alkyl and R and Z are as described above with at least one equivalent of an agent having the structure
HX-M-YH
wherein X, M and Y are as described above in the presence of an acid and a solvant.

2. The method according to claim 1 wherein Z is hydrogen, halogen or methyl; X and Y are oxygen and M is unsubstituted alkylene.

3. The method according to claim 2 wherein the compound is dimethyl 5-(1,3-dioxolan-2-yl) pyridine-2,3-dicarboxylate.

4. The method according to claim 2 wherein the compound is dimethyl 5-(1,3-dioxan-2-yl)pyridine-2,3-dicarboxylate.

5. The method according to claim 2 wherein the compound is dimethyl 5-(1,3-dioxolan-2-yl)-6-methylpyridine-2,3-dicarboxylate.

6. The method according to claim 2, wherein the compound is dimethyl 5-(1,3-dioxepan-2-yl)pyridine-2,3-dicarboxylate.

7. A method for the preparation of a compound characterized by the formula I' structure 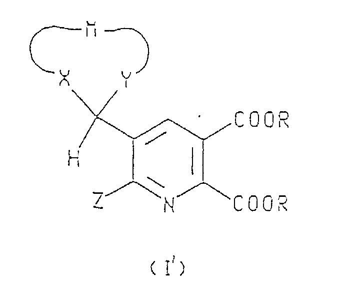 wherein R, Z, X, M, and Y are as described in claim 1 which comprises reacting a compound having the structure 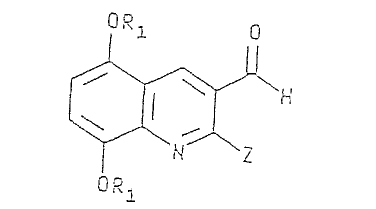 wherein R₁ is C₁-C₄ alkyl, with at least 2 molar equivalents of a tri(C₁-C₄)alkylorthoformate in the presence of a C₁-C₄ alkyl alcohol, ozone and a catalytic amount of an acid to yield a di(C₁-C₄)alkyl acetal pyridinedicarboxylate having the structure 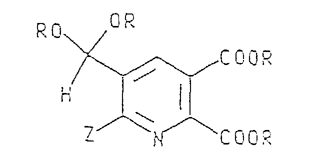 wherein R is C₁-C₄alkyl and reacting said di(C₁-C₄)-alkyl acetal with at least one molar equivalent of an agent having the structure
HX-M-YH
wherein X, M and Y are as described above in the presence of an acid and a solvent to give the formula I 5-heterocyclic-pyridine-2,3-dicarboxylate compound.

8. A method for the preparation of a compound characterized by the formula I' structure 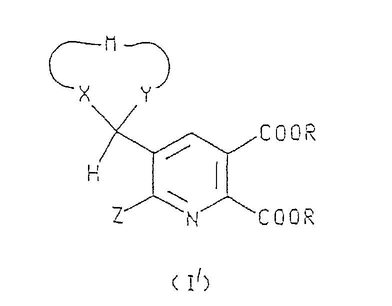 wherein R, Z, X, M and Y are as described in claim 1, which comprises reacting a compound having the structure 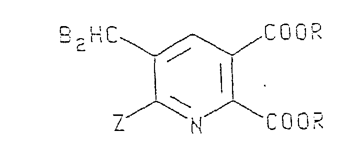 wherein B is halogen, with at least 2 molar equivalents of AgNO₃ in the presence of a C₁-C₄ alkyl alcohol at a temperature greater than about 25°C to yield an intermediate compound having the structure 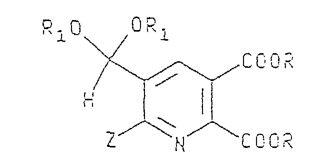 wherein R₁ is C₁-C₄ alkyl and reacting said intermediate compound with an agent having the structure
HX-M-YH
wherein X, M and Y are as described above in the presence of an acid and a solvent to give the formula I 5-heterocyclic pyridine-2,3-dicarboxylate compound.

9. A method for controlling undesirable plant species characterized by applying to the foliage of said plants or to the soil or water containing seeds or other propagating organs thereof, a herbicidally effective amount of a compound characterized by the structure: 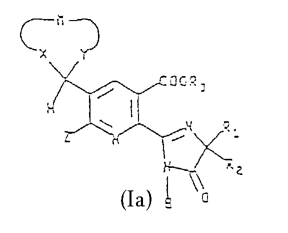 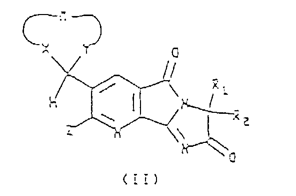 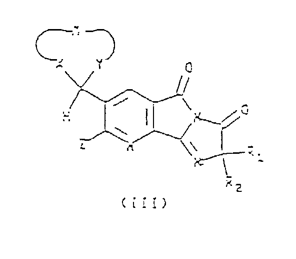 wherein
R₁ is C₁-C₄ alkyl;
R₂ is C₁-C₄ alkyl or C₃-C₆ cycloalkyl and when R₁ and R₂ are taken together with the carbon to which they are attached they may represent C₃-C₆ cycloalkyl;
R₃ is hydrogen,
C₁-C₆ alkyl optionally substituted with one of the following groups; C₁-C₃ alkoxy, halogen or phenyl,
C₃-C₆ alkenyl optionally substituted with one of the following groups: C₁-C₃ alkoxy, phenyl or halogen,
C₃-C₆ alkynyl,
C₃-C₆ cycloalkyl optionally substituted with C₁-C₃ alkyl, or
a cation of alkali metals, ammonium or organic ammonium;
and wherein X, Y, Z, M are as defined in claim 1, and wherein
B is hydrogen or COR₇, provided that when B is COR₇, R₃ is other than hydrogen or a cation;
R₇ is C₁-C₅ alkyl or phenyl optionally substituted with halogen, nitro or methoxy;
the N-oxides thereof, when R₃ is not unsaturated alkyl and when is not alkenylene;
the optical isomers thereof, when R₁ and R₂ represent different substitutents;
the acid addition salts thereof, when R₃ is other than a cation and the tautomers thereof.

10. The method according to claim 9 wherein the compound is characterized by the structure 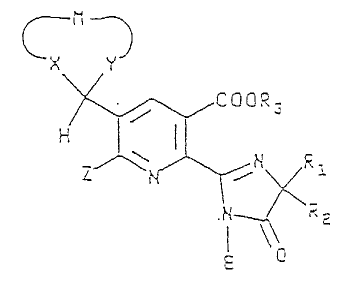 wherein R₁, R₂, R₃, B, X, Y, M and Z are as described in claim 9.

11. The method according to claim 10 wherein
M, X, Y, R₅, R₆ and R₇ are as described therein, Z is hydrogen, C₁-C₄ alkyl, C₁-C₄ alkoxy or halogen, B is hydrogen, R₁ is methyl, R₂ is isopropyl and R₃ is hydrogen, C₁-C₆ alkyl, or a cation of alkali metals, ammonium or organic ammonium.

12. The method according to claim 11 wherein the compound is 5-(1,3-dioxolan-2-yl)-2-(4-isopropyl-4-methyl-5-oxo-2-imidazolin-2-yl)nicotinic acid.

13. The compound according to claim 11 wherein the compound is 5-(1,3-dioxan-2-yl)-2-(4-isopropyl-4-methyl-5-oxo-2-imidazolin-2-yl)nicotinic acid.

14. The method according to claim 12 wherein the compound is 5-(1,3-dioxolan-2-yl)-2-(4-isopropyl-4-methyl-5-oxo-2-imidazolin-2-yl)-6-methylnicotinic acid.

15. The method according to claim 12 wherein the compound is 5-(1,3-dioxepan-2-yl)-2-(4-isopropyl-4-methyl-5-oxo-2-imidazolin-2-yl)nicotinic acid.

16. The method according to claim 10 wherein the compound is selected from the group consisting of 5-(1,3-dioxolan-2-yl)-2-(4-isopropyl-4-methyl-5-oxo-2-imidazolin-2-yl)nicotinic acid; 5-(1,3-dioxan-2-yl)-2-(4-isopropyl-4-methyl-5-oxo-2-imidazolin-2-yl)nicotinic acid; 5-(1,3-dioxolan-2-yl)-2-(4-isopropyl-4-methyl-5-oxo-2-imidazolin-2-yl)-6-methylnicotinic acid; and 5-(1,3-dioxepan-2-yl)-2-(4-isopropyl-4-methyl-5-oxo-2-imidazolin-2-yl)nicotinic acid.

17. A method for preparing a composition for controlling undesirable plant species characterized by mixing a 5-heterocyclic 2-(2-imidazolin-2-yl)pyridine compound as described in claim 9 and an agronomically acceptable carrier.

18. A method for the preparation of a 5-heterocyclic 2-(2-imidazolin-2-yl)pyridine compound characterized by the structure of formula I 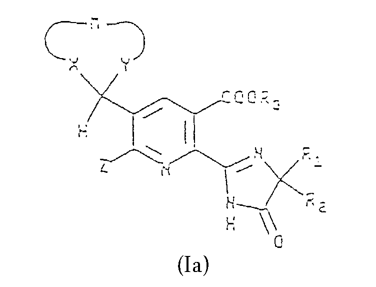 wherein R₃ is hydrogen and R₁, R₂, Z, X, M and Y are as described in claim 1 which comprises reacting a 5-heterocyclic pyridinedicarboxylate compound having the structure 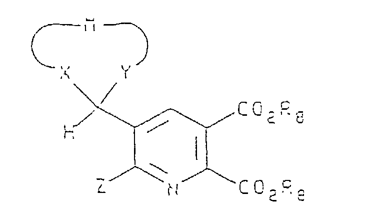 wherein R₈ is C₁-C₄ alkyl with a base to obtain the corresponding 5-heterocyclic 2,3-pyridinedicarboxylic acid; reacting said pyridinedicarboxylic acid with at least one equivalent of an acid anhydride to obtain a 5-heterocyclic-2,3-pyridinedicarboxilic acid anhydride, reacting said pyridinedicarboxylic acid anhydride with at least one equivalent of an aminocarboxamide having the structure 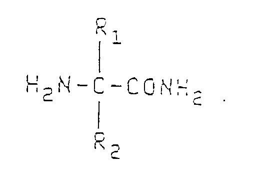 wherein R₁ and R₂ are as described above for formula I in the presence of an inert organic solvent to obtain a nicotinic acid having the structural formula 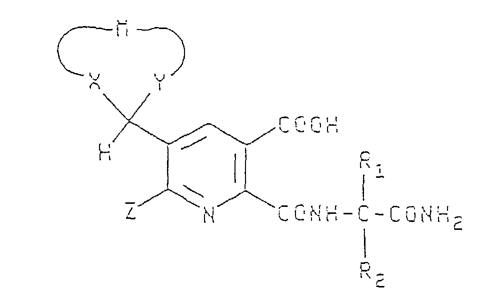 reacting said nicotinic acid with a base, in the presence of a polar solvent to obtain a 5-heterocyclic-2-(2-imidazolin-2-yl)nicotinate and treating said nicotinate with dilute aqueous acid to form a 5-heterocyclic 2-(2-imidazolin-2-yl)pyridine compound of formula I wherein R₃ is hydrogen.

19. A method for the preparation of a 5-heterocyclic 2-(2-imidezolin-2-yl)pyridine compound characterized the formula I structure 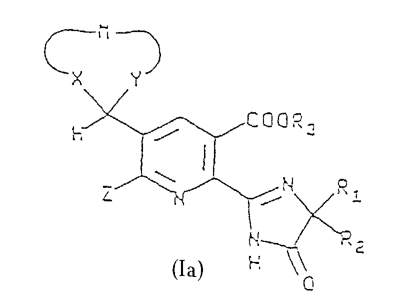 wherein R₃ is hydrogen and R₁, R₂, X, M, Y and Z are as described in claim 9 which comprises reacting a compound having the structure 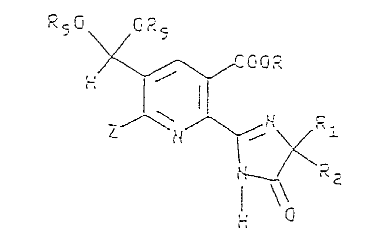 or 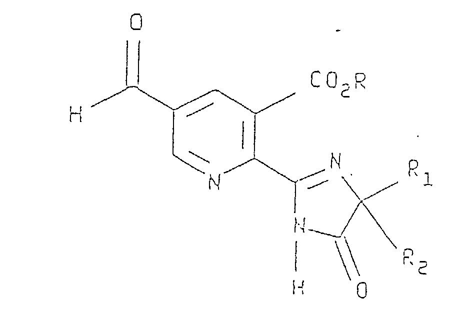 wherein R₉ is C₁-C₄ alkyl with at least two equivalents of hydrochloric acid in water to give the corresponding 5-formyl-2-(5-oxo-2-imidazolin-2-yl)nicotinic acid intermediate, reacting said intermediate with a compound having the structural formula
HX-M-YH
wherein M, X and Y are as described above in the presence of an inert organic solvent at an elevated temperature to form a 5-heterocyclic 2-(2-imidazolin-2-yl)-pyridine compound of formula I wherein R₃ is hydrogen.

20. A composition for controlling undesirable plant species characterized by a 5-heterocyclic 2-(2-imidazolin-2-yl)pyridine compound as described in claim 9 and an agronomically acceptable carrier.

21. The composition according to claim 20 wherein the compound is characterized by the structure 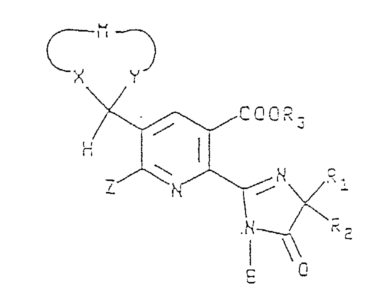 wherein R₁, R₂, R₃, B, X, Y, M and Z are as described in claim 9.

22. The composition according to claim 21 wherein M, X, Y, R₅, R₆ and R₇ are as described therein, Z is hydrogen, C₁-C₄ alkyl, C₁-C₄ alkoxy or halogen, B is hydrogen, R₁ is methyl, R₂ is isopropyl and R₃ is hydrogen, C₁-C₆ alkyl, or a cation of alkali metals, ammonium or organic ammonium.

23. The composition according to claim 22 wherein the compound is 5-(1,3-dioxolan-2-yl)-2-(4-isopropyl-4-methyl-5-oxo-2-imidazolin-2-yl)nicotinic acid.

24. The composition according to claim 22 wherein the compound is 5-(1,3-dioxan-2-yl)-2-(4-isopropyl-4-methyl-5-oxo-2-imidazolin-2-yl)nicotinic acid.

25. The composition according to claim 22 wherein the compound is (1,3-dioxolan-2-yl)-2-(4-isopropyl-4-methyl-5-oxo-2-imidazolin-2-yl)-6-methylnicotinic acid.

26. The composition to claim 22 wherein the compound is (1,3-dioxepan-2-yl)-2-(4-isopropyl-4-methyl-5-oxo-2-imidazolin-2-yl)nicotinic acid.

## Claims (Claims for the following Contracting State(s): ES)

1. A method for the preparation of a compound : characterized by the structure 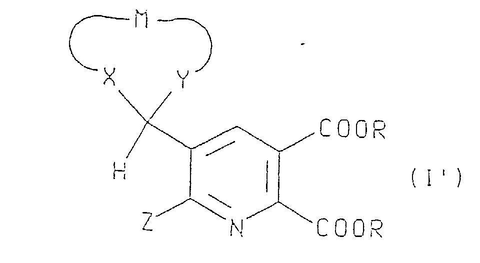 wherein
R is C₁-C₆ alkyl;
X and Y are each independently oxygen, sulfur or NR₄;
R₄ is hydrogen, C₁-C₆ alkyl optionally substituted with C₁-C₄ alkoxy or 1-3 halogens, SO₂R₅, COR₅, CO₂R₅ or CONR₅R₅;
R₅ is hydrogen, C₁-C₆ alkyl optionally substituted with 1-3 halogens, or C₂-C₆ alkenyl;
M is C₂-C₅ alkylene optionally substituted with 1 or 2 C₁-C₄ alkyl groups, C₁-C₄ alkoxy, halogen, CO₂R₆ or oxo, and optionally interrupted by one oxygen or one sulfur,
C₂ alkenylene optionally substituted with 1 or 2 C₁-C₄ alkyl groups or CO₂R₆,
C₃ alkenylene optionally substituted with 1 or 2 C₁-C₄ alkyl groups, CO₂R₆ or oxo,
methyleneamino, optionally substituted with C₁-C₄ alkyl or CO₂R₆, or
a single bond, with the proviso that both X and Y are NR₄,
provided that the ring formed by M, X and Y and the carbon to which they are attached is no more than 8 atoms and provided that when the substituents on M are either alkoxy or halogen the substituted carbon is not bound to X or Y;
R₆ is hydrogen, methyl or ethyl;
Z is hydrogen, halogen, C₁-C₆ alkoxy, or C₁-C₆ alkyl optionally substituted with C₁-C₄, alkoxy or halogen
which comprises reacting a compound having the structure 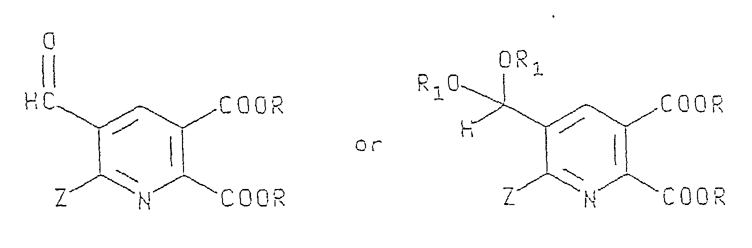 wherein R₁ is C₁-C₄ alkyl and R and Z are as described above with at least one equivalent of an agent having the structure
HX-M-YH
wherein, X, M and Y are as described above in the presence of an acid and a solvent.

2. The method according to claim 1 wherein Z is hydrogen, halogen or methyl; X and Y are oxygen and M is unsubstituted alkylene.

3. The method according to claim 2 wherein the compound is dimethyl 5-(1,3-dioxolan-2-yl) pyridine-2,3-dicarboxylate.

4. The method according to claim 2 wherein the compound is dimethyl 5-(1,3-dioxan-2-yl)pyridine-2,3-dicarboxylate.

5. The method according to claim 2 wherein the compound is dimethyl 5-(1,3-dioxolan-2-yl)-6-methylpyridine-2,3-dicarboxylate.

6. The method according to claim 2, wherein the compound is dimethyl 5-(1,3-dioxepan-2-yl)pyridine-2,3-dicarboxylate.

7. A method for the preparation of a compound characterized by the formula I' structure 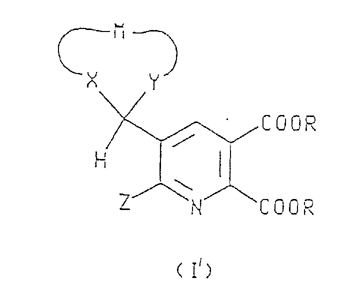 wherein R, Z, X, M, and Y are as described in claim 1 which comprises reacting a compound having the structure 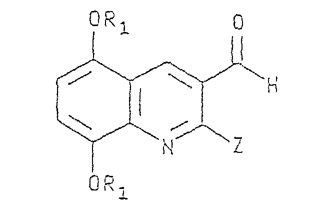 wherein R₁ is C₁-C₄ alkyl, with at least 2 molar equivalents of a tri(C₁-C₄) alkylorthoformate in the presence of a C₁-C₄ alkyl alcohol, ozone and a catalytic amount of an acid to yield a di(C₁-C₄)alkyl acetal pyridinedicarboxylate having the structure 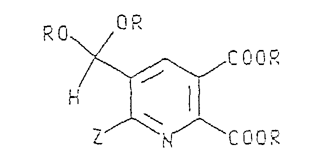 wherein R is C₁-C₄ alkyl and reacting said di(C₁-C₄)alkyl acetal with at least one molar equivalent of an agent having the structure
HX-M-YH
wherein X, M and Y are as described above in the presence of an acid and a solvent to give the formula I 5-heterocyclic-pyridine-2,3-dicarboxylate compound.

8. A method for the preparation of a compound characterized by the formula I' structure 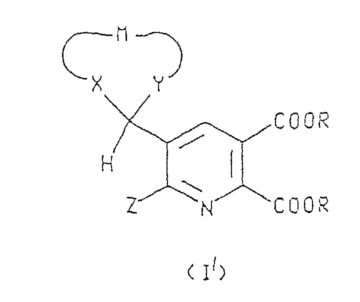 wherein R, Z, X, M and Y are as described in claim 1, which comprises reacting a compound having the structure 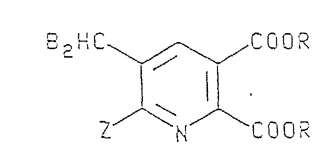 wherein B is halogen, with at least 2 molar equivalents of AgNO₃ in the presence of a C₁-C₄ alkyl alcohol at a temperature greater than about 25°C to yield an intermediate compound having the structure 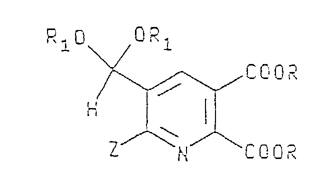 wherein R₁ is C₁-C₄ alkyl and reacting said intermediate compound with an agent having the structure
HX-M-YH
wherein X, M and Y are as described above in the presence of an acid and a solvent to give the formula I 5-heterocyclic pyridine-2,3-dicarboxylate compound.

9. A method for controlling undesirable plant species characterized by applying to the foliage of said plants or to the soil or water containing seeds or other propagating organs thereof, a herbicidally effective amount of a compound characterized by the structure: 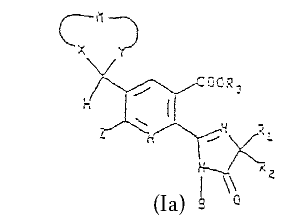 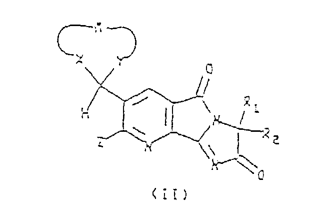 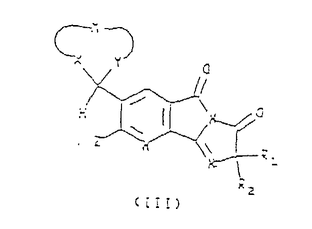 wherein
R₁ is C₁-C₄ alkyl;
R₂ is C₁-C₄ alkyl or C₃-C₆ cycloalkyl and when R₁ and R₂ are taken together with the carbon to which they are attached they may represent C₃-C₆ cycloalkyl;
R₃ is hydrogen,
C₁-C₆ alkyl optionally substituted with one of the following groups: C₁-C₃ alkoxy, halogen or phenyl,
C₃-C₆ alkenyl optionally substituted with one of the following groups: C₁-C₃ alkoxy, phenyl or halogen,
C₃-C₆ alkynyl,
C₃-C₆ cycloalkyl optionally substituted with C₁-C₃ alkyl, or
a cation of alkali metals, ammonium or organic ammonium;
and wherein X, Y, Z, M are as defined in claim 1, and wherein
B is hydrogen or COR₇, provided that when B is COR₇, R₃ is other than hydrogen or a cation;
R₇ is C₁-C₅ alkyl or phenyl optionally substituted with halogen, nitro or methoxy;
the N-oxides thereof, when R₃ is not unsaturated alkyl and when M is not alkenylene;
the optical isomers thereof, when R₁ and R₂ represent different substitutents;
the acid addition salts thereof, when R₃ is other than a cation and the tautomers thereof.

10. The method according to claim 9 wherein the compound is characterized by the structure 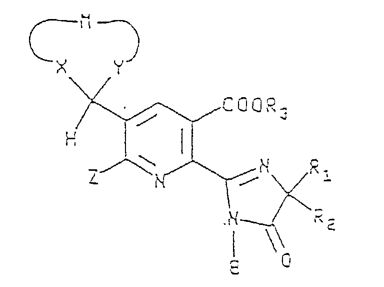 wherein R₁, R₂, R_{3,} B, X, Y, M and Z are as described in claim 9.

11. The method according to claim 10 wherein M, X, Y, R₅, R₆ and R₇ are as described therein, Z is hydrogen, C₁-C₄ alkyl, C₁-C₄ alkoxy or halogen, B is hydrogen, R₁ is methyl, R₂ is isopropyl and R₃ is hydrogen, C₁-C₆ alkyl, or a cation of alkali metals, ammonium or organic ammonium.

12. The method according to claim 11 wherein the compound is 5-(1,3-dioxolan-2-yl)-2-(4-isopropyl-4-methyl-5-oxo-2-imidazolin-2-yl)nicotinic acid.

13. The compound according to claim 11 wherein the compound is 5-(1,3-dioxan-2-yl)-2-(4-isopropyl-4-methyl-5-oxo-2-imidazolin-2-yl)nicotinic acid.

14. The method according to claim 12 wherein the compound is 5-(1,3-dioxolan-2-yl)-2-(4-isopropyl-4-methyl-5-oxo-2-imidazolin-2-yl)-6-methylnicotinic acid.

15. The method according to claim 12 wherein the compound is 5-(1,3-dioxepan-2-yl)-2-(4-isopropyl-4-methyl-5-oxo-2-imidazolin-2-yl)nicotinic acid.

16. The method according to claim 10 wherein the compound is selected from the group consisting of 5-(1,3-dioxolan-2-yl)-2-(4-isopropyl-4-methyl-5-oxo-2-imidazolin-2-yl)nicotinic acid; 5-(1,3-diozan-2-yl)-2-(4-isopropyl-4-methyl-5-oxo-2-imidazolin-2-yl)nicotinic acid; 5-(1,3-dioxolan-2-yl)-2-(4-isopropyl-4-methyl-5-oxo-2-imidazolin-2-yl)-6-methylnicotinic acid; and 5-(1,3-dioxepan-2-yl)-2-(4-isopropyl-4-methyl-5-oxo-2-imidazolin-2-yl)nicotinic acid.

17. A method for preparing a composition for controlling undesirable plant species characterized by mixing a 5-heterocyclic 2-(2-imidazolin-2-yl)pyridine compound as described in claim 9 and an agronomically acceptable carrier.

18. A method for the preparation of a 5-heterocyclic 2-(2-imidazolin-2-yl)pyridine compound characterized by the structure of formula I 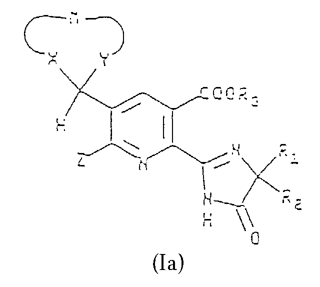 wherein R₃ is hydrogen and R₁, R₂, Z, X, M and Y are as described in claim 1 which comprises reacting a 5-heterocyclic pyridinedicarboxylate compound having the structure 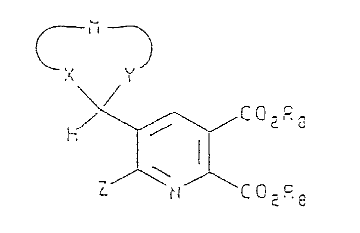 wherein R₈ is C₁-C₄ alkyl with a base to obtain the corresponding 5-heterocyclic 2,3-pyridinedicarboxylic acid; reacting said pyridinedicarboxylic acid with at least one equivalent of an acid anhydride to obtain a 5-heterocyclic-2,3-pyridinedicarboxylic acid anhydride, reacting said pyridinedicarboxylic acid anhydride with at least one equivalent of an aminocarboxamide having the structure 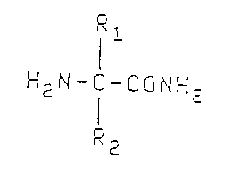 wherein R₁ and R₂ are as described above for formula I in the presence of an inert organic solvent to obtain a nicotinic acid having the structural formula 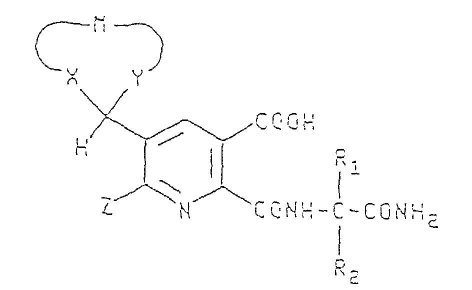 reacting said nicotinic acid with a base, in the presence of a polar solvent to obtain a 5-heterocyclic-2-(2-imidazolin-2-yl)nicotinate and treating said nicotinate with dilute aqueous acid to form a 5-heterocyclic 2-(2-imidazolin-2-yl)pyridine compound of formula I wherein R₃ is hydrogen.

19. A method for the preparation of a 5-heterocyclic 2-(2-imidazolin-2-yl)pyridine compound characterized by the formula I structure 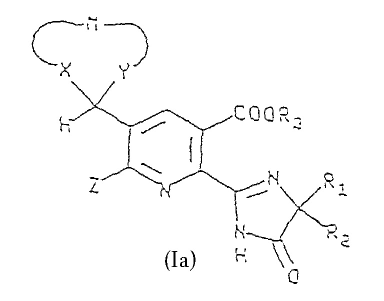 wherein R₃ is hydrogen and R₁, R₂, X, M, Y and Z are as described in claim 9 which comprises reacting a compound having the structure 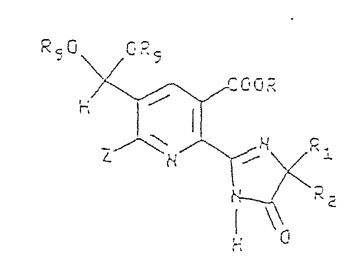 or 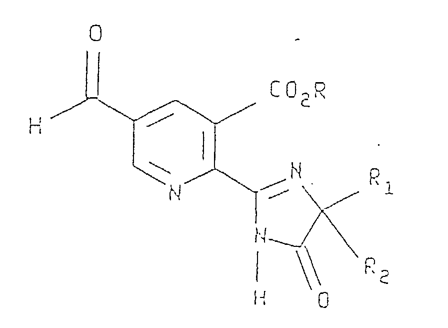 wherein R₉ is C₁-C₄ alkyl with at least two equivalents of hydrochloric acid in water to give the corresponding 5-formyl-2-(5-oxo-2-imidazolin-2-yl)nicotinic acid intermediate, reacting said intermediate with a compound having the structural formula
HX-M-YH
wherein M, X and Y are as described above in the presence of an inert organic solvent at an elevated temperature to form a 5-heterocyclic 2-(2-imidazolin-2-yl)-pyridine compound of formula I wherein R₃ is hydrogen.

## Patentansprüche (Patentansprüche für folgende(n) Vertragsstaat(en): AT, BE, CH, DE, DK, FR, GB, IT, LI, LU, NL, SE)

1. Verbindung, gekennzeichnet durch die Struktur 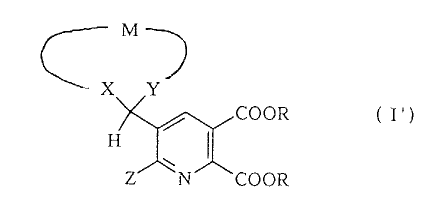 worin
R C₁-C₆-Alkyl ist,
X und Y jeweils unabhängig Sauerstoff, Schwefel oder NR₄ sind,
R₄ Wasserstoff, C₁-C₆-Alkyl ist, das gegebenenfalls mit C₁-C₄-Alkoxy oder 1-3 Halogenen, SO₂R₅, COR₅, CO₂R₅ oder CONR₅R₅ substituiert ist,
R₅ Wasserstoff, C₁-C₆-Alkyl ist, das gegebenenfalls mit 1-3 Halogenen oder C₂-C₆-Alkenyl substituiert ist,
M C₂-C₅-Alkylen, das gegebenenfalls mit 1 oder 2 C₁-C₄-Alkylgruppen, C₁-C₄-Alkoxy, Halogen, CO₂R₆ oder Oxo substituiert und gegebenenfalls durch einen Sauerstoff oder einen Schwefel unterbrochen ist,
C₂-Alkenylen, das gegebenenfalls mit 1 oder 2 C₁-C₄-Alkylgruppen oder CO₂R₆ substituiert ist,
C₃-Alkenylen, das gegebenenfalls mit 1 oder 2 C₁-C₄-Alkylgruppen, CO₂R₆ oder Oxo substituiert ist,
Methylenamino, das gegebenenfalls mit C₁-C₄-Alkyl oder CO₂R₆ substituiert ist oder eine einzelne Bindung ist, unter der Bedingung, daß sowohl X als auch Y NR₄ sind,
unter der Bedingung, daß der durch M, X und Y und das Kohlenstoffatom, an das sie gebunden sind, gebildete Ring nicht mehr als 8 Atome hat, und unter der Bedingung, daß, wenn die Substituenten an M entweder Alkoxy oder Halogen sind, das substituierte Kohlenstoffatom nicht an X oder Y gebunden ist,
R₆ Wasserstoff, Methyl oder Ethyl ist,
Z Wasserstoff, Halogen, C₁-C₆-Alkoxy oder C₁-C₆-Alkyl ist, das gegebenenfalls mit C₁-C₄-Alkoxy oder Halogen substituiert ist.

2. Verbindung nach Anspruch 1, worin Z Wasserstoff, Halogen oder Methyl ist, X und Y Sauerstoff sind und M unsubstituiertes Alkylen ist.

3. Die Verbindung nach Anspruch 2 Dimethyl-5-(1,3-dioxolan-2-yl)pyridin-2,3-dicarboxylat.

4. Die Verbindung nach Anspruch 2 Dimethyl-5-(1,3-dioxan-2-yl)pyridin-2,3-dicarboxylat.

5. Die Verbindung nach Anspruch 2 Dimethyl-5-(1,3-dioxolan-2-yl)-6-methylpyridin-2,3-dicarboxylat.

6. Die Verbindung nach Anspruch 2 Dimethyl-5-(1,3-dioxepan-2-yl)pyridin-2,3-dicarboxylat.

7. Verfahren zum Herstellen einer Verbindung, gekennzeichnet durch die Struktur 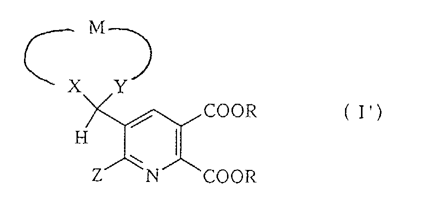 worin R, Z, X, M und Y die in Anspruch 1 angegebene Bedeutung haben, umfassend das Umsetzen einer Verbindung der Struktur 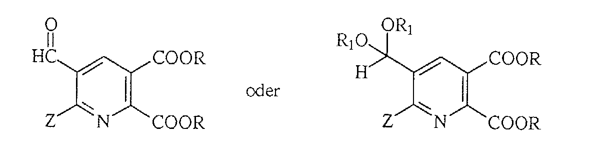 worin R₁ C₁-C₄-Alkyl ist und R und Z die oben genannte Bedeutung haben, mit mindestens einem Äquivalent eines Mittels mit der Struktur
HX-M-YH
worin X, M und Y die oben genannte Bedeutung haben, in Gegenwart einer Säure und eines Lösungsmittels.

8. Verfahren zum Herstellen einer Verbindung, gekennzeichnet durch die Struktur der Formel I' 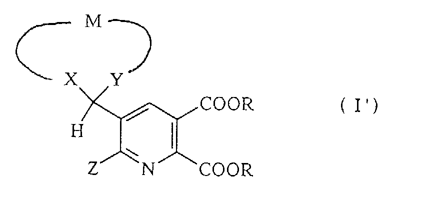 worin R, Z, X, M und Y die in Anspruch 1 genannten Bedeutungen haben, umfassend das Umsetzen einer Verbindung mit der Struktur 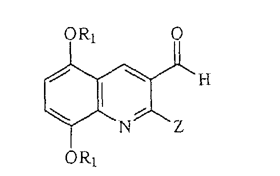 worin R₁ C₁-C₄-Alkyl ist, mit mindestens 2 molaren Äquivalenten eines Tri(C₁-C₄)alkylorthoformiats in Gegenwart von einem C₁-C₄-Alkylalkohol, Ozon und einer katalytischen Menge von einer Säure zu Di(C₁-C₄)alkylacetalpyridindicarboxylat mit der Struktur 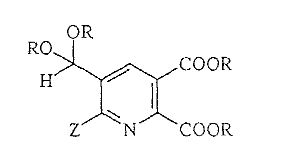 worin R C₁-C₄-Alkyl ist, und Umsetzen dieses Di(C₁-C₄)-alkylacetals mit mindestens einem molaren Äquivalent des Mittels mit der Struktur
HX-M-YH
worin X, M und Y die oben genannte Bedeutung haben, in Gegenwart einer Säure und eines Lösungsmittels, um die in 5-Stellung heterocyclisch substituierte Pyridin-2,3-dicarboxylat-Verbindung der Formel I zu ergeben.

9. Verfahren zum Herstellen einer Verbindung, gekennzeichnet durch die Struktur der Formel I' 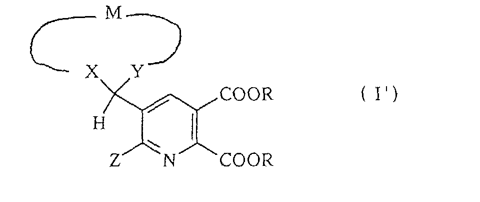 worin R, Z, X, M und Y die in Anspruch 1 angegebene Bedeutung haben, umfassend das Umsetzen einer Verbindung der Struktur 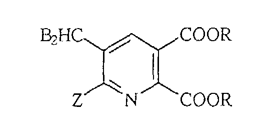 worin B Halogen ist, mit mindestens 2 molaren Äquivalenten von AgNO₃ in Gegenwart eines C₁-C₄-Alkylalkohols bei einer Temperatur von mehr als etwa 25°C zu einer Zwischenverbindung mit der Struktur 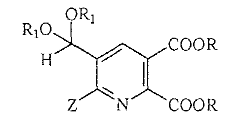 worin R₁ ein C₁-C₄-Alkyl ist, und Umsetzen dieser Zwischenverbindung mit einem Mittel mit der Struktur
HX-M-YH
worin X, M und Y die oben genannte Bedeutung haben, in Gegenwart einer Säure und eines Lösungsmittels zu der Pyridin-2,3-dicarboxylat-Verbindung mit einem heterocyclischen Substituenten in 5-Stellung der Formel I.

10. Verbindung, gekennzeichnet durch die Struktur: 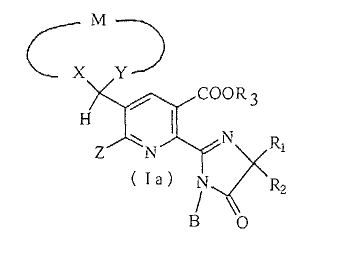 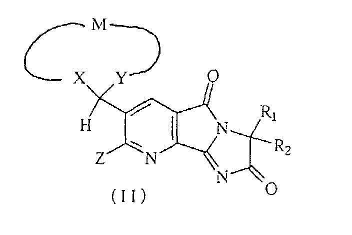 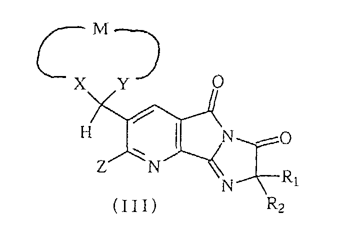 worin
R₁ C₁-C₄-Alkyl ist,
R₂ C₁-C₄-Alkyl oder C₃-C₆-Cycloalkyl ist und, wenn R₁ und R₂ zusammen mit dem Kohlenstoff, an den sie gebunden sind, genommen werden, sie ein C₃-C₆-Cycloalkyl repräsentieren können,
R₃ Wasserstoff,
C₁-C₆-Alkyl, das gegebenenfalls mit einer der folgenden Gruppen substituiert ist: C₁-C₃-Alkoxy, Halogen oder Phenyl,
C₃-C₆-Alkenyl, das gegebenenfalls mit einer der folgenden Gruppen substituiert ist: C₁-C₃-Alkoxy, Phenyl oder Halogen,
C₃-C₆-Alkinyl,
C₃-C₆-Cycloalkyl, das gegebenenfalls mit C₁-C₃-Alkyl substituiert ist oder ein Kation von Alkalimetallen, Ammonium oder organischem Ammonium ist, und
worin X, Y, Z, M die in Anspruch 1 angegebenen Bedeutungen haben und worin
B Wasserstoff oder COR₇ ist, unter der Bedingung, daß, wenn B COR₇ ist, R₃ eine andere Bedeutung hat als Wasserstoff oder ein Kation,
R₇ C₁-C₅-Alkyl oder Phenyl ist, das gegebenenfalls mit Halogen, Nitro oder Methoxy substituiert ist,
deren N-Oxide, wenn R₃ nicht ungesättigtes Alkyl ist, und wenn M nicht Alkenylen ist, deren optische Isomere, wenn R₁ und R₂ unterschiedliche Substituenten repräsentieren, deren Säureadditionssalze, wenn R₃ eine andere Bedeutung als die eines Kations hat, und deren Tautomere.

11. Verbindung nach Anspruch 10, gekennzeichnet durch die Struktur 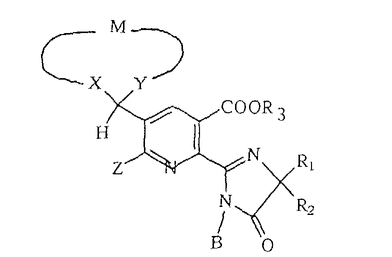 worin R₁, R₂, R₃, B, X, Y, M und Z die in Anspruch 10 angegebenen Bedeutungen haben.

12. Verbindung nach Anspruch 11, worin M, X, Y, R₅, R₆ und R₇ die dort beschriebene Bedeutung haben, Z Wasserstoff, C₁-C₄-Alkyl, C₁-C₄-Alkoxy oder Halogen ist, B Wasserstoff ist, R₁ Methyl ist, R₂ Isopropyl ist und R₃ Wasserstoff, C₁-C₆-Alkyl oder ein Kation von Alkalimetallen, Ammonium oder organischem Ammonium ist.

13. Die Verbindung nach Anspruch 12 5-(1,3-Dioxolan-2-yl)-2-(4-isopropyl-4-methyl-5-oxo-2-imidazolin-2-yl)nicotinsäure.

14. Die Verbindung nach Anspruch 12 5-(1,3-Dioxan-2-yl)-2-(4-isopropyl-4-methyl-5-oxo-2-imidazolin-2-yl)nicotinsäure.

15. Die Verbindung nach Anspruch 12 5-(1,3-Dioxolan-2-yl),2-(4-isopropyl-4-methyl-5-oxo-2-imidazolin-2-yl)-6-methylnicotinsäure.

16. Die Verbindung nach Anspruch 12 5-(1,3-Dioxepan-2-yl)-2-(4-isopropyl-4-methyl-5-oxo-2-imidazolin-2-yl)nicotinsäure.

17. Verfahren zum Bekämpfen unerwünschter Pflanzenarten, gekennzeichnet durch Aufbringen einer herbizid wirksamen Menge einer Verbindung gemäß Anspruch 10 auf das Laub dieser Pflanzen oder auf den Boden oder das Wasser, das Samen oder andere Fortpflanzungsorgane davon enthält.

18. Verfahren nach Anspruch 17, worin die Verbindung die Struktur hat 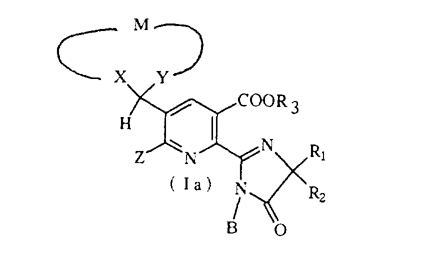 worin R₁, R₂, R₃, B, X, Y, M und Z die in Anspruch 10 angegebenen Bedeutungen haben.

19. Verfahren nach Anspruch 18, worin die Verbindung ausgewählt ist aus der Gruppe bestehend aus 5-(1,3-Dioxolan-2-yl)-2-(4-isopropyl-4-methyl-5-oxo-2-imidazolin-2-yl)nicotinsäure, 5-(1,3-Dioxan-2-yl)-2-(4-isopropyl-4-methyl-5-oxo-2-imidazolin-2-yl)nicotinsäure, 5-(1,3-Dioxolan-2-yl)-2-(4-isopropyl-4-methyl-5-oxo-2-imidazolin-2-yl)-6-methylnicotinsäure und 5-(1,3-Dioxepan-2-yl)-2-(4-isopropyl-4-methyl-5-oxo-2-imidazolin-2-yl)nicotinsäure.

20. Zusammensetzung zum Bekämpfen unerwünschter Pflanzenarten, gekennzeichnet durch eine 5-heterocyclische 2-(2-Imidazolin-2-yl)-pyridin-Verbindung, wie sie in Anspruch 10 beschrieben ist, und einen landwirtschaftlich akzeptablen Träger.

21. Verfahren zum Herstellen einer 2-(2-Imidazolin-2-yl)pyridin-Verbindung, die in 5-Stellung einen heterocyclischen Substituenten hat, gekennzeichnet durch die Struktur der Formel Ia 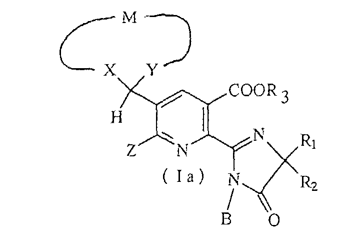 worin R₃ Wasserstoff ist, und R₁, R₂, Z, X, M und Y die in Anspruch 1 angegebenen Bedeutungen haben, umfassend das Umsetzen einer in 5-Stellung einen heterocyclischen Substituenten tragenden Pyridindicarboxylat-Verbindung mit der Struktur 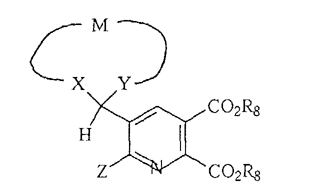 worin R₈ C₁-C₄-Alkyl ist, mit einer Base, um die entsprechende 5-heterocyclische 2,3-Pyridindicarbonsäure zu erhalten, Umsetzen dieser Pyridindicarbonsäure mit mindestens einem Äquivalent eines Säureanhydrids, um ein in 5-Stellung einen heterocyclischen Substituenten tragendes 2,3-Pyridindicarbonsäureanhydrid zu erhalten, Umsetzen dieses Pyridindicarbonsäureanhydrids mit mindestens einem Äquivalent eines Aminocarboxamids der Struktur 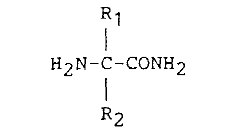 worin R₁ und R₂ die oben für Formel I genannte Bedeutung haben, in Gegenwart eines inerten, organischen Lösungsmittels, um eine Nicotinsäure mit der Strukturformel zu erhalten 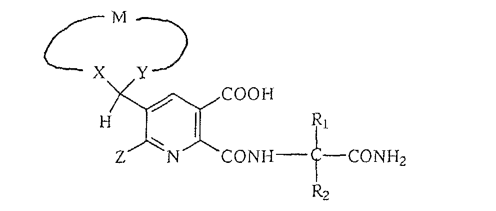 Umsetzen dieser Nikotinsäure mit einer Base in Gegenwart eines polaren Lösungsmittels, um ein 5-heterocyclisches 2-(2-Imidazolin-2-yl)nicotinat zu erhalten, und Behandeln dieses Nicotinats mit verdünnter, wässeriger Säure, um eine 5-heterocyclische 2- (2-Imidazolin-2-yl)pyridin-Verbindung der Formel I zu bilden, worin R₃ Wasserstoff ist.

22. Verfahren zum Herstellen einer 5-heterocyclischen 2-(2-Imidazolin-2-yl)pyridin-Verbindung, gekennzeichnet durch die Struktur der Formel Ia 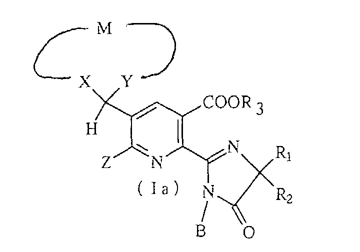 worin R₃ Wasserstoff ist und R₁, R₂, X, M, Y und Z die in Anspruch 10 genannten Bedeutungen haben, umfassend das Umsetzen einer Verbindung mit der Struktur 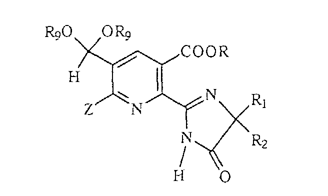 oder 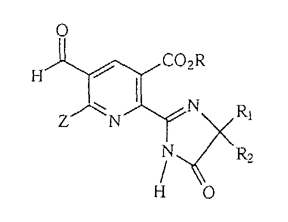 worin R₉ C₁-C₄-Alkyl ist, mit mindestens zwei Äquivalenten Chlorwasserstoffsäure in Wasser, um das entsprechende Zwischenprodukt 2-Formyl-2-(5-oxo-2-imidazolin-2-yl)nicotinsäure zu erhalten, Umsetzen dieses Zwischenproduktes mit einer Verbindung der Strukturformel
HX-M-YH
worin M, X und Y die oben genannte Bedeutung haben, in Gegenwart eines inerten, organischen Lösungsmittels bei einer erhöhten Temperatur, um eine 5-heterocyclische 2-(2-Imidazolin-2-yl)pyridin-Verbindung der Formel I zu bilden, worin R₃ Wasserstoff ist.

## Patentansprüche (Patentansprüche für folgende(n) Vertragsstaat(en): GR)

1. Verfahren zum Herstellen einer Verbindung, gekennzeichnet durch die Struktur 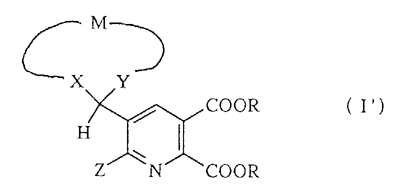 worin
R C₁-C₆-Alkyl ist,
X und Y jeweils unabhängig Sauerstoff, Schwefel oder NR₄ sind,
R₄ Wasserstoff, C₁-C₆-Alkyl ist, das gegebenenfalls mit C₁-C₄-Alkoxy oder 1-3 Halogenen, SO₂R₅, COR₅, CO₂R₅ oder CONR₅R₅ substituiert ist,
R₅ Wasserstoff, C₁-C₆-Alkyl ist, das gegebenenfalls mit 1-3 Halogenen oder C₂-C₆-Alkenyl substituiert ist,
M C₂-C₅-Alkylen, das gegebenenfalls mit 1 oder 2 C₁-C₄-Alkylgruppen, C₁-C₄-Alkoxy, Halogen, CO₂R₆ oder Oxo substituiert und gegebenenfalls durch einen Sauerstoff oder einen Schwefel unterbrochen ist,
C₂-Alkenylen, das gegebenenfalls mit 1 oder 2 C₁-C₄-Alkylgruppen oder CO₂R₆ substituiert ist,
C₃-Alkenylen, das gegebenenfalls mit 1 oder 2 C₁-C₄-Alkylgruppen, CO₂R₆ oder Oxo substituiert ist,
Methylenamino, das gegebenenfalls mit C₁-C₄-Alkyl oder CO₂R₆ substituiert ist oder eine einzelne Bindung ist, unter der Bedingung, daß sowohl X als auch Y NR₄ sind,
unter der Bedingung, daß der durch M, X und Y und das Kohlenstoffatom, an das sie gebunden sind, gebildete Ring nicht mehr als 8 Atome hat, und unter der Bedingung, daß, wenn die Substituenten an M entweder Alkoxy oder Halogen sind, das substituierte Kohlenstoffatom nicht an X oder Y gebunden ist,
R₆ Wasserstoff, Methyl oder Ethyl ist,
Z Wasserstoff, Halogen, C₁-C₆-Alkoxy oder C₁-C₆-Alkyl ist, das gegebenenfalls mit C₁-C₄-Alkoxy oder Halogen substituiert ist,
umfassend das Umsetzen einer Verbindung mit der Struktur 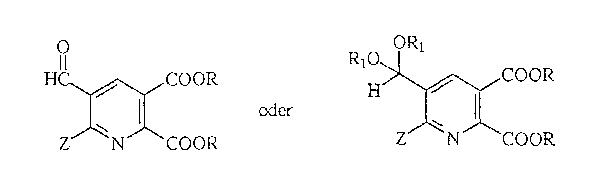 worin R₁ C₁-C₄-Alkyl ist und R und Z die oben genannte Bedeutung haben, mit mindestens einem Äquivalent eines Mittels mit der Struktur
HX-M-YH
worin X, M und Y die oben genannte Bedeutung haben, in Gegenwart einer Säure und eines Lösungsmittels.

2. Verfahren nach Anspruch 1, worin Z Wasserstoff, Halogen oder Methyl ist, X und Y Sauerstoff sind und M unsubstituiertes Alkylen ist.

3. Verfahren nach Anspruch 2, worin die Verbindung Dimethyl-5-(1,3-dioxolan-2-yl)pyridin-2,3-dicarboxylat ist.

4. Verfahren nach Anspruch 2, worin die Verbindung Dimethyl-5-(1,3-dioxan-2-yl)pyridin-2,3-dicarboxylat ist.

5. Verfahren nach Anspruch 2, worin die Verbindung Dimethyl-5-(1,3-dioxolan-2-yl)-6-methylpyridin-2,3-dicarboxylat ist.

6. Verfahren nach Anspruch 2, worin die Verbindung Dimethyl-5-(1,3-dioxepan-2-yl)pyridin-2,3-dicarboxylat ist.

7. Verfahren zum Herstellen einer Verbindung, gekennzeichnet durch die Struktur der Formel I' 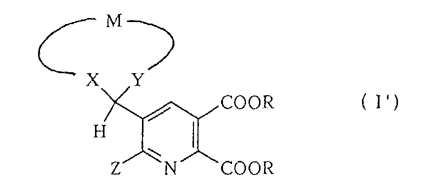 worin R, Z, X, M und Y die in Anspruch 1 genannten Bedeutungen haben, umfassend das Umsetzen einer Verbindung mit der Struktur 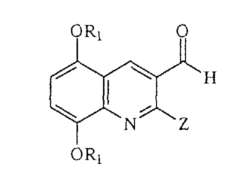 worin R₁ C₁-C₄-Alkyl ist, mit mindestens 2 molaren Äquivalenten eines Tri(C₁-C₄)alkylorthoformiats in Gegenwart von einem C₁-C₄-Alkylalkohol, Ozon und einer katalytischen Menge von einer Säure zu Di(C₁-C₄)alkylacetalpyridindicarboxylat mit der Struktur 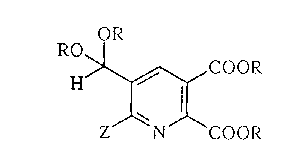 worin R C₁-C₄-Alkyl ist, und Umsetzen dieses Di(C₁-C₄)-alkylacetals mit mindestens einem molaren Äquivalent des Mittels mit der Struktur
HX-M-YH
worin X, M und Y die oben genannte Bedeutung haben, in Gegenwart einer Säure und eines Lösungsmittels, um die in 5-Stellung heterocyclisch substituierte Pyridin-2,3-dicarboxylat-Verbindung der Formel I zu ergeben.

8. Verfahren zum Herstellen einer Verbindung, gekennzeichnet durch die Struktur der Formel I' 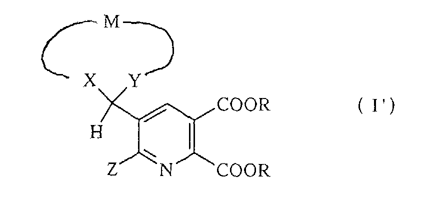 worin R, Z, X, M und Y die in Anspruch 1 angegebene Bedeutung haben, umfassend das Umsetzen einer Verbindung der Struktur 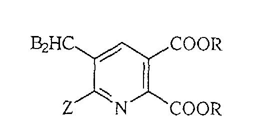 worin B Halogen ist, mit mindestens 2 molaren Äquivalenten von AgNO₃ in Gegenwart eines C₁-C₄-Alkylalkohols bei einer Temperatur von mehr als etwa 25°C zu einer Zwischenverbindung mit der Struktur 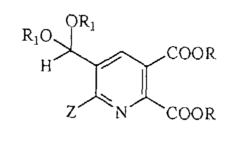 worin R₁ ein C₁-C₄-Alkyl ist, und Umsetzen dieser Zwischenverbindung mit einem Mittel mit der Struktur
HX-M-YH
worin X, M und Y die oben genannte Bedeutung haben, in Gegenwart einer Säure und eines Lösungsmittels zu der Pyridin-2,3-dicarboxylat-Verbindung mit einem heterocyclischen Substituenten in 5-Stellung der Formel I.

9. Verfahren zum Bekämpfen unerwünschter Pflanzenarten, gekennzeichnet durch das Aufbringen auf das Laub der Pflanzen oder den Boden oder das Wasser, das Samen oder andere Fortpflanzungsorgane davon enthält, einer herbizid wirksamen Menge einer Verbindung, gekennzeichnet durch die Struktur: 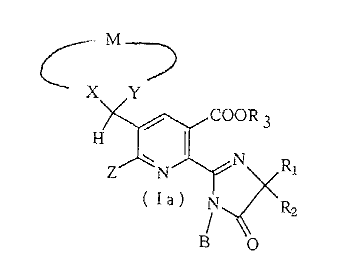 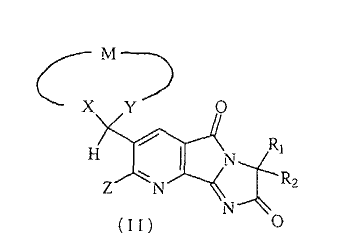 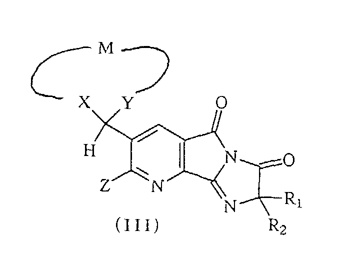 worin
R₁ C₁-C₄-Alkyl ist,
R₂ C₁-C₄-Alkyl oder C₃-C₆-Cycloalkyl ist und, wenn R₁ und R₂ zusammen mit dem Kohlenstoff, an den sie gebunden sind, genommen werden, sie ein C₃-C₆-Cycloalkyl repräsentieren können,
R₃ Wasserstoff,
C₁-C₆-Alkyl, das gegebenenfalls mit einer der folgenden Gruppen substituiert ist: C₁-C₃-Alkoxy, Halogen oder Phenyl,
C₃-C₆-Alkenyl, das gegebenenfalls mit einer der folgenden Gruppen substituiert ist: C₁-C₃-Alkoxy, Phenyl oder Halogen,
C₃-C₆-Alkinyl,
C₃-C₆-Cycloalkyl, das gegebenenfalls mit C₁-C₃-Alkyl substituiert ist oder ein Kation von Alkalimetallen, Ammonium oder organischem Ammonium ist, und
worin X, Y, Z, M die in Anspruch 1 angegebenen Bedeutungen haben und worin
B Wasserstoff oder COR₇ ist, unter der Bedingung, daß, wenn B COR₇ ist, R₃ eine andere Bedeutung hat als Wasserstoff oder ein Kation,
R₇ C₁-C₅-Alkyl oder Phenyl ist, das gegebenenfalls mit Halogen, Nitro oder Methoxy substituiert ist,
deren N-Oxide, wenn R₃ nicht ungesättigtes Alkyl ist, und wenn M nicht Alkenylen ist, deren optische Isomere, wenn R₁ und R₂ unterschiedliche Substituenten repräsentieren, deren Säureadditionssalze, wenn R₃ eine andere Bedeutung als die eines Kations hat, und deren Tautomere.

10. Verfahren nach Anspruch 9, worin die Verbindung durch die Struktur gekennzeichnet ist 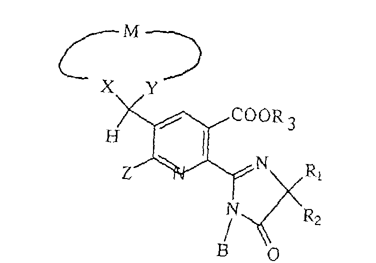 worin R₁, R₂, R₃, B, X, Y, M und Z die in Anspruch 9 angegebenen Bedeutungen haben.

11. Verfahren nach Anspruch 10, worin M, X, Y, R₅, R₆ und R₇ die dort beschriebene Bedeutung haben, Z Wasserstoff, C₁-C₄-Alkyl, C₁-C₄-Alkoxy oder Halogen ist, B Wasserstoffist, R₁ Methyl ist, R₂ Isopropyl ist und R₃ Wasserstoff, C₁-C₆-Alkyl oder ein Kation von Alkalimetallen, Ammonium oder organischem Ammonium ist.

12. Verfahren nach Anspruch 11, worin die Verbindung 5-(1,3-Dioxolan-2-yl)-2-(4-isopropyl-4-methyl-5-oxo-2-imidazolin-2-yl)nicotinsäure ist.

13. Verfahren nach Anspruch 11, worin die Verbindung 5-(1,3-Dioxan-2-yl)-2-(4-isopropyl-4-methyl-5-oxo-2-imidazolin-2-yl)nicotinsäure ist.

14. Verfahren nach Anspruch 12, worin die Verbindung 5-(1,3-Dioxolan-2-yl)-2-(4-isopropyl-4-methyl-5-oxo-2-imidazolin-2-yl)-6-methylnicotinsäure ist.

15. Verfahren nach Anspruch 12, worin die Verbindung 5-(1,3-Dioxepan-2-yl)-2-(4-isopropyl-4-methyl-5-oxo-2-imidazolin-2-yl)nicotinsäure ist.

16. Verfahren nach Anspruch 10, worin die Verbindung ausgewählt ist aus der Gruppe bestehend aus 5-(1,3-Dioxolan-2-yl)-2-(4-isopropyl-4-methyl-5-oxo-2-imidazolin-2-yl)nicotinsäure, 5-(1,3-Dioxan-2-yl)-2-(4-isopropyl-4-methyl-5-oxo-2-imidazolin-2-yl)nicotinsäure, 5-(1,3-Dioxolan-2-yl)-2-(4-isopropyl-4-methyl-5-oxo-2-imidazolin-2-yl)-6-methylnicotinsäure und 5-(1,3-Dioxepan-2-yl)-2-(4-isopropyl-4-methyl-5-oxo-2-imidazolin-2-yl)nicotinsäure.

17. Verfahren zum Herstellen einer Zusammensetzung zum Bekämpfen unerwünschter Pflanzenarten, gekennzeichnet durch eine 5-heterocyclische 2-(2-Imidazolin-2-yl)-pyridin-Verbindung, wie sie in Anspruch 9 beschrieben ist, und einen landwirtschaftlich akzeptablen Träger.

18. Verfahren zum Herstellen einer 2-(2-Imidazolin-2-yl)pyridin-Verbindung, die in 5-Stellung einen heterocyclischen Substituenten hat, gekennzeichnet durch die Struktur der Formel Ia 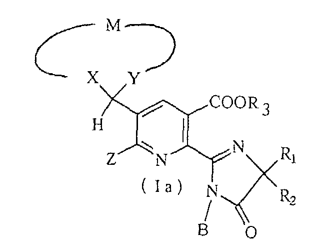 worin R₃ Wasserstoff ist, und R₁, R₂, Z, X, M und Y die in Anspruch 1 angegebenen Bedeutungen haben, umfassend das Umsetzen einer in 5-Stellung einen heterocyclischen Substituenten tragenden Pyridindicarboxylat-Verbindung mit der Struktur 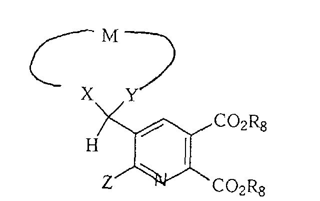 worin R₈ C₁-C₄-Alkyl ist, mit einer Base, um die entsprechende 5-heterocyclische 2,3-Pyridindicarbonsäure zu erhalten, Umsetzen dieser Pyridindicarbonsäure mit mindestens einem Äquivalent eines Säureanhydrids, um ein in 5-Stellung einen heterocyclischen Substituenten tragendes 2,3-Pyridindicarbonsäureanhydrid zu erhalten, Umsetzen dieses Pyridindicarbonsäureanhydrids mit mindestens einem Äquivalent eines Aminocarboxamids der Struktur 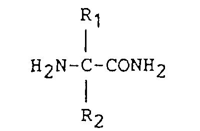 worin R₁ und R₂ die oben für Formel I genannte Bedeutung haben, in Gegenwart eines inerten, organischen Lösungsmittels, um eine Nicotinsäure mit der Strukturformel zu erhalten 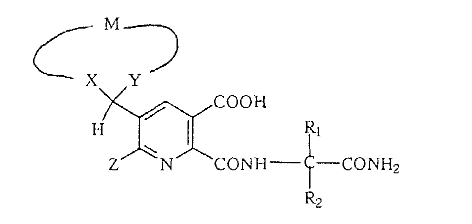 Umsetzen dieser Nikotinsäure mit einer Base in Gegenwart eines polaren Lösungsmittels, um ein 5-heterocyclisches 2-(2-Imidazolin-2-yl)nicotinat zu erhalten, und Behandeln dieses Nicotinats mit verdünnter, wässeriger Säure, um eine 5-heterocyclische 2-(2-Imidazolin-2-yl)pyridin-Verbindung der Formel I zu bilden, worin R₃ Wasserstoff ist.

19. Verfahren zum Herstellen einer 5-heterocyclischen 2-(2-Imidazolin-2-yl)pyridin-Verbindung, gekennzeichnet durch die Struktur der Formel Ia 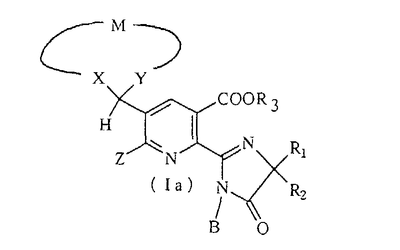 worin R₃ Wasserstoff ist und R₁, R₂, X, M, Y und Z die in Anspruch 9 genannten Bedeutungen haben, umfassend das Umsetzen einer Verbindung mit der Struktur 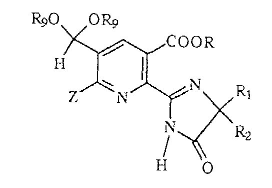 oder 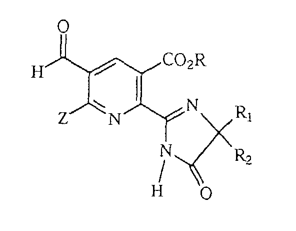 worin R₉ C₁-C₄-Alkyl ist, mit mindestens zwei Äquivalenten Chlorwasserstoffsäure in Wasser, um das entsprechende Zwischenprodukt 2-Formyl-2-(5-oxo-2-imidazolin-2-yl)nicotinsäure zu erhalten, Umsetzen dieses Zwischenproduktes mit einer Verbindung der Strukturformel
HX-M-YH
worin M, X und Y die oben genannte Bedeutung haben, in Gegenwart eines inerten, organischen Lösungsmittels bei einer erhöhten Temperatur, um eine 5-heterocyclische 2-(2-Imidazolin-2-yl)-pyridin-Verbindung der Formel I zu bilden, worin R₃ Wasserstoff ist.

20. Zusammensetzung zum Bekämpfen unerwünschter Pflanzenarten, gekennzeichnet durch eine 5-heterocyclische 2-(2-Imidazolin-2-yl)-pyridin-Verbindung, wie sie in Anspruch 9 beschrieben ist, und einen landwirtschaftlich akzeptablen Träger.

21. Zusammensetzung nach Anspruch 20, worin die Verbindung gekennzeichnet ist durch die Struktur 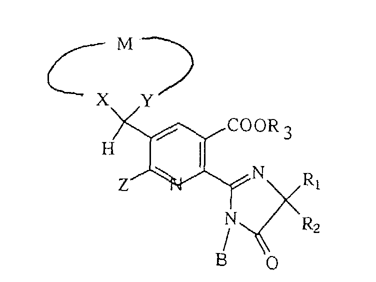 worin R₁, R₂, R₃, B, X, Y, M und Z die in Anspruch 9 angegebenen Bedeutungen haben.

22. Zusammensetzung nach Anspruch 21, worin M, X, Y, R₅, R₆ und R₇ die dort beschriebene Bedeutung haben, Z Wasserstoff, C₁-C₄-Alkyl, C₁-C₄-Alkoxy oder Halogen ist, B Wasserstoff ist, R₁ Methyl ist, R₂ Isopropyl ist und R₃ Wasserstoff, C₁-C₆-Alkyl oder ein Kation von Alkalimetallen, Ammonium oder organischem Ammonium ist.

23. Zusammensetzung nach Anspruch 22, worin die Verbindung 5-(1,3-Dioxolan-2-yl)-2-(4-isopropyl-4-methyl-5-oxo-2-imidazolin-2-yl)nicotinsäure ist.

24. Zusammensetzung nach Anspruch 22, worin die Verbindung 5-(1,3-Dioxan-2-yl)-2-(4-isopropyl-4-methyl-5-oxo-2-imidazolin-2-yl)nicotinsäure ist.

25. Zusammensetzung nach Anspruch 22, worin die Verbindung 5-(1,3-Dioxolan-2-yl)-2-(4-isopropyl-4-methyl-5-oxo-2-imidazolin-2-yl)-6-methylnicotinsäure ist.

26. Zusammensetzung nach Anspruch 22, worin die Verbindung 5-(1,3-Dioxepan-2-yl)-2-(4-isopropyl-4-methyl-5-oxo-2-imidazolin-2-yl)nicotinsäure ist.

## Patentansprüche (Patentansprüche für folgende(n) Vertragsstaat(en): ES)

1. Verfahren zum Herstellen einer Verbindung, gekennzeichnet durch die Struktur 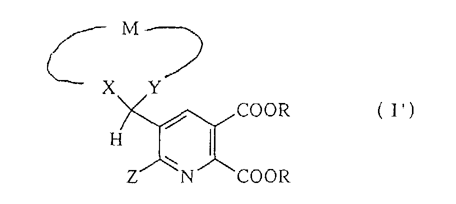 worin
R C₁-C₆-Alkyl ist,
X und Y jeweils unabhängig Sauerstoff, Schwefel oder NR₄ sind,
R₄ Wasserstoff, C₁-C₆-Alkyl ist, das gegebenenfalls mit C₁-C₄-Alkoxy oder 1-3 Halogenen, SO₂R₅, COR₅, CO₂R₅ oder CONR₅R₅ substituiert ist,
R₅ Wasserstoff, C₁-C₆-Alkyl ist, das gegebenenfalls mit 1-3 Halogenen oder C₂-C₆-Alkenyl substituiert ist,
M C₂-C₅-Alkylen, das gegebenenfalls mit 1 oder 2 C₁-C₄-Alkylgruppen, C₁-C₄-Alkoxy, Halogen, CO₂R₆ oder Oxo substituiert und gegebenenfalls durch einen Sauerstoff oder einen Schwefel unterbrochen ist,
C₂-Alkenylen, das gegebenenfalls mit 1 oder 2 C₁-C₄-Alkylgruppen oder CO₂R₆ substituiert ist,
C₃-Alkenylen, das gegebenenfalls mit 1 oder 2 C₁-C₄-Alkylgruppen, CO₂R₆ oder Oxo substituiert ist,
Methylenamino, das gegebenenfalls mit C₁-C₄-Alkyl oder CO₂R₆ substituiert ist oder eine einzelne Bindung ist, unter der Bedingung, daß sowohl X als auch Y NR₄ sind,
unter der Bedingung, daß der durch M, X und Y und das Kohlenstoffatom, an das sie gebunden sind, gebildete Ring nicht mehr als 8 Atome hat, und unter der Bedingung, daß, wenn die Substituenten an M entweder Alkoxy oder Halogen sind, das substituierte Kohlenstoffatom nicht an X oder Y gebunden ist,
R₆ Wasserstoff, Methyl oder Ethyl ist,
Z Wasserstoff, Halogen, C₁-C₆-Alkoxy oder C₁-C₆-Alkyl ist, das gegebenenfalls mit C₁-C₄-Alkoxy oder Halogen substituiert ist,
umfassend das Umsetzen einer Verbindung mit der Struktur 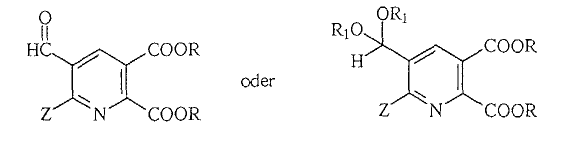 worin R₁ C₁-C₄-Alkyl ist und R und Z die oben genannte Bedeutung haben, mit mindestens einem Äquivalent eines Mittels mit der Struktur
HX-M-YH
worin X, M und Y die oben genannte Bedeutung haben, in Gegenwart einer Säure und eines Lösungsmittels.

2. Verfahren nach Anspruch 1, worin Z Wasserstoff, Halogen oder Methyl ist, X und Y Sauerstoff sind und M unsubstituiertes Alkylen ist.

3. Verfahren nach Anspruch 2, worin die Verbindung Dimethyl-5-(1,3-dioxolan-2-yl)pyridin-2,3-dicarboxylat ist.

4. Verfahren nach Anspruch 2, worin die Verbindung Dimethyl-5-(1,3-dioxan-2-yl)pyridin-2,3-dicarboxylat ist.

5. Verfahren nach Anspruch 2, worin die Verbindung Dimethyl-5-(1,3-dioxolan-2-yl)-6-methylpyridin-2,3-dicarboxylat ist.

6. Verfahren nach Anspruch 2, worin die Verbindung Dimethyl-5-(1,3-dioxepan-2-yl)pyridin-2,3-dicarboxylat ist.

7. Verfahren zum Herstellen einer Verbindung, gekennzeichnet durch die Struktur der Formel I' 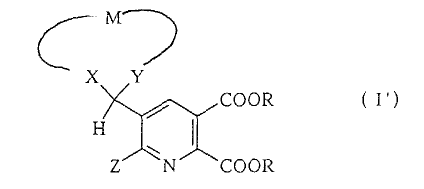 worin R, Z, X, M und Y die in Anspruch 1 genannten Bedeutungen haben, umfassend das Umsetzen einer Verbindung mit der Struktur 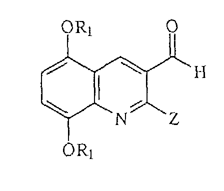 worin R₁ C₁-C₄-Alkyl ist, mit mindestens 2 molaren Äquivalenten eines Tri(C₁-C₄)alkylorthoformiats in Gegenwart von einem C₁-C₄-Alkylalkohol, Ozon und einer katalytischen Menge von einer Säure zu Di(C₁-C₄)alkylacetalpyridindicarboxylat mit der Struktur 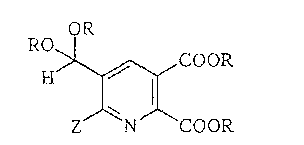 worin R C₁-C₄-Alkyl ist, und Umsetzen dieses Di(C₁-C₄)-alkylacetals mit mindestens einem molaren Äquivalent des Mittels mit der Struktur
HX-M-YH
worin X, M und Y die oben genannte Bedeutung haben, in Gegenwart einer Säure und eines Lösungsmittels, um die in 5-Stellung heterocyclisch substituierte Pyridin-2,3-dicarboxylat-Verbindung der Formel I zu ergeben.

8. Verfahren zum Herstellen einer Verbindung, gekennzeichnet durch die Struktur der Formel I' 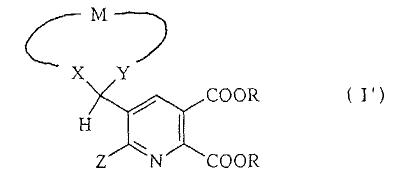 worin R, Z, X, M und Y die in Anspruch 1 angegebene Bedeutung haben, umfassend das Umsetzen einer Verbindung der Struktur 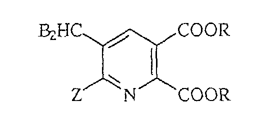 worin B Halogen ist, mit mindestens 2 molaren Äquivalenten von AgNO₃ in Gegenwart eines C₁-C₄-Alkylalkohols bei einer Temperatur von mehr als etwa 25°C zu einer Zwischenverbindung mit der Struktur 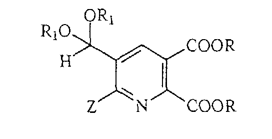 worin R₁ ein C₁-C₄-Alkyl ist, und Umsetzen dieser Zwischenverbindung mit einem Mittel mit der Struktur
HX-M-YH
worin X, M und Y die oben genannte Bedeutung haben, in Gegenwart einer Säure und eines Lösungsmittels zu der Pyridin-2,3-dicarboxylat-Verbindung mit einem heterocyclischen Substituenten in 5-Stellung der Formel I.

9. Verfahren zum Bekämpfen unerwünschter Pflanzenarten, gekennzeichnet durch das Aufbringen auf das Laub der Pflanzen oder den Boden oder das Wasser, der oder das Samen oder andere Fortpflanzungsorgane davon enthält, einer herbizid wirksamen Menge einer Verbindung, gekennzeichnet durch die Struktur: 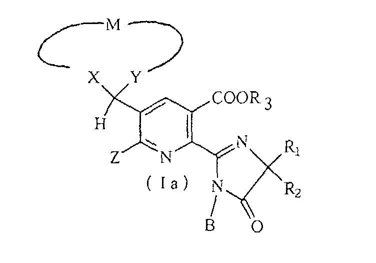 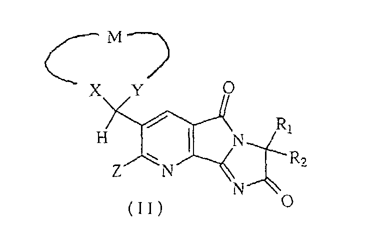 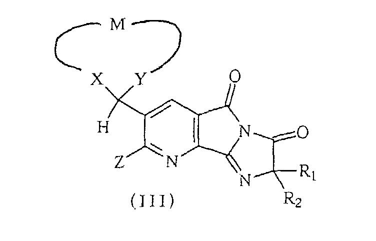 worin
R₁ C₁-C₄-Alkyl ist,
R₂ C₁-C₄-Alkyl oder C₃-C₆-Cycloalkyl ist und, wenn R₁ und R₂ zusammen mit dem Kohlenstoff, an den sie gebunden sind, genommen werden, sie ein C₃-C₆-Cycloalkyl repräsentieren können,
R₃ Wasserstoff,
C₁-C₆-Alkyl, das gegebenenfalls mit einer der folgenden Gruppen substituiert ist: C₁-C₃-Alkoxy, Halogen oder Phenyl,
C₃-C₆-Alkenyl, das gegebenenfalls mit einer der folgenden Gruppen substituiert ist: C₁-C₃-Alkoxy, Phenyl oder Halogen,
C₃-C₆-Alkinyl,
C₃-C₆-Cycloalkyl, das gegebenenfalls mit C₁-C₃-Alkyl substituiert ist oder
ein Kation von Alkalimetallen, Ammonium oder organischem Ammonium ist, und
worin X, Y, Z, M die in Anspruch 1 angegebenen Bedeutungen haben und worin
B Wasserstoff oder COR₇ ist, unter der Bedingung, daß, wenn B COR₇ ist, R₃ eine andere Bedeutung hat als Wasserstoff oder ein Kation,
R₇ C₁-C₅-Alkyl oder Phenyl ist, das gegebenenfalls mit Halogen, Nitro oder Methoxy substituiert ist,
deren N-Oxide, wenn R₃ nicht ungesättigtes Alkyl ist, und wenn M nicht Alkenylen ist, deren optische Isomere, wenn R₁ und R₂ unterschiedliche Substituenten repräsentieren, deren Säureadditionssalze, wenn R₃ eine andere Bedeutung als die eines Kations hat, und deren Tautomere.

10. Verfahren nach Anspruch 9, worin die Verbindung durch die Struktur gekennzeichnet ist 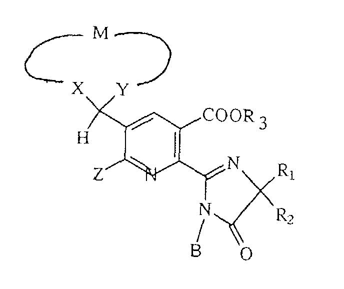 worin R₁, R₂, R₃, B, X, Y, M und Z die in Anspruch 9 angegebenen Bedeutungen haben.

11. Verfahren nach Anspruch 10, worin M, X, Y, R₅, R₆ und R₇ die dort beschriebene Bedeutung haben, Z Wasserstoff, C₁-C₄-Alkyl, C₁-C₄-Alkoxy oder Halogen ist, B Wasserstoff ist, R₁ Methyl ist, R₂ Isopropyl ist und R₃ Wasserstoff, C₁-C₆-Alkyl oder ein Kation von Alkalimetallen, Ammonium oder organischem Ammonium ist.

12. Verfahren nach Anspruch 11, worin die Verbindung 5-(1,3-Dioxolan-2-yl)-2-(4-isopropyl-4-methyl-5-oxo-2-imidazolin-2-yl)nicotinsäure ist.

13. Verfahren nach Anspruch 11, worin die Verbindung 5-(1,3-Dioxan-2-yl)-2-(4-isopropyl-4-methyl-5-oxo-2-imidazolin-2-yl)nicotinsäure ist.

14. Verfahren nach Anspruch 12, worin die Verbindung 5-(1,3-Dioxolan-2-yl)-2-(4-isopropyl-4-methyl-5-oxo-2-imidazolin-2-yl)-6-methylnicotinsäure ist.

15. Verfahren nach Anspruch 12, worin die Verbindung 5-(1,3-Dioxepan-2-yl)-2-(4-isopropyl-4-methyl-5-oxo-2-imidazolin-2-yl)nicotinsäure ist.

16. Verfahren nach Anspruch 10, worin die Verbindung ausgewählt ist aus der Gruppe bestehend aus 5-(1,3-Dioxolan-2-yl)-2-(4-isopropyl-4-methyl-5-oxo-2-imidazolin-2-yl)nicotinsäure, 5-(1,3-Dioxan-2-yl)-2-(4-isopropyl-4-methyl-5-oxo-2-imidazolin-2-yl)nicotinsäure, 5-(1,3-Dioxolan-2-yl)-2-(4-isopropyl-4-methyl-5-oxo-2-imidazolin-2-yl)-6-methylnicotinsäure und 5-(1,3-Dioxepan-2-yl)-2-(4-isopropyl-4-methyl-5-oxo-2-imidazolin-2-yl)nicotinsäure.

17. Verfahren zum Herstellen einer Zusammensetzung zum Bekämpfen unerwünschter Pflanzenarten, gekennzeichnet durch eine 5-heterocyclische 2-(2-Imidazolin-2-yl)-pyridin-Verbindung, wie sie in Anspruch 9 beschrieben ist, und einen landwirtschaftlich akzeptablen Träger.

18. Verfahren zum Herstellen einer 2-(2-Imidazolin-2-yl)pyridin-Verbindung, die in 5-Stellung einen heterocyclischen Substituenten hat, gekennzeichnet durch die Struktur der Formel Ia 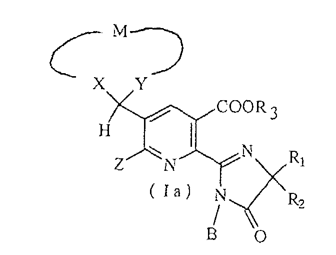 worin R₃ Wasserstoff ist, und R₁, R₂, Z, X, M und Y die in Anspruch 1 angegebenen Bedeutungen haben, umfassend das Umsetzen einer in 5-Stellung einen heterocyclischen Substituenten tragenden Pyridindicarboxylat-Verbindung mit der Struktur 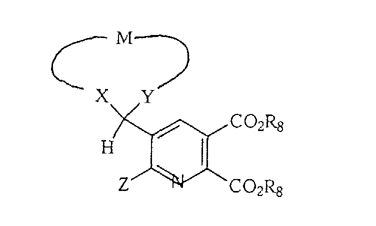 worin R₈ C₁-C₄-Alkyl ist, mit einer Base, um die entsprechende 5-heterocyclische 2,3-Pyridindicarbonsäure zu erhalten, Umsetzen dieser Pyridindicarbonsäure mit mindestens einem Äquivalent eines Säureanhydrids, um ein in 5-Stellung einen heterocyclischen Substituenten tragendes 2,3-Pyridindicarbonsäureanhydrid zu erhalten, Umsetzen dieses Pyridindicarbonsäureanhydrids mit mindestens einem Äquivalent eines Aminocarboxamids der Struktur 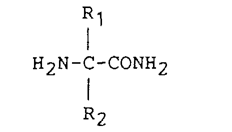 worin R₁ und R₂ die oben für Formel I genannte Bedeutung haben, in Gegenwart eines inerten, organischen Lösungsmittels, um eine Nicotinsäure mit der Strukturformel zu erhalten 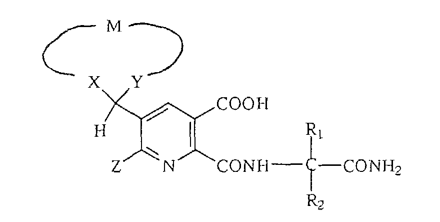 Umsetzen dieser Nikotinsäure mit einer Base in Gegenwart eines polaren Lösungsmittels, um ein 5-heterocyclisches 2-(2-Imidazolin-2-yl)nicotinat zu erhalten, und Behandeln dieses Nicotinats mit verdünnter, wässeriger Säure, um eine 5-heterocyclische 2-(2-Imidazolin-2-yl)pyridin-Verbindung der Formel 1 zu bilden, worin R₃ Wasserstoff ist.

19. Verfahren zum Herstellen einer 5-heterocyclischen 2- (2-Imidazolin-2-yl)pyridin-Verbindung, gekennzeichnet durch die Struktur der Formel Ia 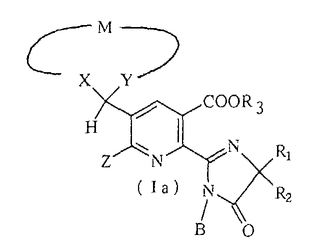 worin R₃ Wasserstoff ist und R₁, R₂, X, M, Y und Z die in Anspruch 9 genannten Bedeutungen haben, umfassend das Umsetzen einer Verbindung mit der Struktur 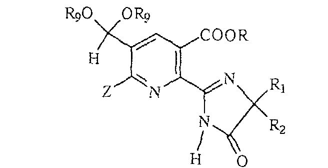 oder 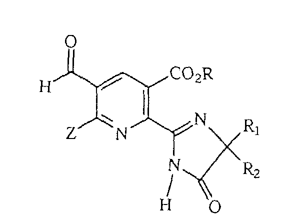 worin R₉ C₁-C₄-Alkyl ist, mit mindestens zwei Äquivalenten Chlorwasserstoffsäure in Wasser, um das entsprechende Zwischenprodukt 2-Formyl-2-(5-oxo-2-imidazolin-2-yl)nicotinsäure zu erhalten, Umsetzen dieses Zwischenproduktes mit einer Verbindung der Strukturformel
HX-M-YH
worin M, X und Y die oben genannte Bedeutung haben, in Gegenwart eines inerten, organischen Lösungsmittels bei einer erhöhten Temperatur, um eine 5-heterocyclische 2-(2-Imidazolin-2-yl)pyridin-Verbindung der Formel I zu bilden, worin R₃ Wasserstoff ist.

## Revendications (Revendications pour l'(les) Etat(s) contractant(s) suivant(s): AT, BE, CH, DE, DK, FR, GB, IT, LI, LU, NL, SE)

1. Composé caractérisé par la structure 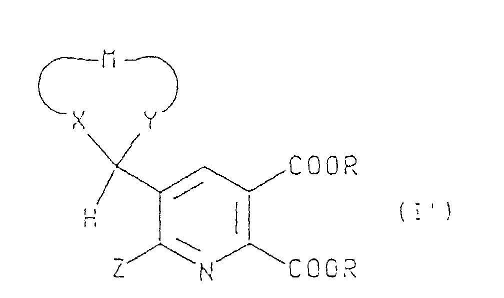 dans laquelle
R est un alkyle en C₁ - C₆;
X et Y sont chacun indépendamment un oxygène, un soufre ou NR₄;
R₄ est un hydrogène, un alkyle en C₁ - C₆ éventuellement substitué par un alcoxy en C₁ - C₄ ou 1 à 3 halogènes, SO₂R₅, COR₅, CO₂R₅ ou CONR₅R₅;
R₅ est un hydrogène, un alkyle en C₁ - C₆ éventuellement substitué par 1 à 3 halogènes, ou un alcényle en C₂ - C₆;
M est un alkylène en C₂ - C₅ éventuellement substitué par 1 ou 2 groupes alkyle en C₁ - C₄, alcoxy en C₁ - C₄, halogènes, CO₂R₆ ou oxo, et éventuellement interrompu par un oxygène ou un soufre,
un alcénylène en C₂ éventuellement substitué par 1 ou 2 groupes alkyles en C₁ - C₄ ou CO₂R₆,
un alcénylène en C₃ éventuellement substitué par 1 ou 2 groupes alkyle en C₁ - C₄, CO₂R₆ ou oxo,
un méthylèneamino, éventuellement substitué par un alkyle en C₁ - C₄ ou CO₂R₆, ou
une liaison simple, à condition que X et Y soient tous les deux NR₄,
à condition que le cycle formé par M, X et Y et le carbone auquel ils sont liés n'ait pas plus de 8 atomes, et à condition que lorsque les substituants de M sont soit un alcoxy, soit un halogène, alors le carbone substitué ne soit pas lié à X ou Y;
R₆ est un hydrogène, un méthyle ou un éthyle;
Z est un hydrogène, un halogène, un alcoxy en C₁ - C₆, ou un alkyle en C₁ - C₆ éventuellement substitué par un alcoxy en C₁ - C₄ ou un halogène.

2. Composé selon la revendication 1 dans lequel Z est un hydrogène, un halogène, ou un méthyle; X et Y sont un oxygène et M est un alkylène non substitué.

3. Composé selon la revendication 2 qui est le 5-(1,3-dioxolan-2-yl)pyridine-2,3-dicarboxylate de diméthyle.

4. Composé selon la revendication 2 qui est le 5-(1,3-dioxan-2-yl)pyridine-2,3-dicarboxylate de diméthyle.

5. Composé selon la revendication 2 qui est le 5-(1,3-dioxolan-2-yl)-6-méthylpyridine-2,3-dicarboxylate de diméthyle.

6. Composé selon la revendication 2, qui est le 5-(1,3-dioxépan-2-yl)pyridine-2,3-dicarboxylate de diméthyle.

7. Procédé de préparation d'un composé: caractérisé par la structure 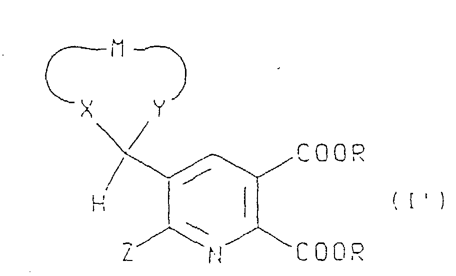 dans laquelle R, Z, X, M et Y sont tels que décrits dans la revendication 1, qui comprend la mise en réaction d'un composé ayant pour structure 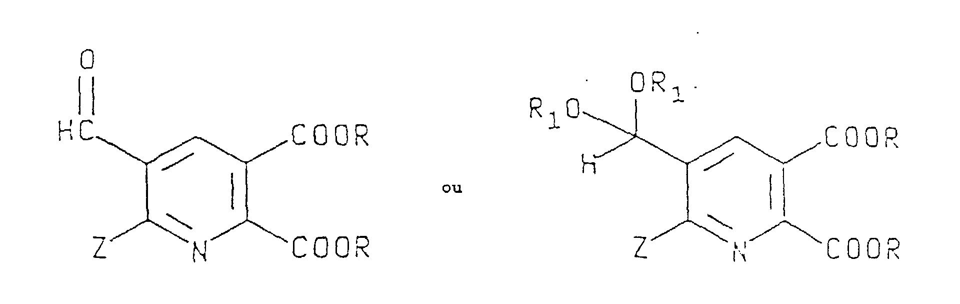 dans laquelle R₁ est un alkyle en C₁ - C₄ et R et Z sont tels que décrits ci-dessus, avec au moins un équivalent d'un agent ayant pour structure
HX - M- YH
dans laquelle X, M et Y sont tels que décrits ci-dessus, en présence d'un acide et d'un solvant.

8. Procédé de préparation d'un composé caractérisé par la structure de formule I' 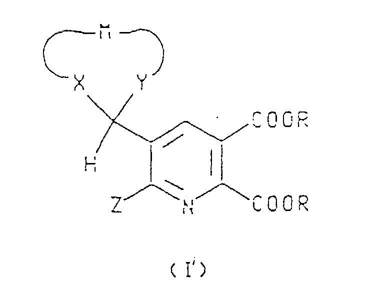 dans laquelle R, Z, X, M et Y sont tels que décrits dans la revendication 1, qui comprend la mise en réaction d'un composé ayant pour structure 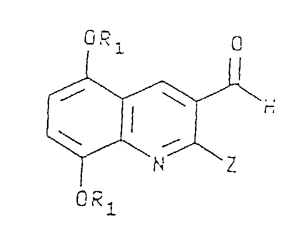 dans laquelle R₁ est un alkyle en C₁ - C₄, avec au moins 2 équivalents molaires d'un trialkyl(en C₁ - C₄)orthoformiate, en présence d'un alcanol en C₁ - C₄, d'ozone et d'une quantité catalytique d'un acide, pour donner un acétal de dialkyle(en C₁ - C₄) de pyridinedicarboxylate ayant pour structure 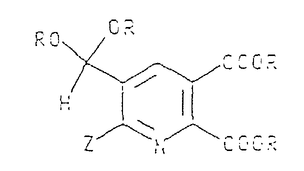 dans laquelle R est un alkyle en C₁ - C₄, et la mise en réaction dudit acétal de dialkyle(en C₁ - C₄) avec au moins un équivalent molaire d'un agent ayant pour structure
HX - M - YH
dans laquelle X, M et Y sont tels que décrits ci-dessus, en présence d'un acide et d'un solvant pour donner le composé 5-hétérocyclique-pyridine-2,3-dicarboxylate de formule I.

9. Procédé de préparation d'un composé caractérisé par la structure de formule I' 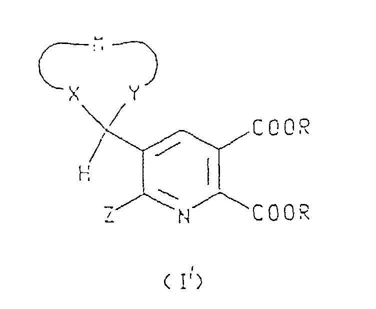 dans laquelle R, Z, X, M et Y sont tels que décrits dans la revendication 1, qui comprend la mise en réaction d'un composé ayant pour structure 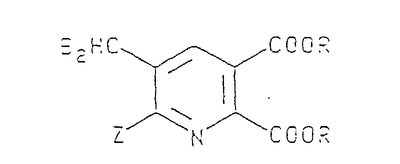 dans laquelle B est un halogène, avec au moins 2 équivalents molaires de AgNO₃ en présence d'un alcanol en C₁ - C₄, à une température supérieure à environ 25°C, pour donner un composé intermédiaire ayant pour structure 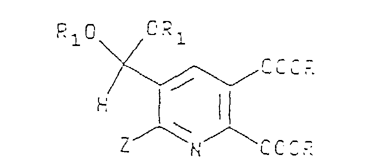 dans laquelle R₁ est un alkyle en C₁ - C₄, et la mise en réaction dudit composé intermédiaire avec un agent ayant pour structure
HX - M - YH
dans laquelle X, M et Y sont tels que décrits ci-dessus, en présence d'un acide et d'un solvant pour donner le composé 5-hétérocyclique - pyridine-2,3-dicarboxylate, de formule I.

10. Composé caractérisé par la structure: 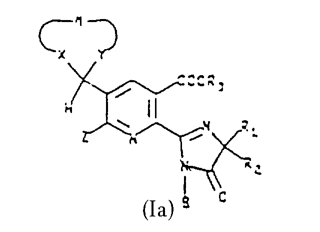 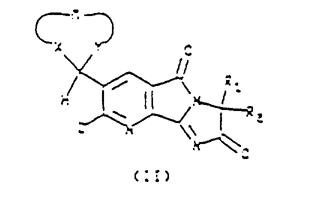 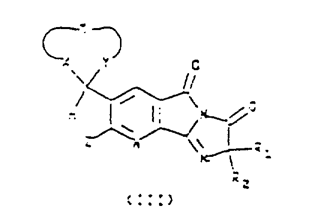 dans laquelle
R₁ est un alkyle en C₁ - C₄;
R₂ est un alkyle en C₁ - C₄ ou un cycloalkyle en C₃ -C₆ et lorsque R₁ et R₂ sont pris conjointement avec le carbone auquel ils sont liés, ils peuvent représenter un cycloalkyle en C₃ - C₆;
R₃ est un hydrogène,
un alkyle en C₁ - C₆ éventuellement substitué par l'un des groupes suivants: un alcoxy en C₁ - C₃, un halogène ou un phényle,
un alcényle en C₃ - C₆ éventuellement substitué par l'un des groupes suivants: un alcoxy en C₁ - C₃, un phényle ou un halogène,
un alcynyle en C₃ - C₆,
un cycloalkyle en C₃ - C₆ éventuellement substitué par un alkyle en C₁ - C₃, ou
un cation de métaux alcalins, l'ammonium ou un ammonium organique;
et dans laquelle X, Y, Z, M sont tels que définis dans la revendication 1,
et dans laquelle B est un hydrogène ou COR₇, pourvu que lorsque B est COR₇, R₃ soit différent d'un hydrogène ou d'un cation;
R₇ est un alkyle en C₁ - C₅ ou un phényle éventuellement substitué par un halogène, un nitro ou un méthoxy,
les N-oxydes de ceux-ci, lorsque R₃ n'est pas un alkyle insaturé et lorsque M n'est pas un alcénylène;
les isomères optiques de ceux-ci, lorsque R₁ et R₂ représentent des substituants différents;
les sels d'addition d'acides de ceux-ci, lorsque R₃ est différent d'un cation, et les tautomères de ceux-ci.

11. Composé selon la revendication 10 caractérisé par la structure 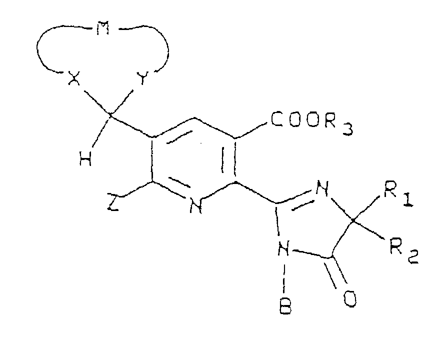 dans laquelle R₁, R₂, R₃, B, X, Y, M et Z sont tels que décrits dans la revendication 10.

12. Composé selon la revendication 11, dans lequel M, X, Y, R₅, R₆ et R₇ sont tels que décrits ici, Z est un hydrogène, un alkyle en C₁ - C₄, un alcoxy en C₁ - C₄ ou un halogène, B est un hydrogène, R₁ est un méthyle, R₂ est un isopropyle et R₃ est un hydrogène, un alkyle en C₁ - C₆, ou un cation de métaux alcalins, l'ammonium ou un ammonium organique.

13. Composé selon la revendication 12 qui est l'acide 5-(1,3-dioxolan-2-yl)-2-(4-isopropyl-4-méthyl-5-oxo-2-imidazolin-2-yl)nicotinique.

14. Composé selon la revendication 12, qui est l'acide 5-(1,3-dioxan-2-yl)-2-(4-isopropyl-4-méthyl-5-oxo-2-imidazolin-2-yl)nicotinique.

15. Composé selon la revendication 12, qui est l'acide 5-(1,3-dioxolan-2-yl)-2-(4-isopropyl-4-méthyl-5-oxo-2-imidazolin-2-yl)-6-méthylnicotinique.

16. Composé selon la revendication 12, qui est l'acide 5-(1,3-dioxépan-2-yl)-2-14-isopropyl-4-méthyl-5-oxo-2-imidazolin-2-yl)nicotinique.

17. Procédé de contrôle d'espèces végétales indésirables, caractérisé par l'application sur le feuillage desdites plantes ou au niveau du sol ou de l'eau contenant les graines ou d'autres organes de propagation de celles-ci, une quantité efficace à titre d'herbicide d'un composé selon la revendication 10.

18. Procédé selon la revendication 17, dans lequel le composé possède la structure 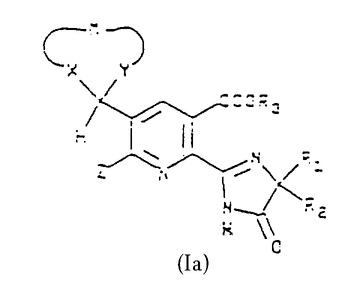 et R₁, R₂, R₃, B, X, Y, M et Z sont tels que décrits dans la revendication 10.

19. Procédé selon la revendication 18, dans lequel le composé est choisi dans le groupe constitué de l'acide 5-(1,3-dioxolan-2-yl)-2-(4-isopropyl-4-méthyl-5-oxo-2-imidazolin-2-yl)nicotinique; l'acide 5-(1,3-dioxan-2-yl)-2-(4-isopropyl-4-méthyl-5-oxo-2-imidazolin-2-yl)nicotinique; l'acide 5-(1,3-dioxolan-2-yl)-2-(4-isopropyl-4-méthyl-5-oxo-2-imidazolin-2-yl)-6-méthylnicotinique; et l'acide 5- (1,3-dioxépan-2-yl)-2-(4-isopropyl-4-méthyl-5-oxo-2-imidazolin-2-yl)nicotinique.

20. Composition permettant de contrôler les espèces végétales indésirables, caractérisée par le composé 5-hétérocyclique-2-(2-imidazolin-2-yl)pyridine tel que décrit dans la revendication 10 et un véhicule agronomiquement acceptable.

21. Procédé de préparation d'un composé 5-hétérocyclique-2-(2-imidazolin-2-yl)pyridine caractérisé par la structure de formule I 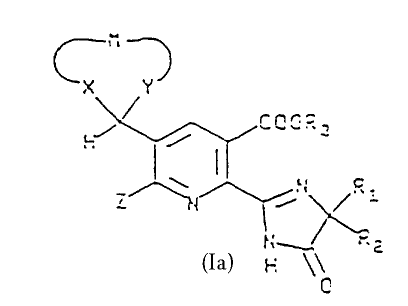 dans laquelle R₃ est un hydrogène et R₁, R₂, Z, X, M et Y sont tels que décrits dans la revendication 1, qui comprend la mise en réaction d'un composé 5-hétérocyclique pyridinedicarboxylate ayant pour structure 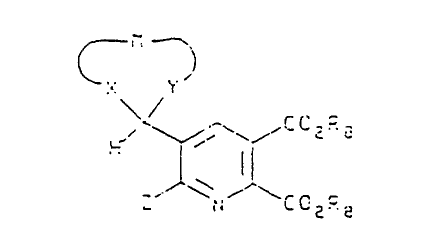 dans laquelle R₈ est un alkyle en C₁ - C₄, avec une base pour obtenir l'acide 5-hétérocyclique-2,3-pyridinedicarboxylique correspondant; la mise en réaction dudit acide pyridine-dicarboxylique avec au moins un équivalent d'un anhydride d'acide pour obtenir un anhydride de l'acide 5-hétérocyclique-2,3-pyridine-dicarboxylique, la mise en réaction dudit anhydride d'acide pyridine-dicarboxylique avec au moins un équivalent d'un aminocarboxamide de structure 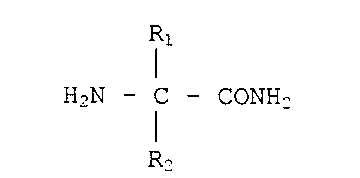 dans laquelle R₁ et R₂ sont tels que décrits ci-dessus pour la formule I, en présence d'un solvant organique inerte, pour obtenir un acide nicotinique ayant pour formule structurelle 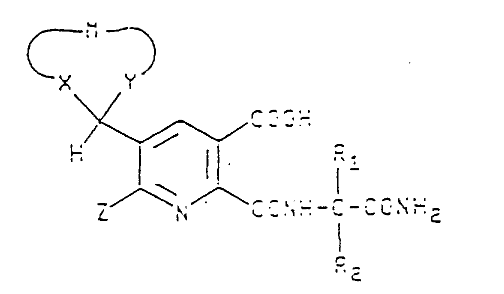 la mise en réaction dudit acide nicotinique avec une base, en présence d'un solvant polaire, pour obtenir un 5-hétérocyclique-2-(2-imidazolin-2-yl)nicotinate, et le traitement dudit nicotinate avec une solution aqueuse d'acide diluée pour former un composé 5-hétérocyclique-2-(2-imidazolin-2-yl)pyridine de formule I dans laquelle R₃ est un hydrogène.

22. Procédé de préparation d'un composé 5-hétérocyclique-2-(2-imidazolin-2-yl)pyridine caractérisé par la structure de formule I 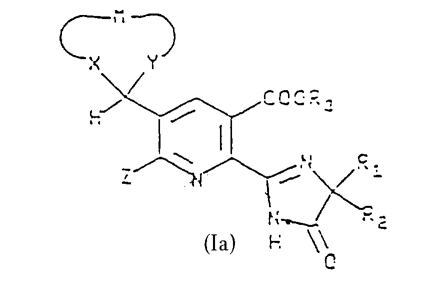 dans laquelle R₃ est un hydrogène et R₁, R₂, X, M, Y et Z sont tels que décrits dans la revendication 10, qui comprend la mise en réaction d'un composé de structure 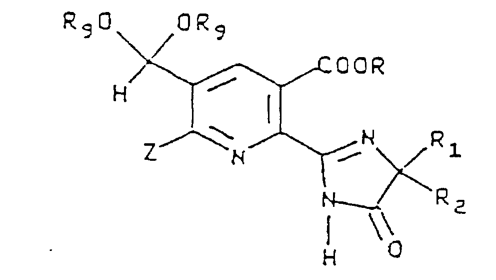 ou 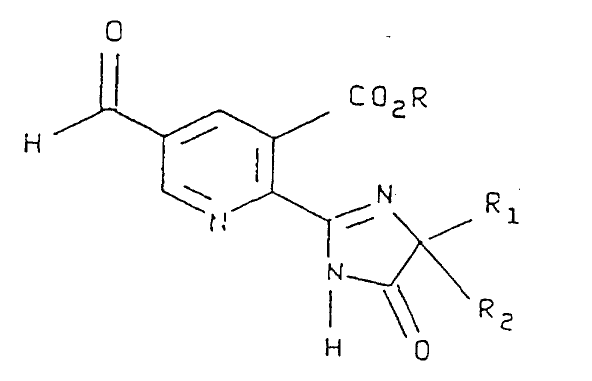 dans laquelle R₉ est un alkyle en C₁ - C₄, avec au moins deux équivalents d'acide chlorhydrique dans de l'eau, pour donner l'intermédiaire correspondant du type acide 5-formyl-2-(5-oxo-2-imidazolin-2-yl)nicotinique, la mise en réaction dudit intermédiaire avec un composé ayant pour formule structurelle
HX - M - YH
dans laquelle M, X et Y sont tels que décrits ci-dessus, en présence d'un solvant organique inerte, à une température élevée, pour former un composé 5-hétérocyclique-2-(2-imidazolin-2-yl)-pyridine de formule I dans laquelle R₃ est un hydrogène.

## Revendications (Revendications pour l'(les) Etat(s) contractant(s) suivant(s): GR)

1. Procédé de préparation d'un composé caractérisé par la structure 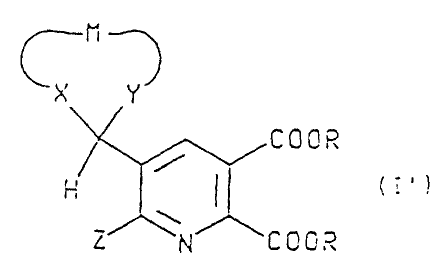 dans laquelle
R est un alkyle en C₁ - C₆;
X et Y sont chacun indépendamment un oxygène, un soufre ou NR₄;
R₄ est un hydrogène, un alkyle en C₁ - C₆ éventuellement substitué par un alcoxy en C₁ - C₄ ou 1 à 3 halogènes, SO₂R₅, COR₅, CO₂R₅ ou CONR₅R₅;
R₅ est un hydrogène, un alkyle en C₁ - C₆ éventuellement substitué par 1 à 3 halogènes, ou un alcényle en C₂ - C₆;
M est un alkylène en C₂ - C₅ éventuellement substitué par 1 ou 2 groupes alkyle en C₁ - C₄, alcoxy en C₁ - C₄, halogènes, CO₂R₆ ou oxo, et éventuellement interrompu par un oxygène ou un soufre,
un alcénylène en C₂ éventuellement substitué par 1 ou 2 groupes alkyle en C₁ - C₄ ou CO₂R₆,
un alcénylène en C₃ éventuellement substitué par 1 ou 2 groupes alkyle en C₁ - C₄, CO₂R₆ ou oxo,
un méthylèneamino, éventuellement substitué par un alkyle en C₁ - C₄ ou CO₂R₆, ou
une liaisons simple, à condition que X et Y soient tous les deux NR₄,
à condition que le cycle formé par M, X et Y et le carbone auquel ils sont liés n'ait pas plus de 8 atomes, et à condition que lorsque les substituants de M sont soit un alcoxy, soit un halogène, alors le carbone substitué ne soit pas lié à X ou Y;
R₆ est un hydrogène, un méthyle ou un éthyle;
Z est un hydrogène, un halogène, un alcoxy en C₁ - C₆, ou un alkyle en C₁ - C₆ éventuellement substitué par un alcoxy en C₁ - C₄ ou un halogène
qui comprend la mise en réaction d'un composé de structure 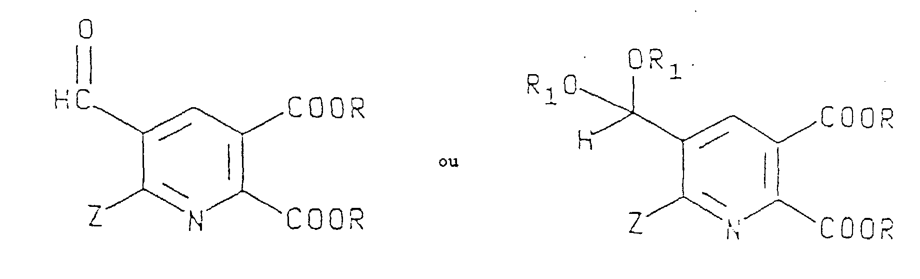 dans laquelle R₁ est un alkyle en C₁ - C₄, et R et Z sont tels que décrits ci-dessus, avec au moins un équivalent d'un agent ayant la structure
HX - M - YH
dans laquelle X, M et Y sont tels que décrits ci-dessus en présence d'un acide et d'un solvant.

2. Procédé selon la revendication 1, dans lequel Z est un hydrogène, un halogène, ou un méthyle; X et Y sont un oxygène et M est un alkylène non substitué.

3. Procédé selon la revendication 2, dans lequel le composé est le 5-(1,3-dioxolan-2-yl)pyridine-2,3-dicarboxylate de diméthyle.

4. Procédé selon la revendication 2, dans lequel le composé est le 5-(1,3-dioxan-2-yl)pyridine-2,3-dicarboxylate de diméthyle.

5. Procédé selon la revendication 2, dans lequel le composé est le 5-(1,3-dioxolan-2-yl)-6-méthylpyridine-2,3-dicarboxylate de diméthyle.

6. Procédé selon la revendication 2, dans lequel le composé est le 5-(1,3-dioxépan-2-yl)pyridine-2,3-dicarboxylate de diméthyle.

7. Procédé de préparation d'un composé caractérisé par la structure de formule I' 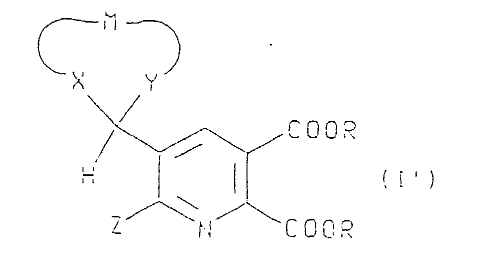 dans laquelle R, Z, X, M et Y sont tels que décrits dans la revendication 1, qui comprend la mise en réaction d'un composé ayant pour structure 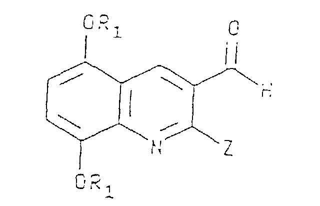 dans laquelle R₁ est un alkyle en C₁ - C₄, avec au moins 2 équivalents molaires d'un trialkyl(en C₁ - C₄)orthoformiate, en présence d'un alcanol en C₁ - C₄, d'ozone et d'une quantité catalytique d'un acide, pour donner un acétal de dialkyle(en C₁ - C₄) de pyridinedicarboxylate ayant pour structure 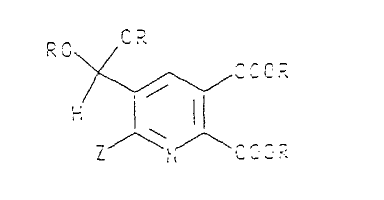 dans laquelle R est un alkyle en C₁ - C₄, et la mise en réaction dudit acétal de dialkyle(en C₁ - C₄) avec au moins un équivalent molaire d'un agent ayant pour structure
HX - M - YH
dans laquelle X, M et Y sont tels que décrits ci-dessus, en présence d'un acide et d'un solvant pour donner le composé 5-hétérocyclique-pyridine-2,3-dicarboxylate de formule I.

8. Procédé de préparation d'un composé caractérisé par la structure de formule I' 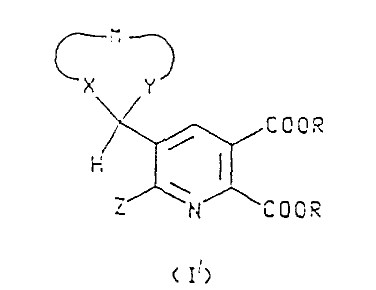 dans laquelle R, Z, X, M et Y sont tels que décrits dans la revendication 1, qui comprend la mise en réaction d'un composé ayant pour structure 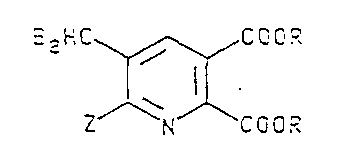 dans laquelle B est un halogène, avec au moins 2 équivalents molaires de AgNO₃ en présence d'un alcanol en C₁ - C₄, à une température supérieure à environ 25°C, pour donner un composé intermédiaire ayant pour structure 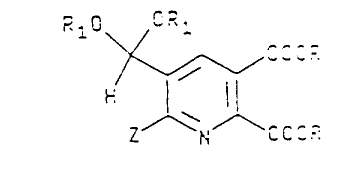 dans laquelle R₁ est un alkyle en C₁ - C₄, et la mise en réaction dudit composé intermédiaire avec un agent ayant pour structure
HX - M - YH
dans laquelle X, M et Y sont tels que décrits ci-dessus, en présence d'un acide et d'un solvant pour donner le composé 5-hétérocyclique - pyridine-2,3-dicarboxylate, de formule I.

9. Procédé de contrôle d'espèces végétales indésirables, caractérisé par l'application sur le feuillage desdites plantes ou au niveau du sol ou de l'eau contenant les graines ou d'autres organes de propagation de celles-ci, une quantité efficace à titre d'herbicide d'un composé caractérisé par la structure: 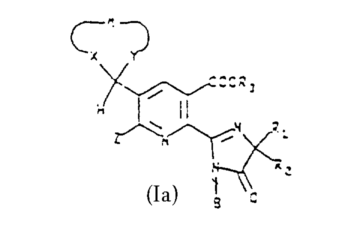 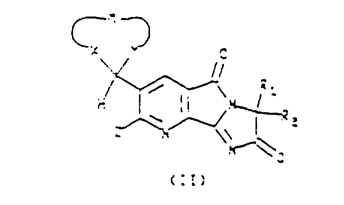 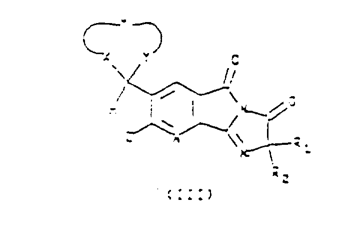 dans laquelle
R₁ est un alkyle en C₁ - C₄;
R₂ est un alkyle en C₁ - C₄ ou un cycloalkyle en C₃ - C₆ et lorsque R₁ et R₂ sont pris conjointement avec le carbone auquel ils sont liés, ils peuvent représenter un cycloalkyle en C₃ - C₆;
R₃ est un hydrogène,
un alkyle en C₁ - C₆ éventuellement substitué par l'un des groupes suivants: un alcoxy en C₁ - C₃, un halogène ou un phényle,
un alcényle en C₃ - C₆ éventuellement substitué par l'un des groupes suivants: un alcoxy en C₁ - C₃, un phényle ou un halogène,
un alcynyle en C₃ - C₆,
un cycloalkyle en C₃ - C₆ éventuellement substitué par un alkyle en C₁ - C₃, ou
un cation de métaux alcalins, l'ammonium ou un ammonium organique;
et dans laquelle X, Y, Z, M sont tels que définis dans la revendication 1, et dans laquelle
B est un hydrogène ou COR₇, pourvu que lorsque B est COR₇, R₃ soit différent d'un hydrogène ou d'un cation;
R₇ est un alkyle en C₁ - C₅ ou un phényle éventuellement substitué par un halogène, un nitro ou un méthoxy,
les N-oxydes de ceux-ci, lorsque R₃ n'est pas un alkyle insaturé et lorsque M n'est pas un alcénylène;
les isomères optiques de ceux-ci, lorsque R₁ et R₂ représentent des substituants différents;
les sels d'addition d'acides de ceux-ci, lorsque R₃ est différent d'un cation, et les tautomères de ceux-ci.

10. Procédé selon la revendication 9, dans lequel le composé est caractérisé par la structure 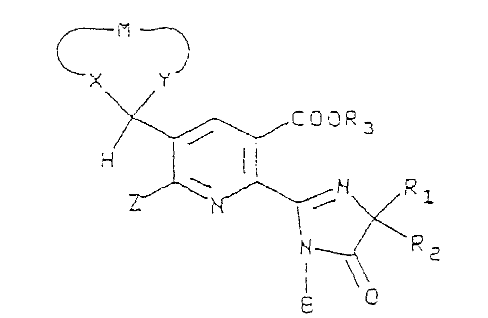 dans laquelle R₁, R₂, R₃, B, X, Y, M et Z sont tels que décrits dans la revendication 9.

11. Procédé selon la revendication 10, dans lequel M, X, Y, R₅, R₆ et R₇ sont tels que décrits ici, Z est un hydrogène, un alkyle en C₁ - C₄, un alcoxy en C₁ - C₄ ou un halogène, B est un hydrogène, R₁ est un méthyle, R₂ est un isopropyle et R₃ est un hydrogène, un alkyle en C₁ - C₆, ou un cation de métaux alcalins, l'ammonium ou un ammonium organique.

12. Procédé selon la revendication 11, dans lequel le composé est l'acide 5-(1,3-dioxolan-2-yl)-2-(4-isopropyl-4-méthyl-5-oxo-2-imidazolin-2-yl)nicotinique.

13. Procédé selon la revendication 11, dans lequel le composé est l'acide 5-(1,3-dioxan-2-yl)-2-(4-isopropyl-4-méthyl-5-oxo-2-imidazolin-2-yl)nicotinique.

14. Procédé selon la revendication 12, dans lequel le composé est l'acide 5-(1,3-dioxolan-2-yl)-2-(4-isopropyl-4-méthyl-5-oxo-2-imidazolin-2-yl)-6-méthylnicotinique.

15. Procédé selon la revendication 12, dans lequel le composé est l'acide 5-(1,3-dioxépan-2-yl)-2-(4-isopropyl-4-méthyl-5-oxo-2-imidazolin-2-yl)nicotinique.

16. Procédé selon la revendication 10, dans lequel le composé est choisi dans le groupe constitué de l'acide 5-(1,3-dioxolan-2-yl)-2-(4-isopropyl-4-méthyl-5-oxo-2-imidazolin-2-yl)nicotinique; l'acide 5-(1,3-dioxan-2-yl)-2-(4-isopropyl-4-méthyl-5-oxo-2-imidazolin-2-yl)nicotinique; l'acide 5-(1,3-dioxolan-2-yl)-2-(4-isopropyl-4-méthyl-5-oxo-2-imidazolin-2-yl)-6-méthylnicotinique; et l'acide 5-(1,3-dioxépan-2-yl)-2-(4-isopropyl-4-méthyl-5-oxo-2-imidazolin-2-yl)nicotinique.

17. Procédé de préparation d'une composition permettant de contrôler les espèces végétales indésirables, caractérisé par le mélange d'un composé 5-hétérocyclique-2-(2-imidazolin-2-yl)pyridine tel que décrit dans la revendication 9 et d'un véhicule agronomiquement acceptable.

18. Procédé de préparation d'un composé 5-hétérocyclique-2-(2-imidazolin-2-yl)pyridine caractérisé par la structure de formule I 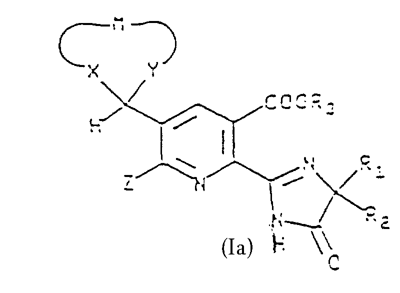 dans laquelle R₃ est un hydrogène et R₁, R₂, Z, X, M et Y sont tels que décrits dans la revendication 1, qui comprend la mise en réaction d'un composé 5-hétérocyclique pyridinedicarboxylate ayant pour structure 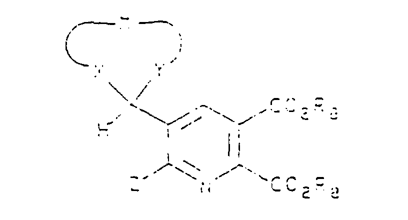 dans laquelle R₈ est un alkyle en C₁ - C₄, avec une base pour obtenir l'acide 5-hétérocyclique-2,3-pyridine-dicarboxylique correspondant; la mise en réaction dudit acide pyridine-dicarboxylique avec au moins un équivalent d'un anhydride d'acide pour obtenir un anhydride de l'acide 5-hétérocyclique-2,3-pyridine-dicarboxylique, la mise en réaction dudit anhydride d'acide pyridine-dicarboxylique avec au moins un équivalent d'un aminocarboxamide de structure 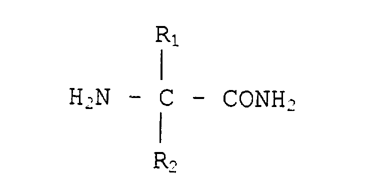 dans laquelle R₁ et R₂ sont tels que décrits ci-dessus pour la formule I, en présence d'un solvant organique inerte, pour obtenir un acide nicotinique ayant pour formule structurelle 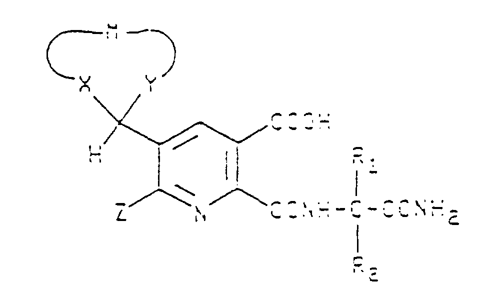 la mise en réaction dudit acide nicotinique avec une base, en présence d'un solvant polaire, pour obtenir un 5-hétérocyclique-2-(2-imidazolin-2-yl)nicotinate, et le traitement dudit nicotinate avec une solution aqueuse d'acide diluée pour former un composé 5-hétérocyclique-2-(2-imidazolin-2-yl)pyridine de formule I dans laquelle R₃ est un hydrogène.

19. Procédé de préparation d'un composé 5-hétérocyclique-2-(2-imidazolin-2-yl)pyridine caractérisé par la structure de formule I 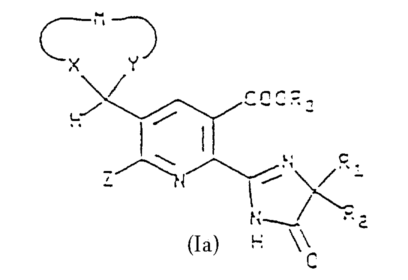 dans laquelle R₃ est un hydrogène et R₁, R₂, X, M, Y et Z sont tels que décrits dans la revendication 9, qui comprend la mise en réaction d'un composé de structure 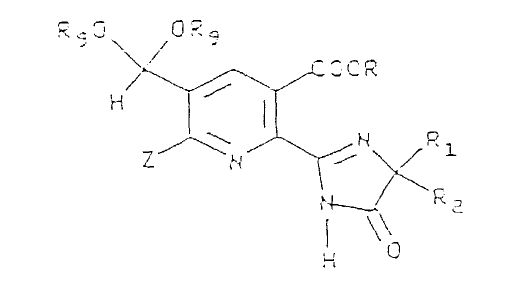 ou 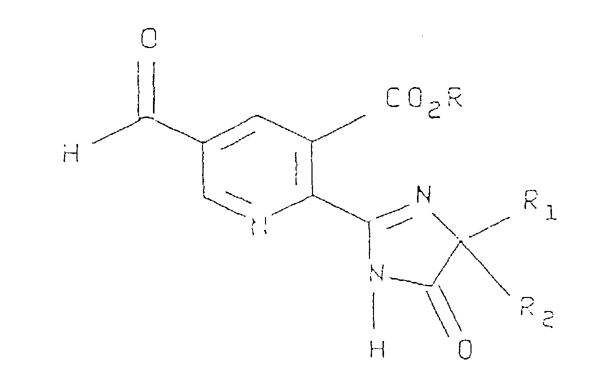 dans laquelle R₉ est un alkyle en C₁ - C₄, avec au moins deux équivalents d'acide chlorhydrique dans de l'eau, pour donner l'intermédiaire correspondant du type acide 5-formyl-2-(5-oxo-2-imidazolin-2-yl)nicotinique, la mise en réaction dudit intermédiaire avec un composé ayant pour formule structurelle
HX - M - YH
dans laquelle M, X et Y sont tels que décrits ci-dessus, en présence d'un solvant organique inerte, à une température élevée, pour former un composé 5-hétérocyclique-2-(2-imidazolin-2-yl)-pyridine de formule I dans laquelle R₃ est un hydrogène.

20. Composition permettant de contrôler les espèces végétales indésirables, caractérisée par le composé 5-hétérocyclique-2-(2-imidazolin-2-yl)pyridine tel que décrit dans la revendication 9 et un véhicule agronomiquement acceptable.

21. Composition selon la revendication 20, dans laquelle le composé est caractérisé par la structure 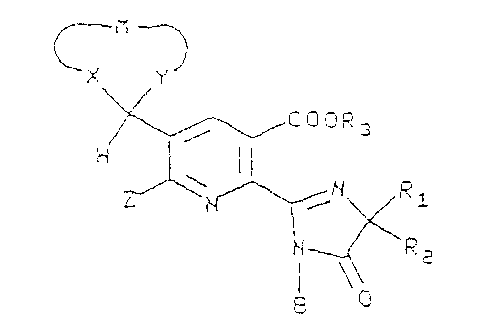 dans laquelle R₁, R₂, R₃, B, X, Y, M et Z sont tels que décrits dans la revendication 9.

22. Composition selon la revendication 21, dans laquelle M, X, Y, R₅, R₆ et R₇ sont tels que décrits ici, Z est un hydrogène, un alkyle en C₁ - C₄, un alcoxy en C₁ - C₄ ou un halogène, B est un hydrogène, R₁ est un méthyle, R₃ est un isopropyle et R₃ est un hydrogène, un alkyle en C₁ - C₆, ou un cation de métaux alcalins, l'ammonium ou un ammonium organique.

23. Composition selon la revendication 22, dans laquelle le composé est l'acide 5-(1,3-dioxolan-2-yl)-2-(4-isopropyl-4-méthyl-5-oxo-2-imidazolin-2-yl)nicotinique.

24. Composé selon la revendication 22, dans laquelle le composé est l'acide 5-(1,3-dioxan-2-yl)-2-(4-isopropyl-4-méthyl-5-oxo-2-imidazolin-2-yl)nicotinique.

25. Composition selon la revendication 22, dans laquelle le composé est l'acide 5-(1,3-dioxolan-2-yl)-2-(4-isopropyl-4-méthyl-5-oxo-2-imidazolin-2-yl)-6-méthylnicotinique.

26. Composition selon la revendication 22, dans laquelle le composé est l'acide 5-(1,3-dioxépan-2-yl)-2-(4-isopropyl-4-méthyl-5-oxo-2-imidazolin-2-yl)nicotinique.

## Revendications (Revendications pour l'(les) Etat(s) contractant(s) suivant(s): ES)

1. Procédé de préparation d'un composé caractérisé par la structure 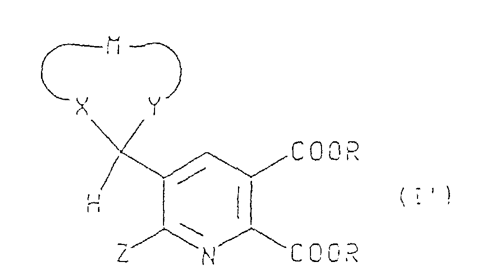 dans laquelle
R est un alkyle en C₁ - C₆;
X et Y sont chacun indépendamment un oxygène, un soufre ou NR₄;
R₄ est un hydrogène, un alkyle en C₁ - C₆ éventuellement substitué par un alcoxy en C₁ - C₄ ou 1 à 3 halogènes, SO₂R₅, COR₅, CO₂R₅ ou CONR₅R₅;
R₅ est un hydrogène, un alkyle en C₁ - C₆ éventuellement substitué par 1 à 3 halogènes, ou un alcényle en C₂ - C₆;
M est un alkylène en C₂ - C₅ éventuellement substitué par 1 ou 2 groupes alkyle en C₁ - C₄, alcoxy en C₁ - C₄, halogènes, CO₂R₆ ou oxo, et éventuellement interrompu par un oxygène ou un soufre,
un alcénylène en C₂ éventuellement substitué par 1 ou 2 groupes alkyles en C₁ - C₄ ou CO₂R₆,
un alcénylène en C₃ éventuellement substitué par 1 ou 2 groupes alkyle en C₁ - C₄, CO₂R₆ ou oxo,
un méthylèneamino, éventuellement substitué par un alkyle en C₁ - C₄ ou CO₂R₆, ou
une liaison simple, à condition que X et Y soient tous les deux NR₄,
à condition que le cycle formé par M, X et Y et le carbone auquel ils sont liés n'ait pas plus de 8 atomes, et à condition que lorsque les substituants de M sont soit un alcoxy, soit un halogène, alors le carbone substitué ne soit pas lié à X ou Y;
R₆ est un hydrogène, un méthyle ou un éthyle;
Z est un hydrogène, un halogène, un alcoxy en C₁ - C₆, ou un alkyle en C₁ - C₆ éventuellement substitué par un alcoxy en C₁ - C₄ ou un halogène.
qui comprend la mise en réaction d'un composé de structure 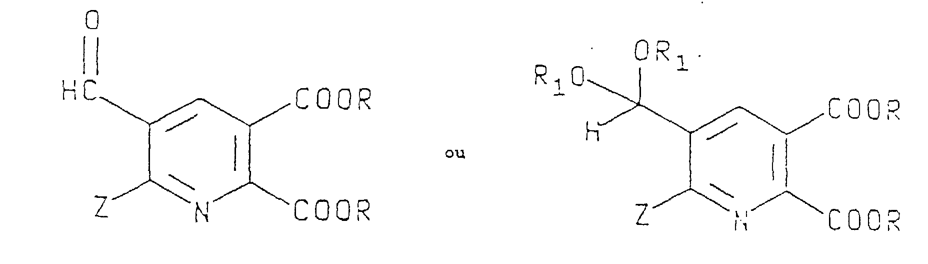 dans laquelle R₁ est un alkyle en C₁ - C₄, et R et Z sont tels que décrits ci-dessus, avec au moins un équivalent d'un agent ayant la structure
HX - M - YH
dans laquelle X, M et Y sont tels que décrits ci-dessus en présence d'un acide et d'un solvant.

2. Procédé selon la revendication 1, dans lequel Z est un hydrogène, un halogène ou un méthyle; X et Y sont un oxygène et M est un alkylène non substitué.

3. Procédé selon la revendication 2, dans lequel le composé est le 5-(1,3-dioxolan-2-yl)pyridine-2,3-dicarboxylate de diméthyle.

4. Procédé selon la revendication 2, dans lequel le composé est le 5-(1,3-dioxan-2-yl)pyridine-2,3-dicarboxylate de diméthyle.

5. Procédé selon la revendication 2, dans lequel le composé est le 5-(1,3-dioxolan-2-yl)-6-méthylpyridine-2,3-dicarboxylate de diméthyle.

6. Procédé selon la revendication 2, dans lequel le composé est le 5-(1,3-dioxépan-2-yl)pyridine-2,3-dicarboxylate de diméthyle.

7. Procédé de préparation d'un composé caractérisé par la structure de formule I' 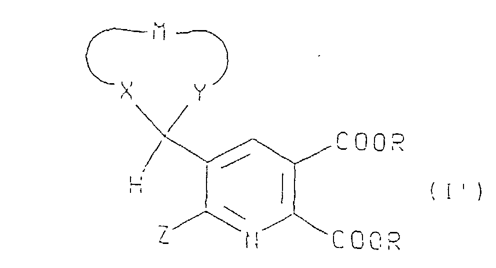 dans laquelle R, Z, X, M et Y sont tels que décrits dans la revendication 1, qui comprend la mise en réaction d'un composé ayant pour structure 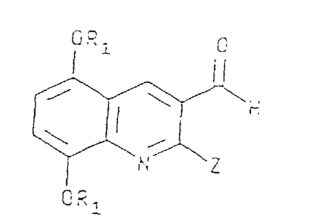 dans laquelle R₁ est un alkyle en C₁ - C₄, avec au moins 2 équivalents molaires d'un trialkyl(en C₁ - C₄)orthoformiate, en présence d'un alcanol en C₁ - C₄, d'ozone et d'une quantité catalytique d'un acide, pour donner un acétal de dialkyle(en C₁ - C₄) de pyridinedicarboxylate ayant pour structure 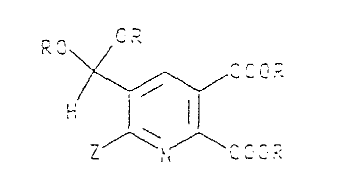 dans laquelle R est un alkyle en C₁ - C₄, et la mise en réaction dudit acétal de dialkyle(en C₁ - C₄) avec au moins un équivalent molaire d'un agent ayant pour structure
HX - M - YH
dans laquelle X, M et Y sont tels que décrits ci-dessus, en présence d'un acide et d'un solvant pour donner le composé 5-hétérocyclique-pyridine-2,3-dicarboxylate de formule I.

8. Procédé de préparation d'un composé caractérisé par la structure de formule I' 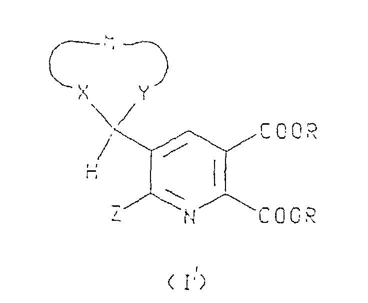 dans laquelle R, Z, X, M et Y sont tels que décrits dans la revendication 1, qui comprend la mise en réaction d'un composé ayant pour structure 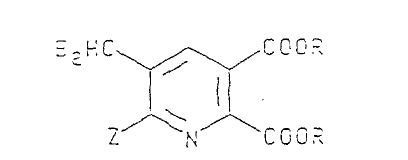 dans laquelle B est un halogène, avec au moins 2 équivalents molaires de AgNO₃ en présence d'un alcanol en C₁ - C₄, à une température supérieure à environ 25°C, pour donner un composé intermédiaire ayant pour structure 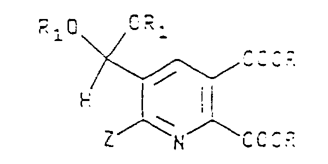 dans laquelle R₁ est un alkyle en C₁ - C₄, et la mise en réaction dudit composé intermédiaire avec un agent ayant pour structure
HX - M - YH
dans laquelle X, M et Y sont tels que décrits ci-dessus, en présence d'un acide et d'un solvant pour donner le composé 5-hétérocyclique - pyridine-2,3-dicarboxylate, de formule I.

9. Procédé de contrôle d'espèces végétales indésirables, caractérisé par l'application sur le feuillage desdites plantes ou au niveau du sol ou de l'eau contenant les graines ou d'autres organes de propagation de celles-ci, une quantité efficace à titre d'herbicide d'un composé caractérisé par la structure: 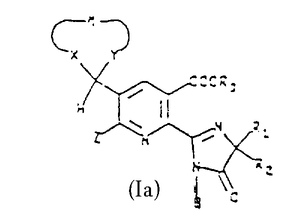 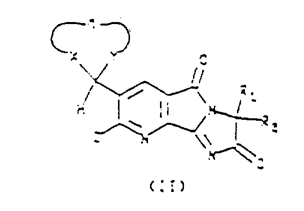 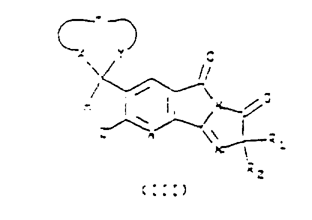 dans laquelle
R₁ est un alkyle en C₁ - C₄;
R₂ est un alkyle en C₁ - C₄ ou un cycloalkyle en C₃ -C₆, et lorsque R₁ et R₂ sont pris conjointement avec le carbone auquel ils sont liés, ils peuvent représenter un cycloalkyle en C₃ - C₆;
R₃ est un hydrogène,
un alkyle en C₁ - C₆ éventuellement substitué par l'un des groupes suivants: un alcoxy en C₁ - C₃, un halogène ou un phényle,
un alcényle en C₃ - C₅ éventuellement substitué par l'un des groupes suivants: un alcoxy en C₁ - C₃, un phényle ou un halogène,
un alcynyle en C₃ - C₆,
un cycloalkyle en C₃ - C₆ éventuellement substitué par un alkyle en C₁ - C₃, ou
un cation de métaux alcalins, l'ammonium ou un ammonium organique;
et dans laquelle X, Y, Z, M sont tels que définis dans la revendication 1, et dans laquelle
B est un hydrogène ou COR₇, pourvu que lorsque B est COR₇, R₃ soit différent d'un hydrogène ou d'un cation;
R₇ est un alkyle en C₁ - C₅ ou un phényle éventuellement substitué par un halogène, un nitro ou un méthoxy,
les N-oxydes de ceux-ci, lorsque R₃ n'est pas un alkyle insaturé et lorsque M n'est pas un alcénylène;
les isomères optiques de ceux-ci, lorsque R₁ et R₂ représentent des substituants différents;
les sels d'addition d'acides de ceux-ci, lorsque R₃ est différent d'un cation, et les tautomères de ceuxci.

10. Procédé selon la revendication 9, dans lequel le composé est caractérisé par la structure 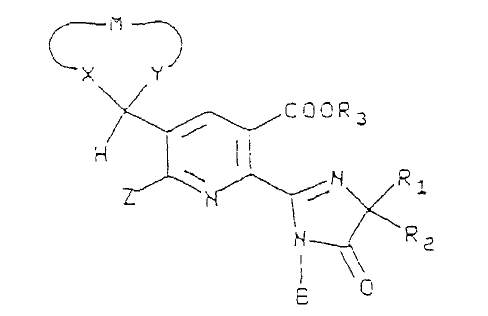 dans laquelle R₁, R₂, R₃, B, X, Y, M et Z sont tels que décrits dans la revendication 9.

11. Procédé selon la revendication 10, dans lequel M, X, Y, R₅, R₆ et R₇ sont tels que décrits ici, Z est un hydrogène, un alkyle en C₁ - C₄, un alcoxy en C₁ - C₄ ou un halogène, B est un hydrogène, R₁ est un méthyle, R₂ est un isopropyle et R₃ est un hydrogène, un alkyle en C₁ - C₆, ou un cation de métaux alcalins, l'ammonium ou un ammonium organique.

12. Procédé selon la revendication 11, dans lequel le composé est l'acide 5-(1,3-dioxolan-2-yl)-2-(4-isopropyl-4-méthyl-5-oxo-2-imidazolin-2-yl)nicotinique.

13. Procédé selon la revendication 11, dans lequel le composé est l'acide 5-(1,3-dioxan-2-yl)-2-(4-isopropyl-4-méthyl-5-oxo-2-imidazolin-2-yl)nicotinique.

14. Procédé selon la revendication 12, dans lequel le composé est l'acide 5-(1,3-dioxolan-2-yl)-2-(4-isopropyl-4-méthyl-5-oxo-2-imidazolin-2-yl)-6-méthylnicotinique.

15. Procédé selon la revendication 12, dans lequel le composé est l'acide 5-(1,3-dioxépan-2-yl)-2-(4-isopropyl-4-méthyl-5-oxo-2-imidazolin-2-yl)nicotinique.

16. Procédé selon la revendication 10, dans lequel le composé est choisi dans le groupe constitué de l'acide 5-(1,3-dioxolan-2-yl)-2-(4-isopropyl-4-méthyl-5-oxo-2-imidazolin-2-yl)nicotinique; l'acide 5-(1,3-dioxan-2-yl)-2-(4-isopropyl-4-méthyl-5-oxo-2-imidazolin-2-yl)nicotinique; l'acide 5-(1,3-dioxolan-2-yl)-2-(4-isopropyl-4-méthyl-5-oxo-2-imidazolin-2-yl)-6-méthylnicotinique; et l'acide 5-(1,3-dioxépan-2-yl)-2-(4-isopropyl-4-méthyl-5-oxo-2-imidazolin-2-yl)nicotinique.

17. Procédé de préparation d'une composition permettant de contrôler les espèces végétales indésirables, caractérisé par le mélange d'un composé 5-hétérocyclique-2-(2-imidazolin-2-yl)pyridine tel que décrit dans la revendication 9 et d'un véhicule agronomiquement acceptable.

18. Procédé de préparation d'un composé 5-hétérocyclique-2-(2-imidazolin-2-yl)pyridine caractérisé par la structure de formule I 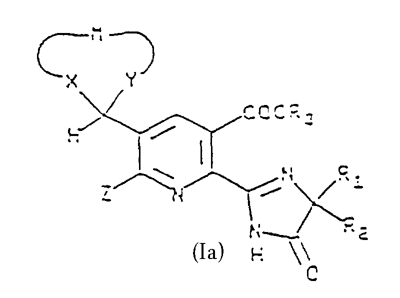 dans laquelle R₃ est un hydrogène et R₁, R₂, Z, X, M et Y sont tels que décrits dans la revendication 1, qui comprend la mise en réaction d'un composé 5-hétérocyclique pyridinedicarboxylate ayant pour structure 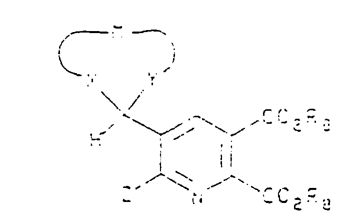 dans laquelle R₈ est un alkyle en C₁ - C₄, avec une base pour obtenir l'acide 5-hétérocyclique-2,3-pyridinedicarboxylique correspondant; la mise en réaction dudit acide pyridine-dicarboxylique avec au moins un équivalent d'un anhydride d'acide pour obtenir un anhydride de l'acide 5-hétérocyclique-2,3-pyridine-dicarboxylique, la mise en réaction dudit anhydride d'acide pyridine-dicarboxylique avec au moins un équivalent d'un aminocarboxamide de structure 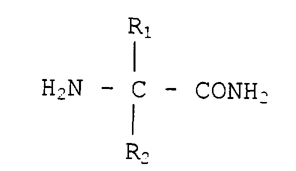 dans laquelle R₁ et R₂ sont tels que décrits ci-dessus pour la formule I, en présence d'un solvant organique inerte, pour obtenir un acide nicotinique ayant pour formule structurelle 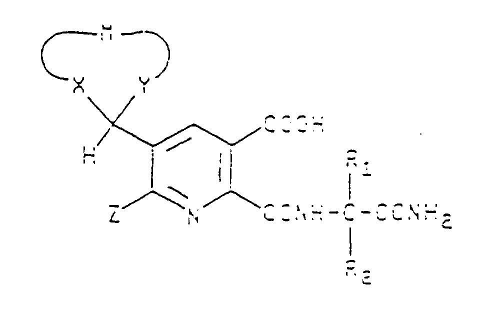 la mise en réaction dudit acide nicotinique avec une base, en présence d'un solvant polaire, pour obtenir un 5-hétérocyclique-2-(2-imidazolin-2-yl)nicotinate, et le traitement dudit nicotinate avec une solution aqueuse d'acide dilué pour former un composé 5-hétérocyclique-2-(2-imidazolin-2-yl)pyridine de formule I dans laquelle R₃ est un hydrogène.

19. Procédé de préparation d'un composé 5-hétérocyclique-2-(2-imidazolin-2-yl)pyridine caractérisé par la structure de formule I 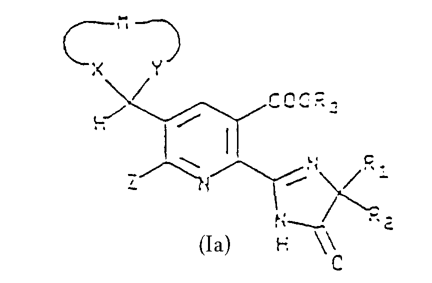 dans laquelle R₃ est un hydrogène et R₁, R₂, X, M, Y et Z sont tels que décrits dans la revendication 9, qui comprend la mise en réaction d'un composé de structure 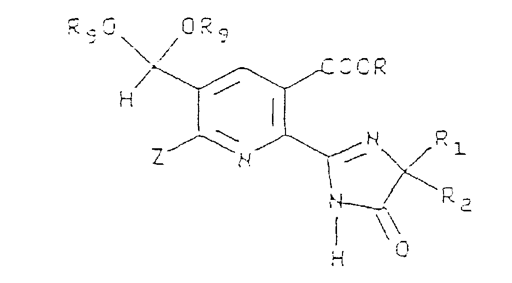 ou 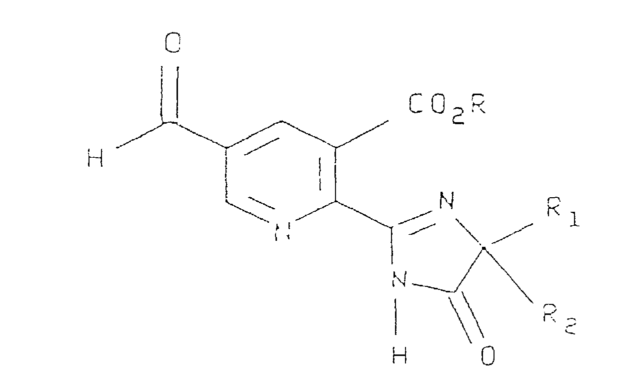 dans laquelle R₉ est un alkyle en C₁ - C₄, avec au moins deux équivalents d'acide chlorhydrique dans de l'eau, pour donner l'intermédiaire correspondant du type acide 5-formyl-2-(5-oxo-2-imidazolin-2-yl)nicotinique, la mise en réaction dudit intermédiaire avec un composé ayant pour formule structurelle
HX - M - YH
dans laquelle M, X et Y sont tels que décrits ci-dessus, en présence d'un solvant organique inerte, à une température élevée, pour former un composé 5-hétérocyclique-2-(2-imidazolin-2-yl)-pyridine de formule I dans laquelle R₃ est un hydrogène.
